(19) Europäisches Patentamt
European Patent Office
Office européen des brevets

(11) EP 1 001 961 B1

(12) EUROPÄISCHE PATENTSCHRIFT

(45) Veröffentlichungstag und Bekanntmachung des Hinweises auf die Patenterteilung:
**26.01.2005 Patentblatt 2005/04**

(21) Anmeldenummer: **98945178.6**

(22) Anmeldetag: **07.08.1998**

(51) Int Cl.[7]: **C07H 1/00**

(86) Internationale Anmeldenummer:
**PCT/EP1998/005025**

(87) Internationale Veröffentlichungsnummer:
**WO 1999/007718 (18.02.1999 Gazette 1999/07)**

(54) **SUBSTITUIERTE TETRAHYDROPYRANDERIVATE SOWIE VERFAHREN ZU DEREN HERSTELLUNG**

SUBSTITUTED TETRAHYDROPYRANE DERIVATIVES AND METHOD FOR PRODUCING SAME

DERIVES SUBSTITUES DU TETRAHYDROPYRANE ET LEUR PROCEDE DE PRODUCTION

(84) Benannte Vertragsstaaten:
**AT BE CH CY DE DK ES FI FR GB GR IE IT LI LU NL PT SE**

(30) Priorität: **08.08.1997 DE 19734392**
**09.05.1998 DE 19820815**

(43) Veröffentlichungstag der Anmeldung:
**24.05.2000 Patentblatt 2000/21**

(73) Patentinhaber: **Aventis Pharma Deutschland GmbH**
**65929 Frankfurt am Main (DE)**

(72) Erfinder:
- **SCHMIDT, Wolfgang**
  **D-65929 Frankfurtam Main (DE)**
- **HENKE, Stephan**
  **D-65719 Hofheim (DE)**
- **KUNZ, Horst**
  **D-55127 Mainz (DE)**
- **KALLUS, Christopher**
  **D-55099 Mainz (DE)**
- **OBATZ, Till**
  **D-55099 Mainz (DE)**
- **WUNBERG, Tobias**
  **D-55099 Mainz (DE)**

(56) Entgegenhaltungen:

| | |
|---|---|
| **EP-A- 0 063 373** | **EP-A- 0 233 837** |
| **EP-A- 0 319 253** | **WO-A-92/05444** |
| **WO-A-92/22564** | **WO-A-93/11776** |
| **WO-A-94/19360** | **WO-A-95/03315** |
| **WO-A-95/11686** | **WO-A-95/18814** |
| **WO-A-96/23479** | **WO-A-97/20855** |
| **WO-A-97/22346** | **WO-A-97/28173** |
| **WO-A-97/45436** | **DE-A- 2 212 014** |
| **DE-A- 2 630 596** | **DE-A- 2 816 340** |
| **DE-A- 3 220 427** | **US-A- 5 220 008** |
| **US-A- 5 444 050** | **US-A- 5 455 240** |

- **TAKEO ET AL.: "Synthesis of 8-methoxycarbonyloctyl-beta-glycosides" J. CARBOHYDR. CHEM., Bd. 13, Nr. 8, 1994, Seiten 1159-77, XP002091924**
- **POCHET, S. ET AL.: "Alkylglycoside carbonates of 3'-azido-3'-deoxythymidine" TETRAHEDR. LETT., Bd. 31, Nr. 42, 1990, Seiten 6021-4, XP002091925**
- **BEYER: LEHRBUCH DER ORGANISCHEN CHEMIE, 1981, Seiten 411-3, XP002091926**
- **WIJNBERGEN, F. ET AL.: "Synthesis of some methyl 6-O-alkyl-alpha-D-glycopyranosides" J. CARBOHYDR. RES., Bd. 280, 1996, Seiten 151-6, XP004018842**
- **VALENTIJN A. ET AL: "Solid-phase synthesis of lysine-based cluster galactosides with high affinity for the asialoglycoprotein receptor" TETRAHEDRON, Bd. 53, Nr. 2, 1997, Seiten 759-770, XP002103084**
- **BUNDESVERBAND DER PHARMAZEUTISCHEN INDUSTRIE: ROTE LISTE,1995, Seiten 51107-51115, XP002103085**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**(Forts. nächste Seite)**

- **PATENT ABSTRACTS OF JAPAN vol. 95, no. 05 & JP 07 048264 A (NIPPON TERUPEN KAGAKU KK), 21. Februar 1995**
- **PATENT ABSTRACTS OF JAPAN vol. 017, no. 291 (C-1067), 4. Juni 1993 & JP 05 017359 A (SANYO CHEM IND LTD), 26. Januar 1993**

## Beschreibung

Die Erfindung betrifft substituierte Tetrahydropyranderivate, und Verfahren zu deren Herstellung.

Für die Auffindung neuer Leitstrukturen und der Identifikation von potentiellen Wirkstoffen stellen Peptide und Peptidmimetika ein wertvolles Hilfsmittel dar. Durch Fixierung von Seitenketten in einem starren Gerüst (Scaffold) erhofft man sich im Vergleich zur konformativ flexibleren Peptidkette eine Erhöhung der Affinität dieser konformativ fixierten Liganden zum Rezeptor.

Als Peptidmimetikum finden bereits verschiedenste Gerüstbausteine Einsatz.

Kohlenhydratbausteine sollten durch ihre Multivalenz und ihre definierte räumliche Anordnung besonders gut als Gerüstbausteine für Peptidmimetika geeignet sein.

So wurde kürzlich gezeigt, daß ein spezielles Monosaccharid als konformativ fixiertes Gerüst die räumliche Anordnung eines bestimmten Cyclopeptides, des Somatostatins nachahmt. (K.C. Nicolaou, J.I. Trujillo, K. Chibale, Tetrahedron 1997, 53, 8751-8778).

Hierbei wurde ausgehend von einen Glycosederivat mit Standardschutzgruppen eine beschränkte Variation an der einfach zugänglichen anomeren Hydroxylfunktion und der C-6 Hydroxylfunktion durchgeführt. Die dort beschriebene Synthesestrategie geht von bereits bekannten Zuckerbausteinen aus und ist durch die Schutzgruppenstrategie beschränkt auf einen engen Anwendungsbereiches des Somatostatins. Gleichzeitig ist die Methode nicht übertragbar auf die gezielte Variation des Gerüstbausteins durch Festphasensynthese.

Die bisherigen Synthesen von Kohlenhydratderivaten in Lösung oder in Form von Substanzbibilotheken an fester Phase konzentrieren sich insbesondere auf die Synthese von Oligosacchariden oder Glycopeptiden ( L. DeNapoli et. al., Tetrahedron Letters1996, 37, 5007-5010; S.J Danishefsky et. al., Science 1995, 269, 202-204, J. J. Krepinski et.al., J. Am. Chem. Soc., 1991, 113, 5095-5097).

Aus Oligosacchariden- oder Glycopetidbausteinen aufgebaute Verbindungen sind jedoch aufgrund ihrer Komplexität für die Auffindung von Leitstrukturen oder als potentielle Wirkstoffe nur sehr beschränkt einsetzbar.

Die Beschränkung auf ein Monosaccharid als Gerüstbaustein vereint hingegen die positive Eigenschaft der definierten räumlichen Anordnung der Liganden mit einer geringen Komplexität, geringem Molekulargewicht, geringer Toxizität und weiteren Eigenschaften, die für potentielle Wirkstoffe von Bedeutung sind.

Aufgrund der Multivalenz der Monosacharide bereitet die gezielte Synthese selektiv funktionalisierter Monosacharide - sowohl in Lösung als auch an der Festphase - große Schwierigkeiten.

Verschieden geschützte Kohlenhydratbausteine sind ebenfalls durch die verschiedenen Arbeiten zur Kohlenhydratchemie bekannt (s. R:R: Schmidt, Pure & Appl. Chem. 1989, 61, 1257). Bei den dort beschriebenen Intermediaten sind die Hydroxylgruppen temporär mehr oder weniger selektiv durch Schutzgruppen blockiert, die dann zur Verknüpfung mit anderen Schutzgruppen entschützt werden, wodurch der Aufbau von Di bzw. Oligosacchariden erfolgt. Diese Intermediate oder die daraus aufgebauten Mehrfachzucker sind zur gezielten Leitstrukturfindung und als potentielle Wirkstoffe jedoch nur eingeschränkt einsetzbar. Diese Strukturen sind z.T. relativ labil und so gegen Abbau bzw. Spaltung nicht resistent.

Auch die für die Herstellung der oben erwähnten Polysaccharide bzw. Glycopeptide entwickelten Linker und Aktivierungsstrategien ( D. Kahne et. al., J. Am. Chem. Soc. 1994, 116, 6953-6954) sind nicht allgemein auf die Darstellung selektiv mehrfach substituierter Monosaccharidverbindungen übertragbar.

Die gezielten Synthese selektiv funktionalisierter Monosaccharidderivate erfordert daher die Entwicklung einer neuen, vollkommenen orthogonalen Schutzgruppenstrategie, die es ermöglicht, die Schutzgruppen aller funktionalen Gruppen selektiv abzuspalten, wobei die hierfür angewandeten Bedingungen stabil zu den Bedingungen der Synthesesequenz sind. Gleichzeitig müssen diese Schutzgruppen die Kompatibifität zu allen Reaktionsbedingungen, die an der Festphasensynthese zur Synthese erforderlich sind, gewährleisten. Für die Synthese an fester Phase ist ferner erforderlich, ein Linkersystem zur Anknüpfung des Monosacharidbausteins, bevorzugt über das anomere Zentrum zur Verfügung zu haben, daß kompatibel zu allen Reaktionsbedingungen ist und selektiv aktiviert werden kann. Eine solche Strategie ermöglicht die gezielte unterschiedliche Variation aller Funktionalitäten des Monosaccharidbausteins zu stabilen Endprodukten.

J. Carbohydr. Chem. 13 (8), 1159-1177 (1994) offenbart mit 4 verschiedenen Schutzgruppen versehene Monosaccharide, die einen Linker am anomeren Zentrum aufweisen, der eine Verknüpfung mit einem Träger erlaubt.

WO-A 94 19 360 beschreibt Verfahren zur trägergestützten Oligosacchäridsynthese unter Verwendung von geschützten Monosacchariden, die an einen festen Träger gebunden sind.

DE-A-32 20 427 beschreibt geschützte Galaktosederivate mit Linkern, wie sie auch unter anderen die vorliegende Anmeldung vorsieht.

Die Erfindung betrifft Verbindungen der Formel IIa, IIb und IIc,

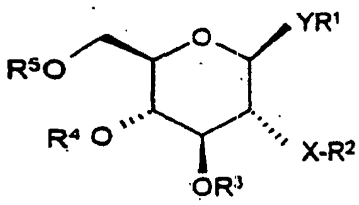 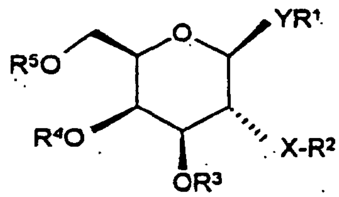 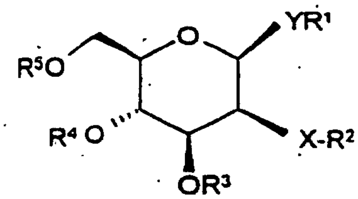

IIa IIb IIc

in denen bedeutet:

- Y S oder O, bevorzugt S
- X O oder N, bevorzugt O;
- $R^1$ eine Linkergruppe, die über eine kovalente Bindung mit einem durch ein Heteroatom, zum Beispiel N, O oder Cl, funktionalisierten Träger verknüpft werden kann;
- $R^2$ für den Fall, daß X gleich O ist, eine basenlabile Schutzgruppe wie Acetyl oder Benzoyl; für den Fall, daß X gleich N ist,
  eine basenlabile Schutzgruppe wie eine Phthaloylschutzgruppe, oder DDE (1-(4,4-Dimethyl-2,6-dioxocyclohexyliden-ethyl) oder NDE (2-Acetyl-4-nitro-indan-1,3-dion);
- $R^3$ eine Allylschutzgruppe;
- $R^4$ eine säurelabile Schutzgruppe , wie Ethoxyethyl oder SEM (2-(Trimethylsilyl)ethoxymethyl);
- $R^5$ eine Silylschutzgruppe, wie tert.Butyldimethylsilyl oder tert.
  Butyldiphenylsilyl.

Geeignete Silylschutzgruppen für $R^5$ sind im allgemeinen fluoridlabile Schutzgruppen, die stabiler, d.h. schwerer abspaltbar sind als ein Trimethylsilylrest.

Die Erfindung betrifft des weiteren Verbindungen der Formel IIb, in denen $R^3$ und $R^4$ zusammen eine acetal- oder ketalartige Schutzgruppe wie zum Beispiel Isopropyliden oder Benzyliden bedeuten und die übrigen Reste X, Y, $R^1$, $R^2$ und $R^5$ wie oben definiert sind.

Eine geeigneter Linkergruppe $R^1$ ist beispielsweise eine Gruppe der Formel III

$$-(C_1\text{-}C_6)\text{-alkylen-}[N\text{-}C(O)]_n\text{-}[(C_6\text{-}C_{12})\text{-arylen}]_p\text{-}(C_0\text{-}C_6)\text{-alkylen-}C(O)R^9 \qquad (III)$$

worin n und p 0 oder 1 bedeuten, wobei p und n nicht beide gleichzeitig 1 sein können;

- $R^9$ $OR^{10}$ oder $NR^{11}R^{11}$ bedeutet, wobei
- $R^{10}$ H, $(C_1\text{-}C_6)$-alkyl, $(C_1\text{-}C_6)$-Alkyl-$(C_6\text{-}C_{12})$-Aryl bedeutet, und
- $R^{11}$ unabhängig voneinander H, $(C_1\text{-}C_6)$-alkyl, $(C_1\text{-}C_6)$-Alkyl-$(C_6\text{-}C_{12})$-Aryl oder einen polymeren festen Träger bedeutet.

Die Verbindungen der Formel IIa, IIb oder IIc sind wertvolle Zwischenprodukte für die Herstellung von Verbindungen der Formel I,

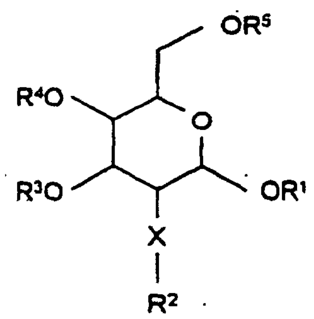

(I)

in welcher bedeuten:

$R^1$, $R^2$, $R^3$, $R^4$, $R^5$ unabhängig voneinander

1. Wasserstoff;
2. $(C_1-C_{12})$-Alkyl;
3. $(C_2-C_8)$-Alkenyl;
4. $(C_2-C_8)$-Alkinyl;
5. $(C_1-C_6)$-Alkylen-$(C_3-C_{10})$-cycloalkyl;
6. $(C_0-C_6)$-Alkylen-$(C_6-C_{12})$ -aryl; bevorzugt Phenyl oder Benzyl;
7. $(C_1-C_6)$-Alkoxy;
8. $(C_0-C_6)$ -Alkylen-CO-$R^8$;
9. $(C_1-C_6)$-Alkylen-$(C_1-C_9)$-heteroaryl;
10. Carbamoyl;
11. -C(O)$NR^6R^7$;
12. -C(O)$OR^6$;
13. einen wie unter 2.-12. definierten Rest, der im Alkylteil und/oder Aryl- bzw. Heteroarylteil einfach, zweifach oder mehrfach substitutiert ist mit einem Rest aus der Reihe $(C_1-C_6)$-Alkyl, $NO_2$, CN, Halogen, $CF_3$, oder $(C_1-C_6)$-Alkoxy;
14. einen wie unter 6. und 9. definierten Rest, der im Aryl- bzw. Heteroarylteil mit ein, zwei oder mehreren Halogenatomen substituiert ist;

$R^6$ und $R^7$ unabhängig voneinander:

1. Wasserstoff;
2. $(C_1-C_{12})$-Alkyl;
3. $(C_2-C_8)$-Alkenyl;
4. $(C_2-C_8)$-Alkinyl;
5. $(C_1-C_6)$-Alkylen-$(C_3-C_{10})$-cycloalkyl;
6. $(C_1-C_6)$-Alkylen-$(C_6-C_{12})$ -aryl; bevorzugt Benzyl;
7. $(C_2-C_6)$-Alkyloxy;
8. $(C_0-C_6)$-Alkylen -CO-$R^8$;
9. $(C_1-C_6)$-Alkylen-$(C_1-C_9)$ -heteroaryl;
10. $(C_0-C_6)$-Alkylen-$(C_1-C_6)$-alkoxy;
11. $(C_3-C_{10})$-Cycloalkyl;
12. $(C_6-C_{12})$-Aryl, bevorzugt Phenyl;

$R^8$ Wasserstoff, $(C_1-C_6)$-Alkyl, $(C_6-C_{12})$-Aryl oder $OR^{12}$;
$R^{12}$ Wasserstoff, $(C_1-C_6)$-Alkyl oder $(C_6-C_{12})$-Aryl;

oder
$R^2$ und $R^3$ zusammen oder $R^3$ und $R^4$ zusammen oder $R^4$ und $R^5$ zusammen $(C_1-C_3)$-Alkylen, das mit 1 oder 2 $(C_1-C_3)$-Alkylresten oder gegebenenfalls substituierten $(C_6-C_{12})$-Arylresten substituiert sein kann;

X N oder O;

mit der Maßgabe, daß $R^2$ nicht -C(O)$OR^6$ bedeutet, wenn X = O ist;
sowie deren physiologisch verträglichen Salze.

Unter ($C_6$-$C_{12}$)-Aryl wird beispielsweise Phenyl, Naphtyl oder Biphenyl verstanden.

Alkyl, Alkenyl, Alkinyl, Alkylen und davon abgeleitete Reste wie beispielsweise Alkoxy können geradkettig oder verzweigt sein wobei solche verzweigten Reste bevorzugt sind, bei denen sich die Verzweigungsstelle nicht direkt an der Anknüpfungstelle zum Monosaccharidgerüst befindet.

Halogen steht vorzugsweise für Fluor; Chlor oder Brom.

Ein Heteroaryl-Rest im Sinne der vorliegenden Erfindung ist der Rest eines monocyclischen oder bicyclischen ($C_3$-$C_9$)-Heteroaromaten, der im Ringsystem ein oder zwei N-Atorne und/oder ein S- oder ein O-Atom enthält. Zum Begriff "Heteroaromat" siehe Garrat, Vollhardt, Aromatizität, Stuttgart 1973, Seiten 131-153. .Beispiele geeigneter Heteroarylreste sind die Reste von Thiophen, Furan, Benzo[b]thiophen, Benzofuran, Pyrrol, Imidazol, Pyrazol, Pyridin, Pyrazin, Pyrimidin, Pyridazin, Indol, Chinolin, Isochinolin, Oxazol, Isoxazol, Thiazol, Isothiazol, Isobenzofuran, Indolizin, Isoindol, Indazol, Phthalazin, Naphthyridin, Chinoxalin, Chinazolin, Cinnolin und Furazan.

Aryl, Alkyl, Heteroaryl und davon abgeleitete Reste können wie oben angegeben einfach oder, falls chemisch möglich, auch mehrfach substituiert sein.

Geeignete polymere feste Träger sind beispielsweise quervernetzte Polystyrole (z.B. Aminometylpolystyrol (AMPS) oder Tentagel.

Chiralitätszentren können, wenn nicht anders angegeben, in der R- oder in der S-Konfiguration vorliegen. Die Erfindung betrifft sowohl die optisch reinen Verbindungen als auch Stereoisomerengemische wie Enantiomerengemische und Diasteromerengernische.

Als Salze kommen insbesondere Alkali- und Erdalkalisalze, Salze mit physiologisch verträglichen Aminen und Salze mit anorganischen oder organischen Säuren wie z. B. HCl, HBr, $H_2SO_4$, Maleinsäure, Fumarsäure in Frage.

Bei den oben genannten Verbindungen der Formel I und II bzw. IIa, IIb oder IIc handelt es sich um Derivate von Tetrahydropyran, die mit Hilfe der hier beschriebenen kombinatorischen Methode schnell und automatisiert in guten Ausbeuten und hohen Reinheiten an fester Phase synthetisiert werden können.

Verbindungen der Formel I lassen sich beispielsweise mit Hilfe von Zwischenprodukten der Formel II herstellen, die ein orthogonales Schutzgruppenmuster mit einer oder bevorzugt mehreren Schutzgruppen aus folgenden verschiedenen Klassen aufweisen:

- Basenlabile Schutzgruppen, wie die Acetat- oder Benzoylgruppe;
- Säurelabile Schutzgruppen, wie acetal - oder ketalartige Schutzgruppen wie die Ethoxyethylgruppe;
- Fluoridlabile Schutzgruppen, wie die tert-Butyldimethylsilyl oder tert-Butyldiphenylsilylgruppe;
- Übergangsmetalkatalysiert abspaltbare Schutzgruppe, wie die Allylgruppe;
- Schwefelhaltige Schutzgruppen, wie im Linkersystem.

Für die Synthese der selektiv geschützten Monosaccaridderivate gemäß Formel . IIa, IIb, oder IIc an fester Phase eignet sich die Anknüpfung über ein Thioglycosid oder ein O-Glycosid, insbesondere über ein Thioglykosid am anomeren Zentrum. Dabei weichen die verschiedenen Monosaccharide wie beispielsweise Glycose, Galaktose oder Mannose in der Konzeption der Schutzgruppen und der Reihenfolge ihrer Einführung nur geringfügig ab. Die Unterschiede in der Reaktivität der funktionellen Gruppen und die damit verbundenen Unterschiede in der Reihenfolge der Einführung der verschiedenen Schutzgruppen ist in der Kohlenhydratchemie ein bekanntes Problem.

Die Synthesestrategie zur Herstellung von Verbindungen der Formel I und IIa, IIb oder IIc ist am Beispiel des Glycosederivates in Schema 1 erläutert und ist mit den obengenannten geringfügigen, Variationen auch auf andere Monosaccharide wie z. B. Galaktose (s. Schema 3) oder Mannose ( s. Schema 4) übertragbar.

Anbindung eines Glykosesaccharids an das Trägermaterial (Schema 1)

Reaktion des bekannten 3-O-Allylgeschützten Glycose-β-Acetats 3 (K. Takeo et al. Carbohydrate Research 133, 1984, 275) mit dem aus 1 hergestellten Succinimidlinker 2 führt zu Verbindung 4. Das β-konfigurierte Thioglycosid 7 kann analog aus dem N-acylierten Cysteamin 6 durch Umsetzung mit 3 unter $BF_3$-Katalyse hergestellt werden. Die Deacteylierung von 4 und 7 ergibt einheitlich 8. Silylierung an der C-6 Hydroxylgruppe 9 und Einführung der Ethoxethylschutzgruppe ergibt 10. Verseifung der Imidstruktur in 10 und Kupplung der entstandenen Säure 11 an einen geeigneten Träger wie beispielsweise Aminomethylpolyrstyrol ergibt das mit dem geschützten Monosaccharid beladenen Harz 12 .

Reaktion an fester Phase (s. Schema 2)

Die Abspaltung und Umsetzung des geschützten Monosaccharids 12 an der festen Phase zu Verbindungen

der Formel I (13) ist beispielhaft in Schema 2 dargestellt. Die C-2 Hydroxylfunktion wird durch Umsetzung mit Hydrazin entacetyliert, die Hydroxylfunktion kann dann aktiviert werden durch Umsetzung mit Kalium-tert-butylat oder Phosphazene als Base (R. Schwesinger, H. Schlemper, Angew. Chem. 99, 1987, 1212-1214). Das aktivierte Derivat wird durch Elektrophile abgefangen. Eine analoge Umsetzung ist bei einer C-2 Aminofunktion durchführbar. Als Schutzgruppe findet hier beispielsweise die Fmoc-Gruppe Einsatz, die durch Piperidin abgespalten werden kann.
Die Abspaltung des Allylethers auf der C-3 Hydroxylgruppe erfolgt Zirconocen katalysiert (E. Negishi, Tetrahedron Lett. 1986, 27, 2829-2832; E. Negishi, Synthesis, 1988, 1-19). Die Funktionalisierung erfolgt analog wie oben beschrieben. Dadurch kann der Einsatz von starken Säuren, wie er bei anderen, dem Fachmann geläufigen Abspaltungsmethoden nötig wäre, vermieden werden und die Orthogonalität zu den anderen Schutzgruppen ist gewährleistet.
Alternativ zur basenkatalysierten Funktionalisierung kann die Allyetherschutzgruppe durch Reduktion mit Diiimin in eine Propylgruppe überführt werden (s. Hüning, H. R. Müller, W. Thier, Angew. Chem. 1965, 77, 368-377). Die C-4 Hydroxylfunktion kann durch Umacetalisierung abgespalten werden analog wie bei THP Acetalen angewandt (vgl. E. J. Corey, H. Niwa, J. Knolle, J. Am. Chem. Soc. 1978, 100, 1942-1943). Die Funktionalisierung erfolgt wie oben beschrieben. Die C-6 Hydroxylfunktion wird durch Umsetzung mit Fluoridionen desilyliert, analog zu C-2 wird die Umsetzung mit Elektrophilen durchgeführt. Die einzelnen Schritte können aufgrund der Kompatibilitäten in unterschiedlicher Reihenfolge durchgeführt werden.

Nach Beendigung der Funktionaliserung der verschiedenen Gruppen wird durch Umsetzung des polymer gebundenen selektiv geschützten Monosaccharids mit Brom/Di-tert-Butytpyridin die anomere Position aktiviert. Das 1-Bromderivat wird durch Umsetzung mit Alkohol in ein am anomeren Zentrum funktionalisiertes Derivat überführt.

Synthesesequenz zur Herstellung von Galaktosederivaten (s. Schema 3)

Ausgehend von Galaktose-Pentaacetat 14 wird durch Bortrifluorid -katalysierte Umsetzung mit 15 das Thioglycosid 16 dargestellt. Umsetzung mit Natriummethanolat ergibt unter Deacetylierung 17. Die selektive Silylierung von 17 erfolgt an der C-6 Hydroxylfunktion. Der Silylether 18 wird wird mit Dimethoxypropan in das Isopropyliden geschützte Derivat 19 überführt. Acetylierung durch Umsetzung mit Essigsäureanhydrid ergibt 20. Nach Abspaltung der Isopropylidenschutzgruppe wird die Dihydroxyverbindung mit Dibutylzinnoxid und Allylbromid in das an C-4 geschützte Allyletherderivat überführt. Einführung der Ethoxyethylschutzgruppe ergibt 21. Der Ester wird analog zur Glycose mit Lithiumhydroxid verseift.

Synthesesequenz zur Herstellung von Mannosederivaten (s. Schema 4)

Mannose -Pentaacetat 22 wird mit Thiol 6 zum unter Bortrifluorid Katalyse zum Thiomannosid 23 umgesetzt. Abspaltung der Acetatschutzgruppen durch Natriummethanolat ergibt 24. Umsetzung mit Dimethoxybenzaldehyd ergibt das Acetal 25. Reaktion mit Dibutylzinnoxid und Allylbromid führt zu dem 3-O- Allylester. Durch Acetylierung mit Acetanhydrid wird 26 dargestellt. Die Abspaltung des Ketals und anschließende selektive Silylierung an C-6 ergibt einen Silylether. Einführung der Ethoxyethylschutzgruppe ergibt 27. Der Ester in 27 wird analog zur Glycose mit Lithiumhydroxid verseift.

Schema 1

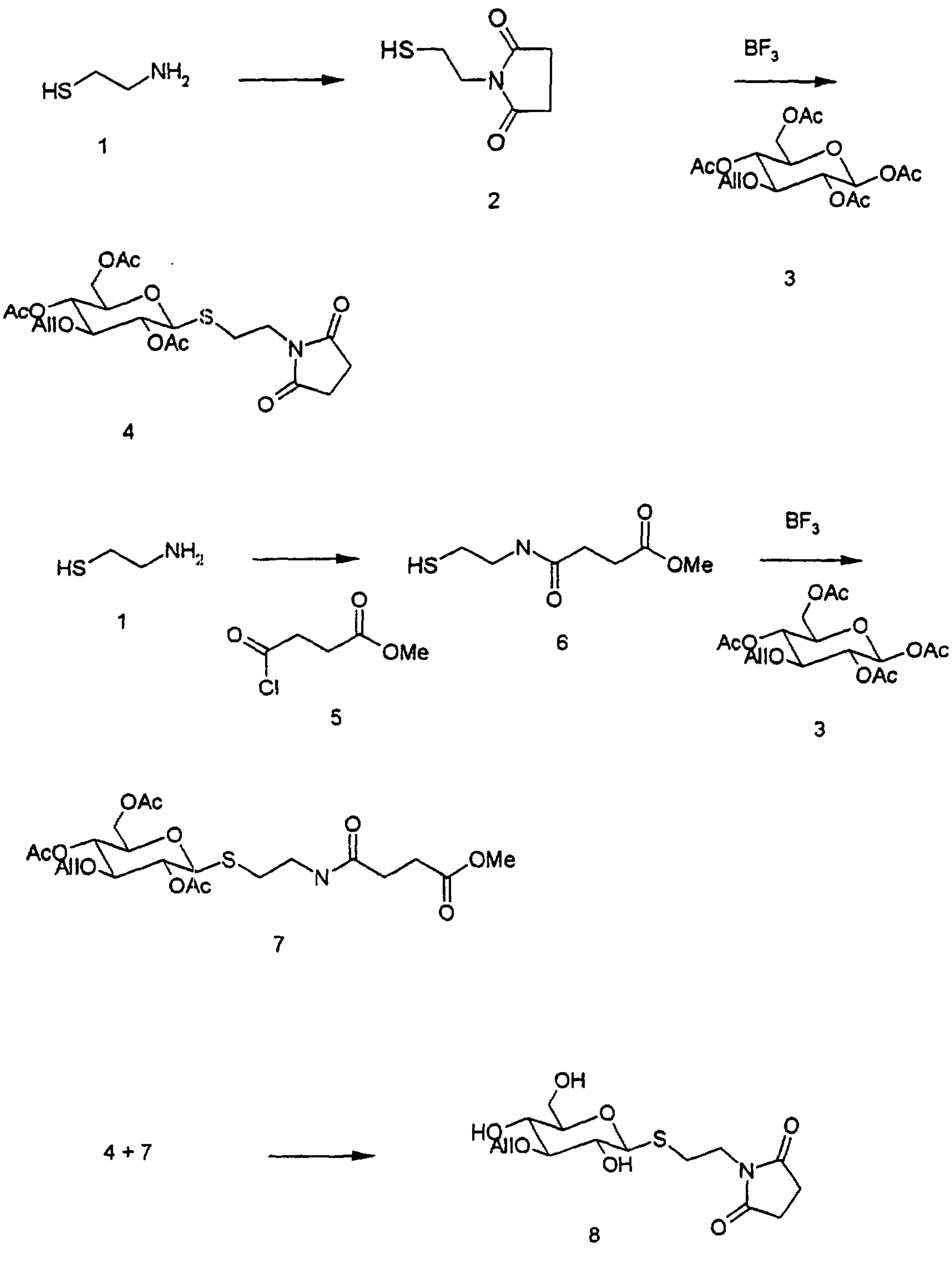


1 2 3 4

1 5 6 3 7

4 + 7 8

Schema 1 (Fortsetzung)

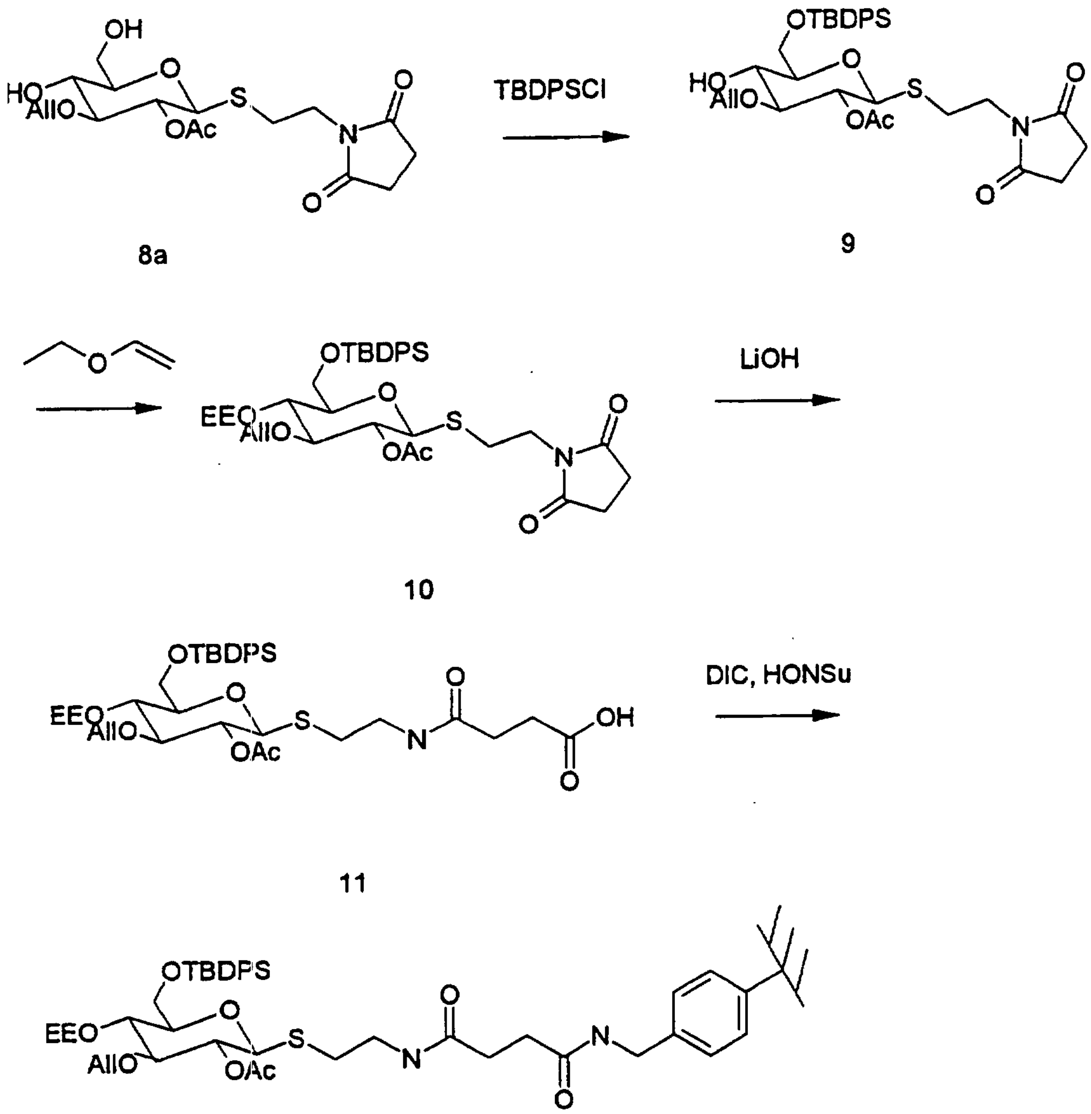


8a

9

10

11

12

Schema 2

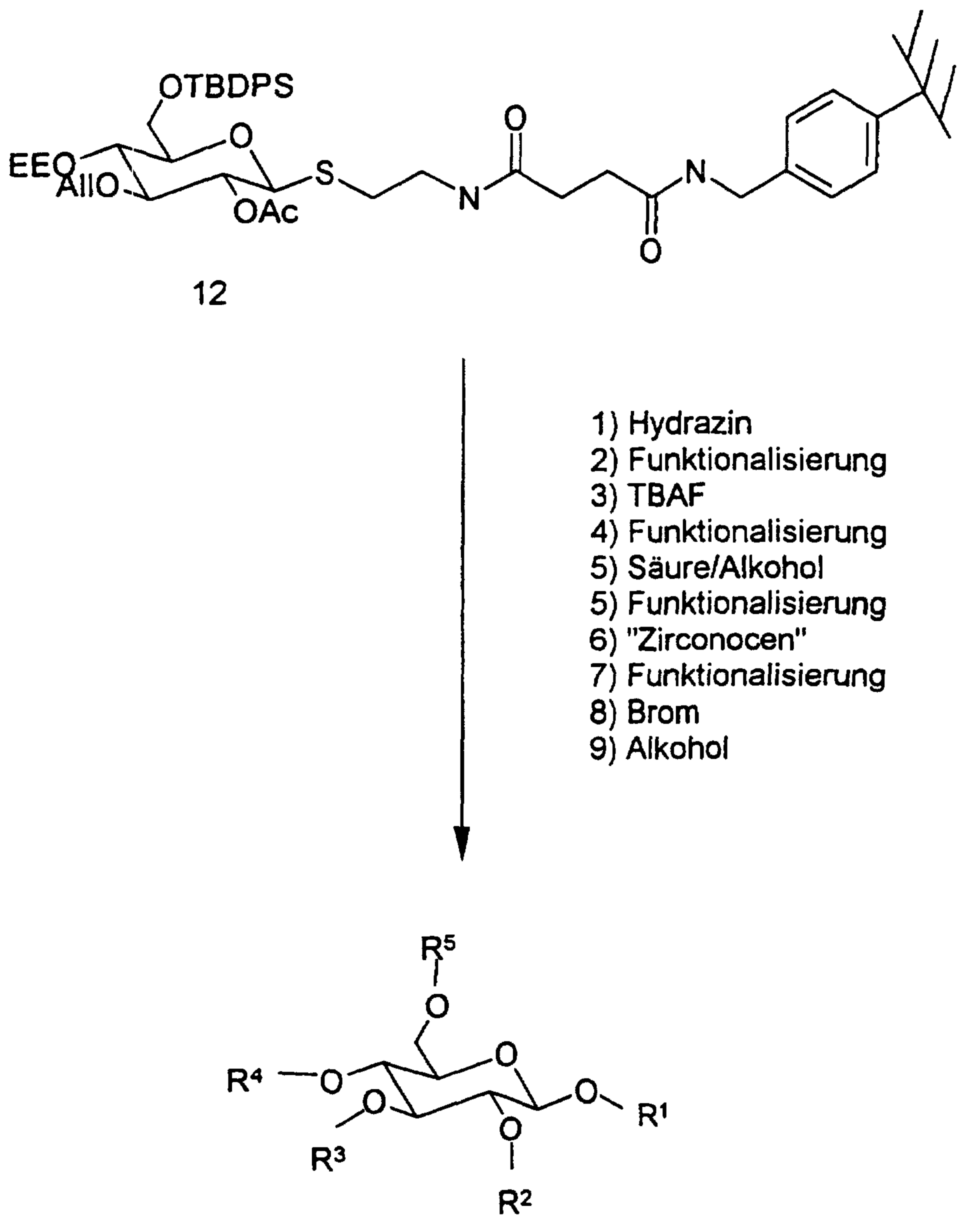


12

13

**Schema 3**

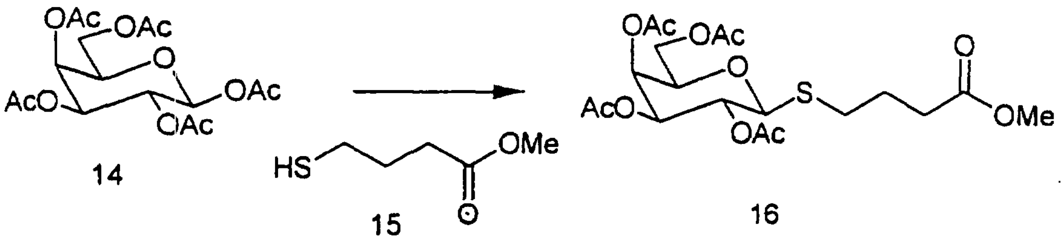

14 15 16

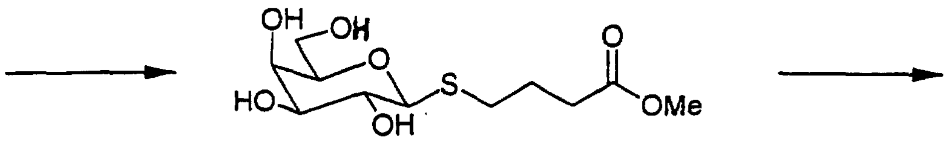

17

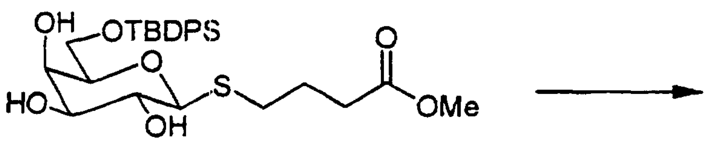

18

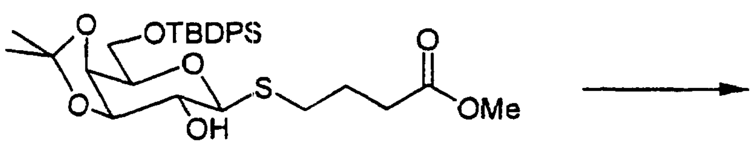

19

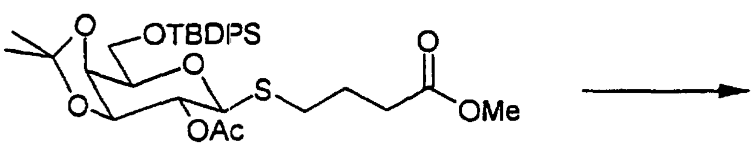

20

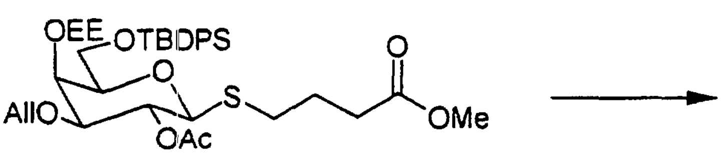

21

**Schema 4**

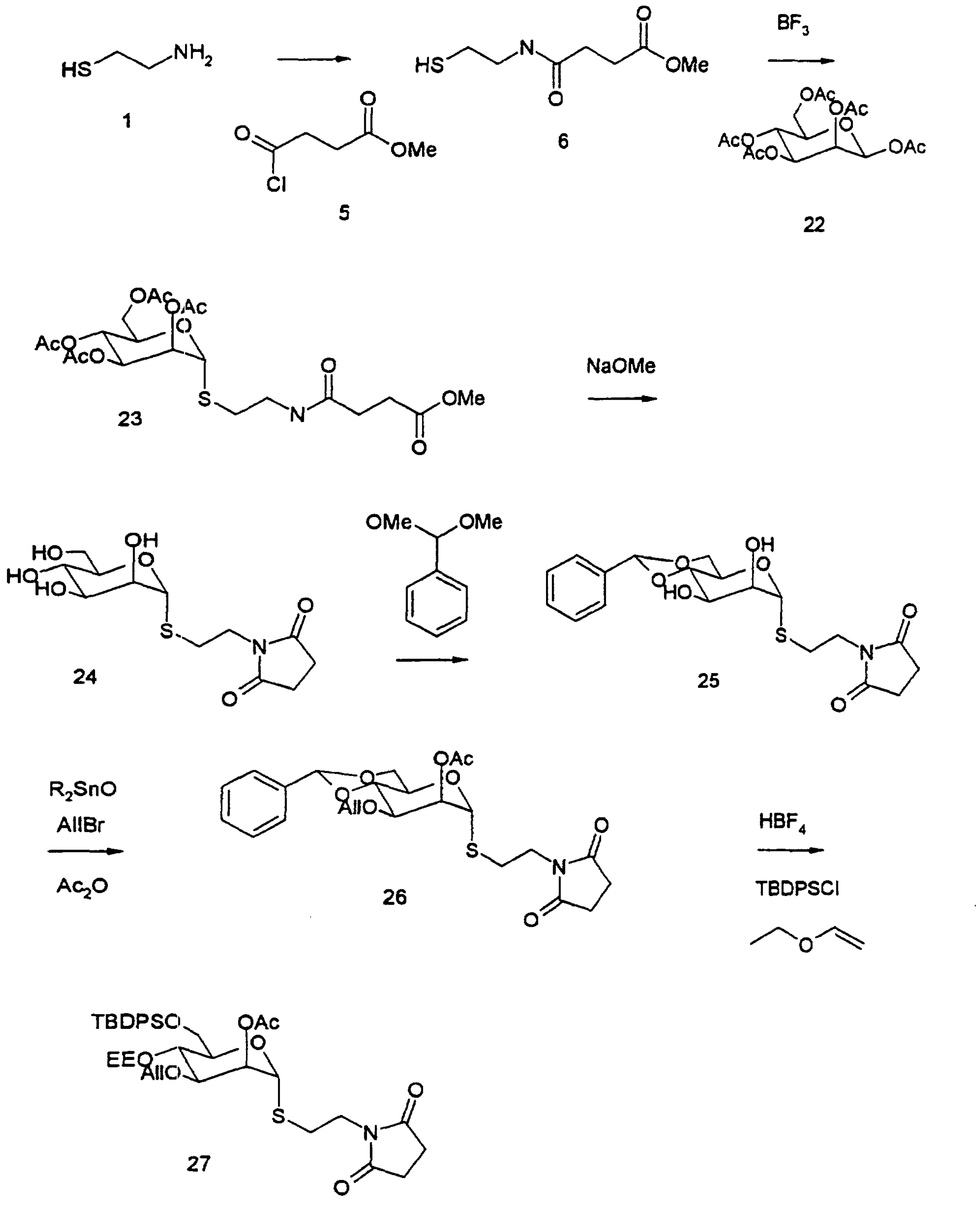


1 5 6 22 23 24 25 26 27

Kupplung eines Galactosylmercaptobuttersäuremethylesters auf einen aminofunktionalisierten polymeren Träger über die 1-Position (Schema 5)

Schema 5

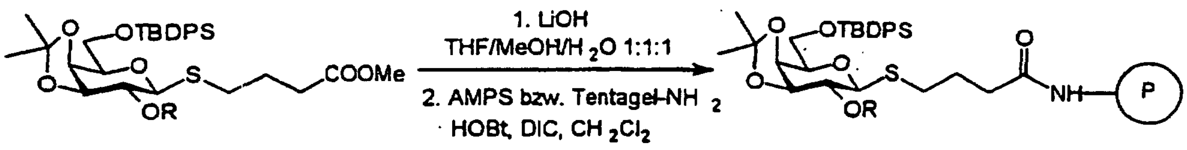


R = H 19
= Me 32

| R | P | |
|---|---|---|
| H | AMPS | 33 (0.78-0.81 mmol/g) |
| H | Tentagel | 34 (0.20 mmol/g) |
| Me | AMPS | 35 (0.78 mmol/g) |
| Me | Tentagel | 36 (0.27mmol/g) |

Die Verbindungen der Formel IIa, IIb und IIc sind aufgrund ihrer Multivalenz und ihrer definierten räumlichen Anordnung als Gerüstbausteine für biologische Mimetika, zum Beispiel Peptidmimetika geeignet und stellen ein wertvolles Hilfsmittel für die Herstellung und/oder Auffindung neuer Leitstrukturen und der Identifikation von potentiellen Wirkstoffen dar.

**Beispiele**

**Beispiel 1**

N-(2-Thioethyl)-succinimid 2

Eine Lösung von 24.2 g (0.21 mol) Cysteaminhydrochlorid 1 in 50 ml $H_2O$ wird mit 19.7 g (0.23 mol) $NaHCO_3$ versetzt und für 45 min gerührt. Anschließend wird i. Vak. eingeengt und der Rückstand in 100 ml Essigsäure aufgenommen. Nach Zugabe von 21.3 g (0.21 mol) Bernstein-säureanhydrid wird die Suspension 3 h unter Reflux erhitzt. Der beim Erkalten der Lösung entstandene Niederschlag wird abfiltriert, mit kalter Essigsäure gewaschen und das Filtrat i. Vak. vom Lösungsmittel befreit. Das Produkt wird durch Chromatographie an Kieselgel mit Petrolether/Ethylacetat (1:1) gereinigt. Ausbeute : 13.0 g (38%), farbloser, amorpher Feststoff.
$R_f$= 0.71 (EtOAc:HOAc = 30:1 v/v), Schmp.: 44-45□C.
$C_6H_9NO_2S$ (159.2) Ber.:C 45.26 H 5.70 N 8.80 S 20.14
Gef.: C 45.16 H 5.76 N 8.71 S 20.20

Beispiel 2

N-[2-S-(2',4',6'-Tri-O-acetyl-3'-O-allyl-D-glucopyranosyl)-thioethyl]-succinimid 4

Zu einer unter Argon auf 0°C abgekühlten Lösung von 2.0 g (5.15 mmol) des Acetats 3 und 980 mg (6.18 mmol) N-(2-Thioethyl)-succinimid 2 in 55 ml absol. $CH_2Cl_2$ werden 4 ml (32 mmol) Bortrifluoridetherat in 10 ml absol. $CH_2Cl_2$ zugetropft. Danach wird die Eiskühlung entfernt und die Reaktion bei Raumtemp. weitergeführt. Nach 16 h wird mit 100 ml $CH_2Cl_2$ versetzt und zweimal mit ges. $NaHCO_3$-Lösung extrahiert. Die organische Phase wird über $MgSO_4$ getrocknet und das Lösungsmittel i. Vak. entfernt. Das Produkt wird durch Chromatographi an Kieselgel mit Petrolether/Ethylacetat (2:1) gereinigt und anschließend das Produkt aus Ethylacetat/n-Pentan umgefällt. Ausbeute : 1.71 g (75%), farbloser, amorpher Feststoff.
$R_f$ = 0.27 (PE:EtOAc = 1:2 v/v), $[a]_D^2$ = - 41.8 (c 1.0, $CHCl_3$), Schmp.: 108°C.

| | | | | | |
|---|---|---|---|---|---|
| $C_{21}H_{29}NO_{10}S$ (487.5) | Ber. | C51.74 | H 6.00 | N2.87 | S 6.58 |
| | Gef. | C 51.64 | H 6.13 | N 2.88 | S 6.50 |

200 MHz-$^1$H-NMR ($CDCl_3$) :[ppm] = 5.80-5.61 (m 1H, $CH_2$=CH); 5.18-4.97 ($CH_2$=CH, H-2' & H-4'); 4.38 (d, 1H, $J_{2,1}$ = 10.01 Hz, H-1'); 4.19-3.94 (m, 4H, =CH-$CH_2$, H-6a/b'); 3.79-3.50 (m, 4H, H-3□, H-5' & $NCH_2$ Cya); 2.93-2.79

(m, 1 H, $SCH_2$ Cya); 2.76-2.61 (m, 1 H, $SCH_2$ Cya); 2.65 (s, 4H, $COCH_2$ Suc); 2.03, 2.02 (2x s, 9H, $CH_3$ Ac). 100.6 MHz-$^{13}$C-NMR ($CDCl_3$) : [ppm] = 176.6 ($COCH_2$); 169.1 ($COCH_3$); 134.2 ($CH_2$=CH); 116.8 ($CH_2$=CH); 83.3, 81.0, 76.3, 73.0, 71.1, 69.4 (C-1', C-2', C-3', C-4', C-5', =CH-$CH_2$); 62.3 (C-6'); 38.2 ($CH_2$CO Suc); 28.0 ($NCH_2$Cya); 27.2 ($SCH_2$ Cya); 20.8, 20.7, 20.6 ($CH_3$ Ac). Durch Chromatographie kann das entstandene a-Anomers abgetrennt werden. Ausbeute : 0.19 g (8%), farbloses Öl. $R_f$ = 0.33 (PE:EtOAc = 1.2 v/v),

Beispiel 3

N-(2-Thioethyl)-bernsteinsäuremonomethylesteramid 6

In 75 ml absol. Acetonitril werden unter Argon 11.0 g (96.8 mmol) Cysteaminhydrochlorid 1 suspendiert. Im Argongegenstrom werden hierzu unter Eiskühlung 60 ml (345.0 mmol) Hünigs Base langsam zugetropft. Nach 5 min werden 16.4 ml (130.0 mmol) Trimethylchlorsilan auf einmal zugegeben. Es wird 10 min bei O□C gerührt, bevor eine Lösung von 11.93 ml (96.8 mmol) Bernsteinsäuremonomethylesterchlorid 5 in 20 ml absol. Acetonitril zugetropft wird. Nach 30 min bei O□C und 2 h bei Raumtemp. wird die Lösung in 200 ml Eiswasser geschüttet wird und das Produkt zweimal mit je 200 ml Ethylacetat extrahiert wird. Die vereinigten organischen Phasen werden mit 30 ml 1 N HCl, 50 ml ges. $NaHCO_3$-Lösung sowie 50 ml ges. NaCl-Lösung gewaschen, über $MgSO_4$ getrocknet und i. Vak. das Lösungsmittel entfernt. Ausbeute : 13.1 g (71%), schwach gelbliches Öl.
$R_f$= 0.54 (EtOAc), $R_f$= 0.58 (EtOAc:HOAc = 30:1 v/v).

| $C_7H_{13}NO_3S$ (191.3) | | | | |
|---|---|---|---|---|
| Ber. | C 43.96 | H 6.85 | N 7.32 | S 16.76 |
| Gef. | C 43.97 | H 6.78 | N 7.65 | S 16.16 |

90 MHz-$^1$H-NMR ($CDCl_3$) : [ppm] = 3.62 (s, 3H, $OCH_3$); 3.37 (q, $J_{gem}$ = 6.26 Hz, $CH_2$N Cya); 2.80-2.17 (m, 6H, $SCH_2$, 2x $CH_2$CO).

Beispiel 4

N-[2-S-(2',4',6'-Tri-O-acetyl-3'-O-allyl-b-D-glucopyranosyl)-thioethyl]-bernsteinsäuremonomethylesteramid 7

In 120 ml absol. $CH_2Cl_2$ werden 6.0 g (15.5 mmol) 3 gelöst. Nach Zugabe von 3.32 g (18.5 mmol) des Thiols wird die Lösung unter Argon auf 0°C abgekühlt. Zu dieser Mischung wird eine Lösung von 17.5 ml (139 mmol) Bortrifluoridetherat in 20 ml absol. $CH_2Cl_2$ langsam zugetropft. Danach wird die Eiskühlung entfernt und 6 h bei Raumtemp gerührt. Das Reaktionsgemisch wird zweimal mit ges. $NaHCO_3$ -Lösung extrahiert, die organische Phase abgetrennt, über $MgSO_4$ getrocknet und das Lösungsmittel i. Vak. entfernt. Nach Chromatographie an Kieselgel mit Petrolether/Ethylacetat/HOAc (60:30:1) erhält man 6.8 g (85%) eines farblosen, amorphen Feststoffs.
$R_f$ = 0.44 (EtOAc), $R_f$ = 0.51 (Toluol:EtOH = 4:1 v/v), Schmp.: 75-77°C, = -5.3 (c 1, $CHCl_3$). $C_{22}H_{33}NO_{11}S$ (519.57)

| | | | | |
|---|---|---|---|---|
| Ber. | C 50.82 | H 6.40 | N 2.70 | S 6.17 |
| Gef. | C 50.74 | H 6.44 | N 2.76 | S 6.23 |

200 MHz-$^1$H-NMR ($CDCl_3$) : [ppm] = 6.33 ($t_b$, 1H, $J_{gem}$ = 5.13 Hz, NH); 5.80-5.61 (m, 1 H, $CH_2$=CH); 5.18-4.87 (m, 4H, $CH_2$=CH, H-2' & H-4'); 4.38 (d, 1H, $J_{2,1}$ = 9.76 Hz, H-1'); 4.11-4.00 (m, 2H, H-6'a/b); 3.62 (s, 3H, $CO_2CH_3$); 3.59-3.41 (m, 3H, H-3', H-4', H-5'); 3.38-3.24 (m, 2H, $CH_2$N Cya); 2.89-2.51 (m, 4H, $SCH_2$ Cya & $CH_2CO_2$ Suc); 2.43 (t, 2H, $J_{gem}$ = 6.41 Hz, $CH_2$CON Suc); 2.05, 2.02, 2.01 (3x s, 9H, $CH_3$ Ac).

Beispiel 5

N-[2-S-(3'-O-Allyl-D-glucopyranosyl)-thioethyl]-succinimid 8

Zu 7.76 g (14.93 mmol) Thioglycosid 7 (Rohprodukt) in 60 ml Methanol p.a. gelöst werden unter Argon 2.6 ml (2.6 mmol) einer 1 M NaOMe-Lösung in Methanol gegeben. Die Reaktionslösung wird 12 h bei 50°C gerührt, mit saurem Ionenaustauscher Amberlyst□ 15 neutralisiert (5 min), der Ionenaustauscher durch Filtration entfernt und mit Methanol gewaschen. Das Filtrat wird i. Vak. vom Lösungsmittel befreit. Nach Chromatographie an Kieselgel mit Toluol/EtOH (4:1) erhält man 4.86 g (86%) eines farblosen Öls, das nach einiger Zeit zu einem farblosen, amorphen Feststoff erstarrt. $R_f$ = 0.27 (Toluol:EtOH = 4:1 v/v), Schmp.: 98-99°C, = -37.2 (c 1.0, $CHCl_3$).
$C_{15}H_{23}NO_7S$ (361.4)

| | | | | |
|---|---|---|---|---|
| Ber. | C 49.85 | H 6.41 | N 3.88 | S 8.87 |
| Gef. | C 49.89 | H 6.62 | N 3.86 | S 8.83 |

400 MHz-$^1$H-NMR ($CDCl_3$) : [ppm] = 5.98-5.88 (m, 1 H, $CH_2$=CH); 5.27 (d, 1H, $J_{vic,trans}$ = 17.32 Hz, $CH_2$=CH); 5.16 (d, 1H, $J_{vic,cis}$ = 10.27 Hz, $CH_2$=CH); 4.43 (dd, 1 H, $J_{gem}$ = 12.62 Hz, $J_{vic}$ = 5.58 Hz, =CH-$CH_2$); 4.32 (d, 1 H, $J_{2,1}$ = 9.69 Hz, H-1'); 4.26 (dd, 1 H, $J_{gem}$= 12.91 Hz, $J_{vic}$ = 5.87 Hz, =CH-$CH_2$); 3.89-3.86 (m, 1 H, H-6'a); 3.79-3.68 (m, 3H, H-2', H-4' & H-6'b); 3.54 (t, 1H, $J_{2,3}$ = $J_{4,3}$ = 9.25 Hz, H-3'); 3.44-3.34 (m, 2H, $CH_2$N Cya); 3.29 (t, $J_{3,4}$ = $J_{5,4}$ = 8.51 Hz, H-5') □; 3.09, 3.03 (2x $s_b$, 2H, OH); 2.97-2.90 (m, 1 H, $SCH_2$ Cya), 2.83-2.75 (m, 1 H, $SCH_2$ Cya); 2.71 (s, 4H, $CH_2$CO Suc).

Beispiel 6

N-[2-S-(2'-O-Acetyl-3'-O-allyl-D-glucopyranosyl)-thioethyl]-succinimid 8a

Variante 1 : durch Deacetylierung des Succinimids 4

In 25 ml Methanol p.a. werden 1.15 g (2.36 mmol) Thioglycosids unter Argon auf 0°C abgekühlt. Zu den resultierenden Suspension werden 13 mg (0.24 mmol) NaOMe gegeben. Nach ca. 2 h löst sich der Niederschlag auf und nach weiteren 45 min wird die Reaktion durch Zugabe von saurem Ionenaustauscher Amberlyst□ 15 beendet. Es wird filtriert, der Ionenaustauscher mit Methanol gewaschen und die vereinigten Filtrate i. Vak. vom Lösungsmittel befreit. Nach Chromatographie an Kieselgel mit Toluol/EtOH (4:1) erhält man 895 mg (94%) eines farblosen, amorphen Feststoffs.

Variante 2 : durch Deacetylierung des acyclischen Derivates 7

Bei -15°C wird eine Lösung von 2.6 g (5.0 mmol) des Glycosids in 50 ml Methanol p.a. mit 27.0 mg (0.5 mmol) NaOMe versetzt. Nach 1 h läßt sich dünnschichtchromatographisch kein Umsatz erkennen, so daß weitere 27 mg (0.5 mmol) NaOMe zugegeben werden und die Temperatur auf 0°C erhöht wird. Da im Dünnschichtchromatogramm das Entstehen eines weiteren, polareren Produktes zu erkennen ist (Deacetylierung an der 2-Position), wird nach 6 h die Reaktion durch Zugabe von saurem Ionenaustauscher Amberlyst□ 15 abgebrochen. Die Lösung wird filtriert, der Ionenaustauscher mit Methanol gewaschen und i. Vak. das Lösungsmittel von den vereinigten Filtraten entfernt. Das Produkt wird durch Chromatographie an Kieselgel mit Toluol/EtOH (4:1) gereinigt. Ausbeute : 1.2 g (90%), farbloser, amorpher Feststoff.

| | | | | | |
|---|---|---|---|---|---|
| $C_{17}H_{25}NO_8S$ (403.5) | Ber. | C 50.61 | H 6.25 | N 3.47 | S 7.95 |
| | Gef. | C 49.85 | H 6.59 | N 3.87 | S 7.95 |

200 MHz-$^1$H-NMR ($CDCl_3$) : [ppm] = 5.92-5.73 (m, 1H, $CH_2$=CH); 5.24-5.08 (m, 2H, $CH_2$=CH); 4.86 (t, 1H, $J_{1,2}$ = $J_{3,2}$ = 9.52 Hz, H-2'); 4.37 (d, 1 H, $J_{2,1}$ = 7.76 Hz, H-1'); 4.25-4.08 (m, 2H, =CH-$CH_2$); 3.91-3.55 (m, 4H, H-3', H-4', H-6'a/b); 3.49-3.34 (m, 4H, H-5', $CH_2$N Cya & OH); 2.99-2.71 (m, 2H, $SCH_2$ Cya); 2.68 (s, 4H, $CH_2$CO Suc); 2.04 (s, 3H, $CH_3$ Ac).

Beispiel 7

N-[2-S-(2'-O-Acetyl-3'-O-allyl-6'-O-tert.-butyldiphenylsilyl- D-glucopyranosyl)-thioethyl]-succinimid 9

In 20 ml absol. $CH_2Cl_2$ wird 1.0 g (2.48 mmol) des Succinimids 8a gelöst, mit 583 mg (10.7 mmol) Imidazol, 0.74 ml (3.57 mmol) tert.-Butyldiphenylchlorsilan sowie einer Spatelspitze DMAP versetzt und bei Raumtemp. gerührt. Nach 2 h wird mit 100 ml $CH_2$ $Cl_2$ verdünnt und mit 50 ml 1N HCl und ges. NaCl-Lösung extrahiert. Die organische Phase wird über $MgSO_4$ getrocknet und i. Vak. das Lösungsmittel entfernt. Nach Chromatographie an Kieselgel mit Petrolether/Ethylacetat (1:1) erhält man 1.43 g (90%) eines farblosen Feststoffs.
$R_f$ = 0.47 (PE:EtOAc = 1:1 v/v), Schmp.: 39-40°C.

| | | | | | |
|---|---|---|---|---|---|
| $C_{33}H_{43}NO_8SSi$ (641.9) | Ber. | C 61.75 | H 6.75 | N 2.18 | S 5.00 |
| | Gef. | C 61.58 | H 7.12 | N 2.17 | S 4.81 |

400 MHz-$^1$H-NMR ($CDCl_3$) : [ppm] = 7.67-7.65 (m, 4H, PhSi); 7.42-7.33 (m, 6H, PhSi); 5.89-5.82 (m, 1 H, $CH_2$=CH); 5.24 (dd, 1 H, $J_{vic,trans}$ = 17.22 Hz, $J_{gem}$ = 1.58 Hz, $CH_2$=CH); 5.14 (d, 1H, $J_{vic,cis}$ = 10.41 Hz, $CH_2$=CH); 4.91 (t, 1H, $J_{1,2}$ = $J_{3,2}$ = 9.56 Hz, H-2'); 4.42 (d, 1H, $J_{2,1}$ = 9.97 Hz, H-1'); 4.25-4.14 (m, 2H, =CH-$CH_2$); 3.90 (d, 2H, J = 4.51 Hz, H-6'a/b); 3.76 (t, 1 H, $J_{3,4}$ = $J_{5,4}$ = 9.21 Hz, H-4'); 3.70-3.63 (m, 2H, H-3' & H-5'); 3.47-3.41 (m, 2H, $CH_2$N Cya); 2.89 (s, 1H, OH); 2.87-2.81 (m, 1 H, $SCH_2$ Cya); 2.75-2.62 (m, 1 H, $SCH_2$ Cya); 2.61 (s, 4H, $CH_2$CO Suc); 2.07 (s, 3H, $CH_3$ Ac); 1.01 (s, 9H, $CH_3$ tBuSi).

Beispiel 8

N-[2-S-(2'-O-Acetyl-3'-O-allyl-6'-O-tert.-butyldiphenylsilyl-4'-O-(1"-(R/S)-ethoxyethyl)-D-glucopyranosyl)-thioethyl]-succinimid 10

Eine Lösung von 1.26 g (1.96 mmol) des Succinimids 9 in 20 ml absol. $CH_2Cl_2$ wird mit 0.94 ml (9.80 mmol) Ethylvinylether und 246 mg (0.98 mmol) Pyridinium-toluol-4-sulfonat versetzt und bei Raumtemp. gerührt. Nach 3 h wird die Reaktionslösung mit 50 ml $CH_2Cl_2$ verdünnt und zweimal mit je 30 ml ges. $NaHCO_3$-Lösung extrahiert. Die organische Phase wird über $MgSO_4$ getrocknet und i. Vak. das Lösungsmittel entfernt.
Ausbeute : 1.37 g (98%), farbloses Öl.
$R_f$ = 0.59 (PE:EtOAc = 1:1 v/v),

| | | | | | |
|---|---|---|---|---|---|
| $C_{37}H_{51}NO_9SSi$ (714.0) | Ber. | C 62.25 | H 7.20 | N 1.96 | S 4.49 |
| | Gef. | C 61.61 | H 6.86 | N 2.00 | S 5.06 (Rohprodukt) |

400 MHz-$^1$H-NMR ($CDCl_3$) : [ppm] = 7.70-7.65 (m, 4H, PhSi); 7.40-7.33 (m, 6H, PhSi); 5.85-5.81 (m, 1 H, $CH_2$=CH); 5.25-5.08 (m, 2H, $CH_2$=CH); 4.93-4.87 (m, 1 H, H-2□); 4.80 (q, □0.5H, $J_{gem}$ = 5.29 Hz, $CHCH_3$ EE); 4.65 (q, □0.5H, $J_{gem}$ = 5.28 Hz, $CHCH_3$ EE); 4.42 (d, 1 H, $J_{2,1}$ = 9.68 Hz); 4.23 (dd, 1 H, $J_{gem}$ = 12.62 Hz, $J_{vic}$ = 5.57 Hz, =CH-$CH_2$); 4.16 (dd, 1H, $J_{gem}$ = 12.62 Hz, $J_{vic}$ = 5.58 Hz, =CH-$CH_2$); 3.90-3.86 (m, 2H, H-6□a/b); 3.75 (t, 1 H, $J_{3,4}$ = $J_{5,4}$ = 9.25 Hz, H-4□); 3.68-3.58 (m, 3H, H-3□ & $CH_2$N Cya); 3.52-3.41 (m, 3H, H-5□ & $CH_3CH_2O$ EE); 2.86-2.81 (m, 1 H, $SCH_2$ Cya); 2.74 (m, 1 H, $SCH_2$ Cya); 2.60 (s, 4H, $CH_2$CO Suc); 2.06 (s, 3H, $CH_3$ Ac); 1.28-1.11 (m, □4.5H, $CHCH_3$ & $CH_3CH_2O$ EE); 1.00 (s, 9H, $CH_3$ tBuSi); 0.89 (t, □1.5H, J = 6.90 Hz, $CH_3CH_2O$ EE).

Beispiel 9

$N^1$-[2-S-(2'-O-Acetyl-3'-O-allyl-6'-O-tert.-butyldiphenylsilyl- D-glucopyranosyl)-thioethyl]-$N^4$-benzyl-bersteinsäurediamid

In 30 ml THF werden 2.8 g (4.36 mmol) des Succinimids 9 gelöst und auf 0°C abgekühlt. Nach Zugabe von 15 mg LiOH (4.8 mmol) in 10 ml $H_2O$ wird 1.5 h bei 0°C gerührt, anschließend mit 1 N HCl auf pH = 2.5 angesäuert und zweimal mit je 50 ml CH $_2Cl_2$ extrahiert. Die vereinigten organischen Phasen werden über $MgSO_4$ getrocknet und i. Vak. vom Lösungsmittel befreit.

Das so erhaltene Rohprodukt wird in 30 ml absol. $CH_2Cl_2$ mit 0.96 ml (8.72 mmol) Benzylamin, 767 mg (6.54 mmol) N-Hydroxysuccinimid sowie 900 mg (4.36 mmol) N,N'-Dicyclohexyl-carbodiimid versetzt. Nach 16 h wird der ausgefallene Harnstoff abfiltriert und mit $CH_2Cl_2$ gewaschen. Die vereinigten Filtrate mit 50 ml 1N HCl sowie 50 ml ges. $NaHCO_3$-Lösung extrahiert. Die organische Phase wird über $MgSO_4$ getrocknet und i. Vak. das Lösungsmittel entfernt. Das Produkt wird durch Chromatographie an Kieselgel mit Petrolether/Ethylacetat-Gemischen gereinigt.
Ausbeute : 2.13 g (67%), farbloser, amorpher Feststoff.
$R_f$= 0.53 (EtOAc), $R_f$= 0.33 (EtOAc:PE:HOAc = 30:30:1 v/v), Schmp.: 46-47°C,

| | | | | | |
|---|---|---|---|---|---|
| $C_{40}H_{52}N_2O_8SSi$ (735.0) | Ber. | C 65.37 | H 7.13 | N 3.81 | S 4.36 |
| | Gef. | C 63.68 | H 6.83 | N 3.71 | S 4.45 |

200 MHz-$^1$H-NMR ($CDCl_3$) : [ppm] = 7.77-7.64 (m, 4H, PhSi); 7.50-7.28 (m, 6H, PhSi); 7.25-7.13 (m, 5H, Ph Amid); 6.52 (t, 1H, $J_{gem}$ = 5.37 Hz, NH); 6.34 (t, 1 H, $J_{gem}$ = 5.37 Hz, NH); 5.95-5.76 (m, 1 H, $CH_2$=CH); 5.28-5.12 (m,

2H, $CH_2$=CH); 4.90 (t, 1 H, $J_{1,2}$ = $J_{3,2}$ = 9.53 Hz, H-2□); 4.46-4.36 (m, 3H, H-1□, $CH_2$-Ph Amid); 4.28-4.04 (m, 2H, =CH-$CH_2$); 3.91-3.89 (m, 2H, H-6□a/b); 3.69 (t, 1H, $J_{3,4}$ = $J_{5,4}$ = 9.28 Hz, H-4□); 3.52-3.26 (m, 5H, H-3□, H-5□, $CH_2N$ Cya); 2.94 (s, 1H, OH); 2.85-2.58 (m, 2H, $SCH_2$ Cya); 2.47-2.34 (m, 4H, $CH_2CO$ Suc); 2.08 (s, 3H, $CH_3$ Ac); 1.03 (s, 9H, $CH_3$ tBuSi).

Darstellung des Galactose-Bausteines

Beispiel 10

4-S-(2',3',4',6'-tetra-O'-acetyl-β-D-galactopyranosyl)-mercaptobuttersäuremethylester 16

Eine Lösung von 12 g (30 mmol) 1,2,3,4,6-Penta-O-acetyl-galactose 14 und 5.5 g Mercaptobuttersäuremethylester 15 in 150 ml abs. Dichlormethan wird mit 10 g ausgeheiztem Molsieb 4Å wird 1 h vorgerührt. Man kühlt dann auf 0 □C ab und tropft 30 ml Bortrifluorid-Ethyletherat in 30 ml abs. Dichlormethan zu der Reaktionsmischung. Anschließend läßt man auf Raumtemp. kommen. Nach 24 h saugt man über Celite ab und rührt die organische Phase dreimal mit je 300 ml ges. $NaHCO_3$-Lösung Anschließend wird mit 600 ml Wasser gewaschen, über $MgSO_4$ getrocknet und vom Lösungsmittel befreit. Man reinigt das Produkt durch Chromatographie an Kieselgel (Laufmittel Petrolether/Ethylacetat 2:1, Säule 20 ' 8 cm). Ausb. 12.5 g (90 %), gelber Sirup, Rf = 0.46 (Petrolether/Ethylacetat 1:1)

Beispiel 11

4-S-(6-O'-tert-Butyldiphenylsilyl-β-D-galactopyranosyl)-mercaptobuttersäuremethylester 18

Man löst 4.21 g (9.1 mmol) 16 in 40 ml abs. Methanol und fügt 0.098 g (1.82 mmol) Natriummethanolat zu. Nach 4 h neutralisiert man mit saurem Ionentauscher Amberlite□ IR 120. Das Harz wird abfiltriert und mit Methanol gewaschen. Nach dem Abdestillieren des Lösungsmittels i. Vak. und Trocknen i. Hochvak. erhält man quant. S-b-D-galactopyranosyl-mercaptobuttersäuremethylester 17 als farbl. Feststoff, $R_F$ = 0.56 (Chloroform/Methanol 2: 1). Das Rohprodukt wird in 20 ml DMF gelöst und mit 1.24 g (18.1 mmol) Imidazol und 3.25 ml (12.7 mmol) tert-Butyldiphenylsilylchlorid versetzt. Man läßt 5 h bei Raumtemp. rühren und bricht dann die Reaktion durch Zusatz von 10 ml Wasser ab. Nach 10 min verdünnt man mit 60 ml Dichlormethan und wäscht dreimal mit je 40 ml Wasser. Die org. Phase wird über $MgSO_4$ getrocknet und nach Filtration i. Vak. eingedampft. Der Rückstand wird an Kieselgel chromatographisch gereinigt. Ausb. 4.44 g (92 %) ,
$R_F$ = 0.23 (Petrolether/Ethylacetat 1:1), 0.63 (Ethylacetat/Essigsäure 30:1). □ 400 MHz-$^1$H-NMR ($CDCl_3$): d[ppm] = 7.98□7.64; 7.40□7.36 (m, 10H, $SiPh_2$), 4.25 (d, 1 H, $J_{1,2}$ = 9.6 Hz, 1-H), 4.10 (s, 1 H, 4-H), 3.88 (dd, 1 H, $J_{gem}$ = 10.4 Hz, $J_{6a,5}$ = 6.3 Hz, 6-$H_a$), 3.84 (dd, 1H, $J_{gem}$ = 10.9 Hz, $J_{6b,5}$ = 5.3 Hz, 6-$H_b$), 3.69 (dd, 1 H, $J_{2,1}$ = 9.4 Hz, $J_{2,3}$ = 9.2 Hz, 2-H), 3.59 (s, 3H, $COOCH_3$), 3.56 (d, 1H, $J_{3,4}$ = 3.1 Hz, 3-H), 3.52 (dd, 1 H, $J_{5,6a}$ = 5.7 Hz, $J_{5,6b}$ = 5.0 Hz, 5-H), 3.06 ($s_{br}$, □ 3H, OH), 2.72 (dt, 1H, $J_{gem}$ = 13.9 Hz, $J_{vic}$ = 7.0 Hz, $SCH_a$), 2.66 (dt, 1 H, $J_{gem}$ = 13.9 Hz, $J_{vic}$ = 7.0 Hz, $SCH_b$), 2.40-2.36 ($m_c$, 2H, $CH_2COOMe$), 1.95-1.89 ($m_c$, 2H, $SCH_2CH_2$), 1.02 (s, 9H, $SiC(CH_3)_3$). □ 100.6 MHz-$^{13}$C-NMR ($CDCl_3$): d[ppm] = 173.5 (COOMe), 135.6; 135.5; 133.1; 133.0; 129.9; 127.8 ($SiPh_2$), 86.0; 78.4; 75.0; 70.5; 69.1; 63.2 (C-1 □ C-6), 51.5 ($COOCH_3$), 32.7 ($SCH_2$), 29.2 ($CH_2COOMe$), 26.8 ($SiC(CH_3)_3$), 25.3 ($SCH_2CH_2$), 19.1 ($SiC(CH_3)_3$).

Beispiel 12

4-S-(6-O'-tert-butyldiphenylsilyl-3,4-O'-isopropyliden-β-D-galactopyranosyl)-mercaptopropionsäuremethylester 19

Man löst 4.15 g (7.78 mmol) 18 in 35 ml Acetondimethylacetal und rührt nach Zugabe von 28 mg p-TsOH 4 h bei Raumtemp. Anschließend neutralisiert man mit Triethylamin. Die Lösung wird i. Vak. eingeengt und der ölige Rückstand durch Chromatographie an Kieselgel (Laufmittel Petrolether/Ethylacetat 2:1, Säule 20 ' 5 cm) von Verunreinigungen befreit.
Ausb. 4.03 g (90 %), farbloses Öl, $R_F$ = 0.54 1:1 (Petrolether/Ethylacetat 1:1). □ 400 MHz-$^1$H-NMR ($CDCl_3$): d[ppm] = 7.70□7.66, 7.44□7.33 (m, 10H, Ph-H), 4.31 (dd, 1 H, $J_{4,3}$ = 5.3 Hz, $J_{4,5}$ = 1.3 Hz, 4-H), 4.21 (d, 1 H, $J_{1,2}$ = 10.3 Hz, 1-H), 4.04 (dd, 1 H, $J_{3,2}$ = 7.0 Hz, $J_{3,4}$ = 5.6 Hz, 3-H), 3.90 (dd, 1 H, $J_{gem}$ = 10.8 Hz, $J_{6a,5}$ = 1.2 Hz, 6-$H_a$), 3.88 (dd, 1H, $J_{gem}$ = 10.8 Hz, $J_{6b,5}$ = 4.8 Hz, 6-$H_b$), 3.87□3.85 (m, 1H, 5-H), 3.60 (s, 3H, $COOCH_3$), 3.52 (dd, $J_{2,1}$ = 8.8 Hz, $J_{2,3}$ = 8.5 Hz, 2-H), 2.75 (dt, 1 H, $J_{gem}$ = 13.2 Hz, $J_{vic}$ = 7.0 Hz. $SCH_a$), 2.35 (dt, 1 H, $J_{gem}$ = 13.2 Hz, $J_{vic}$ = 7.0 Hz, $SCH_b$), 2.42-2.39 ($m_c$, 2H, $CH_2COOMe$), 1.96□1.89 ($m_c$, 2H, $SCH_2CH_2$), 1.50 (s, 3H, $C(CH_3)_2$), 1.34 (s, 3H, $C(CH_3)_2$), 1.03 (s, 9H, $C(CH_3)_3$). □100.6 MHz-$^{13}$C-NMR ($CDCl_3$), d[ppm] = 173.3 (COOMe), 135.6; 135.5; 129.7; 127.7; 127.6 (C-Ph), 110.0 ($C(CH_3)_3$), 85.6; 79.1; 77.3; 73.3; 72.4; 62.7 (C-1 - C-6), 51.5 ($COOCH_3$), 32.5 ($SCH_2$), 29.4 ($CH_2COOMe$), 28.2

$(C(CH_3)_2)$, 26.8 $(C(CH_3)_3)$, 26.2 $(C(CH_3)_2)$, 25.2 $(SCH_2CH_2)$.

Beispiel 13

4-S-(2-O'-acetyl-6-O'-tert-butyldiphenylsilyl-3,4-O'-isopropyliden-β-Dgalactopyranosyl)-mercaptobuttersäuremethylester 20

Eine Lösung von 27.6 g (48 mmol) 19 in 100 ml Acetanhydrid-Pyridin (1:1) wird bei Raumtemp. 5 h gerührt. Anschließend engt man i. Hochvak. ein und nimmt in 100 ml Dichlormethan auf. Die Lösung wird mit je 50 ml 0.5 N HCl, ges. $NaHCO_3$-Lösung und Wasser gewaschen. Nach Trocknen über $MgSO_4$ befreit man i. Vak. vom Lösungsmittel. Eine Chromatographie an Kieselgel (Laufmittel Petrolether/Ethylacetat 4:1, Säule 30 ' 10 cm) liefert die Titelverbindung. Ausb. 24.3 g (82%), farbloses Öl,

$R_F$ = 0.66 (Petrolether/Ethylacetat 1:1). □ 400 MHz-$^1$H-NMR ($CDCl_3$): d[ppm] = 7.70□7.66, 7.43□7.33 (m, 10H, Ph-H), 4.98 (dd, $J_{2,1}$ = 10.0 Hz, $J_{2,3}$ = 7.3 Hz, 2-H), 4.34 (d, 1 H, $J_{4,3}$ = 5.3 Hz, 4-H), 4.31 (d, 1 H, $J_{1,2}$ = 10.3 Hz, 1-H), 4.15 (dd, 1 H, $J_{3,2}$ = 7.3 Hz, $J_{3,4}$ = 5.2 Hz, 3-H), 4.10□3.86 (m, 3H, 6-$H_{a,b}$, 5-H, 3.58 (s, 3H, $COOCH_3$), 2.72 (dt, 1 H, $J_{gem}$ = 12.9 Hz, $J_{vic}$ = 7.0 Hz, $SCH_a$), 2.60 (dt, 1H, $J_{gem}$ = 12.6 Hz, $J_{vic}$ = 7.0 Hz, $SCH_b$), 2.41□2.32 ($m_c$, 2H, $CH_2COOMe$), 2.08 (s, 3H, $COCH_3$), 1.93-1.83 ($m_c$, 2H, $SCH_2CH_2$), 1.53 (s, 3H, $C(CH_3)_2$), 1.33 (s, 3H, $C(CH_3)_2$), 1.03 (s, 9H, $C(CH_3)_3$). □ 100.6 MHz-$^{13}$C-NMR ($CDCl_3$): d[ppm] = 173.2 (COOMe), 169.5 (COMe), 135.5; 135.5; 133.4; 133.3; 129.7; 127.6; 127.6 (C-Ph), 110.2 $(C(CH_3)_2)$, 82.7; 77.4; 76.7; 73.4; 71.7; 62.6 (C-1 - C-6), 51.4 $(COOCH_3)$, 32.6 $(SCH_2)$, 29.1 $(CH_2COOMe)$, 27.8 $(C(CH_3)_2)$, 26.8 $(C(CH_3)_3)$, 26.3 $(C(CH_3)_2)$, 24.9 $(C(CH_3)_3)$, 20.9 $(COCH_3)$, 19.2 $(SCH_2CH_2)$.

Beispiel 14

4-S-(3-O'-allyl-2-O'-acetyl-4-O'-[1-(R/S)-ethoxyethyl]-6-O'-tert-butyldiphenylsilyl-β-Dgalactopyranosyl)-mercaptobuttersäuremethylester 21

a) 4-S-(2-O'-acetyl-6-O'-tert-butyldiphenylsilyl-β-D-galactopyranosyl)-mercaptobuttersäuremethylester

Eine Lösung von 23.97 g (38.86 mmol) 20 in 370 ml $CHCl_3$ wird mit 0.35 g (1.84 mmol) p-Toluolsulfonsäure und 22.70 ml (270 mmol) Ethandithiol zum Rückfluß erhitzt. Nach 5 h kühlt man auf Raumtemp. ab und wäscht mit je 50 ml ges. Natriumhydrogencarbonat-Lösung, 0.5 N HCl und Wasser. Die organische Phase wird über Magnesiumsulfat getrocknet. Das Produkt wird durch Chromatographie an Kieselgel (Laufmittel Petrolether/Ethylacetat 1:1) rein gewonnen. Ausb. 16.66 g (74 %), farbloses Öl,
$R_F$ = 0.16 (Petrolether/Ethylacetat 2:1). □ 400 MHz-$^1$H-NMR ($CDCl_3$): d[ppm] = 7.68-7.63, 7.42-7.34 (m, 10H, Ph-H), 5.06 (dd, $J_{2,1}$ = 9.6 Hz = $J_{2,3}$ = 9.6 Hz, 2-H), 4.31 (d, 1H, $J_{1,2}$ = 9.9 Hz, 1-H), 4.11 (d, 1 H, $J_{4,3}$ = 5.3 Hz, 4-H), 3.92-3.84 (m, 2H, 6-$_{Ha,b}$), 3.61 (d, 1 H, $J_{3,4}$ = 3.4 Hz, 3-H), 3.58 (s, 3H, $COOCH_3$), 3.50 (t, 1 H, $J_{5,6}$ = 5.5 Hz, 5-H), 2.73 (dt, 1 H, $J_{gem}$ = 13.0 Hz, $J_{vic\,=}$ 7.2 Hz, $SCH_a$), 2.59 (dt, 1H, $J_{gem}$ = 13.0 Hz, $J_{vic\,=}$ 7.2 Hz, $SCH_b$), 2.40-2.16 ($m_c$, 2H, $CH_2COOMe$), 2.09 (s, 3H, $COCH_3$), 2.01-1.81 ($m_c$, 2H, $SCH_2CH_2$), 1.03 (s, 9H, $C(CH_3)_3$). □ 100.6 MHz-$^{13}$C-NMR ($CDCl_3$), d[ppm] = 173.4 (COOMe), 170.8 (COMe), 135.5; 135.4; 132.9; 132.7; 129.9; 127.8 (C-Ph), 83.2; 78.1; 73.7; 71.2; 69.6; 63.3 (C-1 - C-6), 51.5 $(COOCH_3)$, 32.6 $(SCH_2)$, 28.8 $(CH_2COOMe)$, 26.8 $(C(CH_3)_3)$, 25.1 $(C(CH_3)_3)$, 20.9 $(COCH_3)$ 19.1 $(SCH_2CH_2)$.

b) 4-S-(3-O'-allyl-2-O'-acetyl-6-O'-tert-butyldiphenylsilyl-β-D-galactopyranosyl)-mercaptobuttersäuremethylester

Man erhitzt eine Mischung von 5.7 g (9.8 mmol) der Verbindung aus Bsp. 14a) und 3.3 g (9.43 mmol) Dibutylzinnoxid in 70 ml Benzol 8 h am Wasserabscheider zum Rückfluß. Anschließend destilliert man 35 ml Benzol ab und versetzt mit 1.76 g (9.43 mmol) Tetrabutylammoniumbromid und 1.35 ml (15.6 mmol) Allylbromid. Die Lösung wird 16 h bei 50°C gerührt. Nach Zusatz von 5 ml Methanol wird i. Vak. weitgehend eingeengt. Man nimmt in 50 ml Dichlormethan auf und wäscht mit dreimal mit je 10 ml Wasser. Nach Trocknen über $MgSO_4$ wird das Lösungsmittel i. Vak. entfernt. Das Rohprodukt wird durch Chromatographie (Laufmittel Petrolether/Ethylacetat 4:1, Säule 15 ' 5 cm).
an Kieselgel gereinigt. Ausb. 3.79 g (63%) gelbliches Öl,
$R_F$ = 0.34 (Petrolether/Ethylacetat 4:1). □ 400 MHz-$^1$H-NMR ($CDCl_3$): d[ppm] = 7.68□7.65; 7.42□7.34 (m, 10H, Ph-H), 5.90□5.80 ($m_c$, 1 H, =CH), 5.26 (d, 1 H, $J_{vic\,=}$ 17.6 Hz, $CH_{trans}$=), 5.20 (dd, 1 H, $J_{2,1}$ = 10.0 Hz, $J_{2,3}$ = 9.8 Hz, 2-H, R+S), 5.18 (d, 1 H, $J_{vic\,=}$ 11.8 Hz, $CH_{cis}$=), 4.30 (d, 1 H, $J_{1,2}$ = 10.0 Hz, 1-H), 4.16 (d, 1 H, $J_{4,5}$ = 2.7 Hz, 4-H), 4.11 (dd, 1 H, $J_{gem}$ = 12.9 Hz, $J_{vic\,=}$ 5.6 Hz, =CH-$CH_a$), 4.03 (dd, 1 H, $J_{gem}$ = 12.9 Hz, $J_{vic\,=}$ 5.6 Hz, =CH-$CH_b$), 3.94 (dd, 1 H, $J_{gem}$ = 10.3 Hz, $J_{6a,5}$ = 6.5 Hz, 6-$H_a$), 3.86 (dd, 1 H, $J_{gem}$ = 10.3 Hz, $J_{6b,5}$ = 5.6 Hz, 6-$H_b$), 3.59 (s, 3H, $COOCH_3$) 3.50 (dd, 1 H, $J_{5,6a}$ = $J_{5,6b}$ = 5.9 Hz, 5-H), 3.44 (dd, 1 H, $J_{3,2}$ = 9.4 Hz, $J_{3,4}$ = 2.7 Hz, 3-H), 2.75 (dt, 1 H, $J_{gem}$ = 13.2 Hz, $J_{vic}$

= 7.0 Hz, $SCH_a$), 2.61 (dt, 1 H, $J_{gem}$ = 13.2 Hz, $J_{vic}$ = 7.0 Hz, $SCH_b$), 2.37□? ($m_c$, 2H, $SCH_2CH_2$), 2.07 (s, 3H, $COCH_3$), 1.88□? ($m_c$, 2H, $CH_2COOMe$), 1.03 (s, 9H, $SiC(CH_3)_3$).

c) 4-S-(3-O'-allyl-2-O'-acetyl-4-O'-[1-(R/S)-ethoxyethyl]-6-O'-tertbutyldiphenylsilyl-β-D-galactopyranosyl)-mercaptobuttersäuremethylester

In 45 ml Dichlormethan werden 1.95 g (3.15 mmol) der Verbindung nach Beispiel 14b) gelöst und nach Zugabe von 45 ml Ethylvinylether und 0.39 g (1.56 mmol) Pyridium-ptoluolsulfonat 4 h bei Raumtemp. gerührt. Der Ansatz wird in ges. $NaHCO_3$-Lösung gegossen und die wäßr. Phase mit Ethylacetat extrahiert. Anschließend werden die vereinigten org. Phasen über $MgSO_4$ getrocknet und i. Vak. vom Lösungsmittel befreit. Die Reinigung erfolgt durch Chromatographie an Kieselgel (Laufmittel Petrolether/Ethylacetat 4:1, Säule 15 '
2 cm). Ausb. 1.49 g (69%), farbloses Öl, $R_F$= 0.39 (Petrolether/Ethylacetat 4:1). □400 MHz-$^1$H-NMR ($CDCl_3$): d[ppm] = 8.03□8.01; 7.69□7.36 (m, 10H, Ph-H), 5.80□5.65 (2$m_c$, 1 H, =CH, R+S), 5.56, 5.50 (2dd, 1 H, $J_{2,1}$ = $J_{2,3}$ = 9.8 Hz, 2-H, R+S), 5.26-5.01 (m, 2H, $CH_{trans}$=, $CH_{cis}$=, R+S), 4.94 (q, 1H, $CHCH_3$), 4.52, 4.46 (2d, 1H, $J_{1,2}$ = 10.3 Hz, 1-H, R+S), 4.16□3.45 (m, 7H, 4-H, 6-$H_{a,b}$, 5-H, 3-H, =CH-$CH_{a,b}$, R+S), 3.54, 3.53 (2s, 3H, $COOCH_3$, R+S) 3.69□3.63, 3.32□3.25 (2$m_c$, 2H, $CH_2CH_3$, R+S), 3.04□2.94, 2.89□2.78 (2$m_c$, 2H, $SCH_2$, R+S) 2.66, 2.60 (2t, $J_{vic}$ = 7.3 Hz, $SCH_2CH_2$, R+S), 1.33, 1.26 (2d, 3H, $J_{vic}$ = 5.3 Hz, $CHCH_3$, R+S), 1.17, 0.95 (2t, 3H, $J_{vic}$ = 7.0 Hz, $CH_2CH_3$, R+S), 1.07, 1.05 (2s, 9H, $C(CH_3)_3$, R+S

Die Verseifung des Esters erfolgt wie bei der Glycose beschrieben.

Darstellung der Mannosederivate

Beispiel 15

N-[2-S-(2',3',4',6'-Tetra-O-acetyl-α-D-mannopyranosyl)-thioethyl]-bernsteinsäuremonomethylesteramid 23

Zu 10.0 g (25.6 mmol) des Anomerengemisches 22 in 300 ml $CH_2Cl_2$ tropft man unter Eiskühlung eine Lösung von 11.1 g (34.3 mmol) N-(2-Thioethyl)-bernsteinsäuremonomethylesteramid 6 und 43 ml (0.34 mol) Bortrifluoridetherat in 70 ml $CH_2Cl_2$ zu. Die Lösung wird auf Raumtemp. erwärmt und 12 h gerührt. Der Ansatz wird zweimal mit jeweils 500 ml ges. $NaHCO_3$-Lösung gewaschen und die organische Phase über $MgSO_4$ getrocknet. Nach Chromatographie an Kieselgel mit Petrolether/Ethylacetat (1:2) erhält man 5.5 g (44%) reines α-Produkt als farbloses Öl sowie 2.3 g (19%) einer α,β-Mischfraktion als gelbliches Öl.
$R_f$= 0.24 (PE/EtOAc = 1:2 v/v), $R_f$= 0.52 (EtOAc/HOAc = 30:1 v/v),

200 MHz-$^1$H-NMR ($CDCl_3$) : δ [ppm] = 6.28 ($s_b$, 1 H, NH); 5.30-5.14 (m, 4H, H-1□, H-2□, H-3□ & H-4□); 4.37-4.06 (m, 3H, H-5□ & H-6□a/b); 3.65 (s, 3H, $CO_2CH_3$); 3.57-3.39 (m, 2H, $CH_2N$ Cya); 2.85-2.71 (m, 2H, $SCH_2$ Cya); 2.63 (t, 2H, $J_{vic}$= 7.08 Hz, $CH_2CO$ Suc); 2.45 (t, 2H, $J_{vic}$ = 7.09 Hz, $CH_2CON$ Suc);.08. 20.7, 20.5 (3x s, 12H, $CH_3$ Ac).

Beispiel 16

N-[2-S-(α-D-Mannopyranosyl)-thioethyl]-succinimid 24

Zu 7.9 g (15.2 mmol) des Glycosid 23 in 100 ml Methanol p.a. gibt man unter Argon und Eiskühlung 1.5 ml einer 1 M Lösung von Natriummethanolat in Methanol, rührt 2 h bei Raumtemp. gerührt und neutralisiert mit Amberlyst□ 15. Der Ionenaustauscher wird abfiltriert, mit Methanol gewaschen und i. Vak. das Lösungsmittel entfernt. Durch Kodestillation mit Toluol werden Reste von Methanol entfernt. Man erhält 4.8g (97%) als gelbliches Öl, das noch geringe Verunreinigungen aufweist. Das Rohprodukt wird ohne weitere Reinigung in der nächsten Stufe eingesetzt.
Rf= (Toluol:EtOH = 4:1 v/v).

Beispiel 17

N-[2-S-(4',6'-O-Benzyliden-α-D-mannopyranosyl)-thioethyl]-succinimid 25

In 75 ml Methanol werden 5.5 g (10.55 mmol) des Thioglycosids 23 bei Raumtemp. mit 100 mg (1.8 mmol) NaOMe versetzt. Nach 2 h zeigt die dünnschichtchromatographische Kontrolle unvollständigen Umsatz an, weshalb weitere 100 mg NaOMe zugegeben werden. Nach weiterem 1.5 h wird mit saurem Ionenaustauscher Amberlyst□ 15 neutralisiert. Der Ionenaustauscher wird abfiltriert und das Lösungsmittel i. Vak. entfernt.
Das Rohprodukt wird in 50 ml absol. DMF mit 3.1 ml (20 mmol)
Benzaldehyddimethylacetal sowie 112 mg (1 mmol) p-Toluolsulfonsäure versetzt und 2 h bei 50°C und 50-70 mbar

gerührt. Der Ansatz wird mit 10 ml Triethylamin neutralisiert und das Lösungsmittel i. Hochvak. entfernt. Der Rückstand wird in 200 ml $CH_2Cl_2$ aufgenommen und zweimal mit 75 ml $NaHCO_3$ extrahiert. Die organische Phasen wird abgetrennt, über $MgSO_4$ getrocknet und das Lösungsmittel i. Vak. entfernt. Durch Chromatographie an Kieselgel mit Petrolether/Ethylacetat-Gemischen erhält man 3.33 g (77%) eines farblosen Öls.
$R_f$= 0.44 (EtOAc/HOAc = 30:1 v/v),
200 MHz-$^1$H-NMR ($CDCl_3$) : δ [ppm] = 7.43-7.41 (m, 2H, Ph); 7.33-7.29 (m, 3H, Ph); 5.48 (s, 1 H, PhCH); 5.32 (s, 1 H, H-1□); 4.19-3.43 (m, 8H, H-2□, H-3□, H-4□, H-5□, H-6a/b□, $NCH_2$ Cya); 2.80-2.64 (m, 2H, $SCH_2$ Cya); 2.58 (s, 4H, $COCH_2$ Suc).

Beispiel 18

N-[2-S-(2'-O-Acetyl-3'-O-allyl-4',6'-O-benzyliden-α-D-mannopyranosyl)-thioethyl]-succinimid 26

330 mg (0.81 mmol) 25 werden in 20 ml Methanol mit 227 mg (0.91 mmol) Dibutylzinnoxid versetzt. Die Suspension wird 2.5h unter Rückfluß erhitzt, i. Vak. vom Lösungsmittel befreit und der Rückstand i. Hochvak. getrocknet.
Das Zinnacetal wird in 20 ml absol. Toluol mit 0.12 ml (1.4 mmol) Allylbromid sowie 517 mg (1.4 mmol) TBAI versetzt und 6 h bei 40°C gerührt. Die dünnschichtchromatographische Kontrolle zeigt geringen Umsatz an. Daher wird die Reaktion bei 80°C fortgeführt. Nach 7 h wird das Lösungsmittel i. Vak. entfernt und das Produkt durch zweimalige Chromatographie an Kieselgel mit Petrolether/Ethylacetat-Gemischen isoliert. Man erhält 300 mg eines rostfarbenen Öls, das laut NMR-Spektrum noch Zinnrückstände enthält.
$R_f$= 0.56 (Toluol/EtOH = 4:1 v/v).
200 MHz-$^1$H-NMR ($CDCl_3$) : δ [ppm] = 7.48-7.43 (m, 2H, Ph); 7.36-7.31 (m, 3H, Ph); 5.96-5.77 (m, 1 H, $CH_2$=CH); 5.55 (s, 1 H, PhCH); 5.40 (s, 1 H, H-1□); 5.26 (dd, 1H, $J_{gem,trans}$ = 17.09 Hz, $J_{vic}$ = 1.46 Hz, $CH_2$=CH); 5.16 (dd, 1 H, $J_{gem,cis}$ = 10.26 Hz, $J_{vic}$ = 0.98 Hz, $CH_2$=CH); 4.31-4.01 (m, 5H, H-2□, H-6a/b□, =CH-$CH_2$); 3.88-3.60 (m, 3H, H-4□, $NCH_2$ Cya); 3.29-3.20 (m, 2H, H-3□, H-5□); 2.85-2.76 (m, 2H, $SCH_2$ Cya); 2.69 (s, 4H, $COCH_2$ Suc).
In 10 ml Pyridin p.a. werden 300 mg (0.67 mmol) 25 mit 2 ml Acetanhydrid sowie einer Spatelspitze 1.5 h bei Raumtemp. gerührt. Das Lösungsmittel wird i. Vak. entfernt und das Produkt durch Chromatographie an Kieselgel mit Petrolether/Ethylacetat (1:1) gereinigt. Man erhält 191 mg (48% über zwei Stufen) eines gelben Öls. $R_f$= 0.63 (Toluol/EtOH = 4:1 v/v),
200 MHz-$^1$H-NMR ($CDCl_3$) : δ [ppm] = 7.47-7.42 (m, 2H, Ph); 7.37-7.32 (m, 3H, Ph); 5.96-5.70 (m, 1 H, $CH_2$=CH); 5.66 (s, 1 H, PhCH); 5.33-5.08 (m, 3H, $CH_2$=CH, H-2□); 5.25 (s, 1 H, H-1 □); 4.44-4.0 (m, 5H, H-40, H-6a/b□, =CH-$CH_2$); 3.85-3.45 (m, 4H, H-3□, H-5□, $NCH_2$ Cya); 2.87-2.72 (m, 2H, $SCH_2$ Cya); 2.65 (s, 4H, $COCH_2$ Suc); 2.12 (s, 3H, $CH_3$ Ac).

Beispiel 19

N-[2-S-(2'-O-Acetyl-3'-O-allyl-6'-O-tert.-butyldiphenylsilyl-4'-O-(1"-(R/S)-ethoxyethyl)-α-D-mannopyranosyl)-thioethyl]-succinimid 27

Zu 180 mg (0.37 mmol) 26 in 10 ml absol. Acetonitril werden 0.12 ml einer 48%igen Lösung von $HBF_4$ in Wasser gegeben und die Reaktionslösung 2 h bei Raumtemp. gerührt. Die dünnschichtchromatographische Kontrolle zeigt vollständigen Umsatz an und der Ansatz wird mit 10 ml ges. $NaHCO_3$-Lösung versetzt und zweimal mit 50 ml $CH_2Cl_2$ extrahiert. Die vereinigten organischen Phasen werden über $MgSO_4$ getrocknet und das Lösungsmittel i. Vak. entfernt.
Der Rückstand wird in 10 ml absol. $CH_2Cl_2$ aufgenommen und mit 0.19 ml (0.72 mmol) tert.-Butyldiphenylchlorsilan sowie 100 mg (1.5 mmol) Imidazol versetzt. Nach 16 h wird die Lösung mit 50 ml $CH_2Cl_2$ verdünnt und mit 0.5 N HCl-Lösung extrahiert. Die organische Phase wird über $MgSO_4$ getrocknet und i. Vak. das Lösungsmittel entfernt. Durch Chromatographie an Kieselgel mit Petrolether/Ethylacetat-Gemischen erhält man 69 mg (30%) des Produktes als farbloses Öl. $R_f$= 0.21 (Toluol/EtOH = 4:1 v/v),.
400 MHz-$^1$H-NMR ($CDCl_3$) : δ [ppm] = 7.69-7.65 (m, 4H, PhSi); 7.41-7.33 (m, 6H, PhSi); 5.89-5.80 (m, 1H, $CH_2$=CH); 5.32 (d, 1 H, $J_{1,2}$= 1.17 Hz, H-2□); 5.29 (s, 1 H, H-1□); 5.26 (dd, 1H, $J_{vic,trans}$ = 16.88 Hz, $J_{gem}$ = 1.62 Hz, $CH_2$=CH); 5.18 (dd, 1H, $J_{vic,cis}$ = 11.74 Hz, $J_{gem}$ = 1.47 Hz, $CH_2$=CH); 4.11 (dd, 1H, $J_{gem}$ = 12.32 Hz, $J_{vic}$ = 5.58 Hz, =CH-$CH_2$); 4.00-3.88 (m, 5H, H-3□, H-4□ & H-6□a/b); 3.78-3.71 (m, 1H, H-5□); 3.64-3.58 (m, 2H, $CH_2$N Cya); 2.82-2.68 (m, 2H, $SCH_2$ Cya); 2.66 (s, 4H, $CH_2$CO Suc); 2.08 (s, 3H, $CH_3$ Ac); 1.03 (s, 9H, $CH_3$ tBuSi).
In 10 ml absol. $CH_2Cl_2$ werden 69 mg (0.11 mmol) des Succinimids mit 0.1 ml (1.1 mmol) Ethylvinylether sowie 27 mg (0.11 mmol) Pyridinium-toluol-4-sulfonat zur Reaktion gebracht. Nach 4 h beträgt der Umsatz laut Dünnschichtchromatogramm nur ca. 70%, so daß weitere 0.1 ml Ethylvinylether und 27 mg Pyridinium-toluol-4-sulfonat

zugegeben werden und die Reaktionszeit auf 16 h verlängert wird. Der Ansatz wird mit 30 ml $CH_2Cl_2$ verdünnt und mit ges. $NaHCO_3$-Lösung extrahiert. Die organische Phase wird über $MgSO_4$ getrocknet und i. Vak. das Lösungsmittel entfernt. Durch Chromatographie an Kieselgel mit Petrolether/Ethylacetat (1:1) erhält man 42 mg (56%) eines farblosen Öls. Desweiteren fallen einige Fraktionen an, die nicht umgesetztes Edukt enthalten. $R_f$= 0.44 (Toluol/EtOH = 4:1 v/v), = + 96.1 (c 1, $CHCl_3$).
400 MHz-$^1$H-NMR ($CDCl_3$) : δ [ppm] = 7.70-7.63 (m, 4H, PhSi); 7.38-7.31 (m, 6H, PhSi); 5.88-5.76 (m, 1 H, $CH_2$=CH); 5.32-5.26 (m, 2H, H-1□ & H-2□); 5.24, 5.18 (dm, 1H, $J_{vic,trans}$ = 16.88 Hz, $CH_2$=CH); 5.15, 5.12 (dm, 1 H, $J_{vic,cis}$ = 10.57 Hz, $CH_2$=CH); 4.90-4.87 (m, 1H, $CHCH_3$ EE); 4.10-3.42 (m, 10.5H, H-3□, H-4□, H-5□, H-6□a/b, =CH-$CH_2$, $CH_3CH_2O$ EE & $CH_2N$ Cya); 3.22-3.16 (m, 0.5H, H-5□); 2.74-2.70 (m, 2H, $SCH_2$ Cya); 2.67, 2.65 (2x s, 4H, $CH_2CO$ Suc); 2.10, 2.09 (2x s, 3H, $CH_3$ Ac); 1.24-1.20 (m, 3H, $CHCH_3$ EE); 1.15 (t, 1.5H, $J_{gem}$ = 7.05 Hz, $CH_3CH_2O$ EE); 1.04, 1.02 (2x s, 9H, $CH_3$ tBuSi); 0.89 (t, 1.5H, $J_{gem}$ = 7.05 Hz, $CH_3CH_2O$ EE).
Die Verseifung des Esters erfolgt wie bei der Glycose beschrieben Allgemeine Vorschriften zur Synthese an polymeren Trägern

Allgemeine Vorschrift zur Kupplung der Thioglycoside auf den aminofunktionalisierten polymeren Träger

Eine Lösung von 14.6 mmol eines Thioglycosids wird in einem Festphasenreaktor mit 15.4 g (19.8 mmol, 1.28 mmol/g) Aminomethylpolystyrol, 3.7 ml (14.6 mmol) Diisopropylcarbodiimid und 3.86 g (14.6 mmol) N-Hydroxybenzotriazol über Nacht geschüttelt. Anschließend saugt man ab und wäscht zehnmal mit je 50 ml DMF und Dichlormethan. Das beladene Polymer wird i. Vak. getrocknet und die Beladung mit Kohlenhydratmatrix über Elementaranalyse bestimmt. Die Beladung beträgt in der Regel 50-80 % der maximal möglichen Beladung.
Allgemeine Vorschrift zur Abspaltung der Glycosidderivate vom polymeren Träger

a) analytisch: Eine Suspension von 80 mg (0.056 mmol) polymergebundenem Derivat in 1.5 ml abs. Dichlormethan wird in einer 5 ml- PE-Spritze (PE-Fritte, Plastikkappe) mit 0.3 ml einer 3.5-prozentigen Lösung von Brom in abs. Dichlormethan und 0.08 ml (0. mmol) 2,6-Di-tert-butylpyridin oder entsprechender Menge polymergebundenem 2,6-Di-tert-Butylpyridin bei Raumtemp. geschüttelt. Nach 15 min setzt man 0.2 ml Cyclohexen, 0.2 ml des zu glycosylierenden abs. Alkohol und 25 mg (0.056 mmol) Tetraethylammoniumbromid zu. Nach 2.5 h filtriert man ab und wäscht fünfmal mit 1 ml Dichlormethan. Die vereinigten Filtrate werden i. Vak. vom Lösungsmittel befreit. Das erhaltene Rohprodukt wird in wenig Dichlormethan auf eine Kieselgelkartusche aufgebracht. Man eluiert zunächst mit 30 ml Petrolether. Diese Fraktion wird verworfen. Das Produkt erhält man durch Eluieren mit Petrolether/Ethylacetat (1:1). Es schließt sich eine Charakterisierung durch HPLC- und MS-Analytik an.
b) präparativ: Eine Suspension von 400 mg (0.312 mmol) polymergebundenem Galactosederivat in 3 ml abs. Dichlormethan wird in einer 5 ml- PE-Spritze (PE-Fritte, Plastikkappe) mit 1.2 ml einer 3.5-prozentigen Lösung von Brom in abs. Dichlormethan und 0.32 ml (0. mmol) 2,6-Di-tert-butylpyridin bei Raumtemp. geschüttelt. Nach 15 min filtriert man ab und wäscht fünfmal mit je 3 ml Dichlormethan nach. Zum Filtrat werden 0.5 ml Cyclohexen, 0.5 ml des zu glycosylierenden abs. Alkohol und 25 mg (0.056 mmol) Tetraethylammoniumbromid zugesetzt. Die organische Phase wird mit Wasser gewaschen, über Magnesiumsulfat getrocknet, i. Vak. eingeengt und durch Flash*-Chromatographie an Kieselgel gereinigt.

Allgemeine Vorschrift zur Alkylierung der Glycoside am polymeren Träger mit Kalium-tert-butylat

Zu einer Suspension von 100 mg (0.078) mmol beladenem Polymer in 1.5 ml abs. DMF gibt man eine Lösung von 87 mg (0.78 mmol) Kalium-tert-butylat in abs. DMF. Die Mischung wird 15 min geschüttelt. Man filtriert und verwirft das Filtrat. Anschließend fügt man 0.78 mmol des entsprechenden Alkylierungsmittels in 1.5 ml abs. DMF zu und 4 h schütteln. Die Lösung wird vom Harz abgetrennt und fünfmal mit je 2 ml DMF, Toluol und Dichlormethan gewaschen.
Allgemeine Vorschrift zur Alkylierung der Galactoside am polymeren Träger mit tert-Butyl-$P_4$-Base (Schwesinger-Base)
Zu einer Suspension von 80 mg (0.056) mmol beladenem Polymer in 1.5 ml abs. DMF gibt man eine Lösung von 0.22 ml (0.22 mmol) tert-Butyl-$P_4$-Base in abs. DMF. Die Mischung wird 10 min geschüttelt. Anschließend fügt man 0.56 mmol des entsprechenden Alkylierungsmittels zu und läßt 2-4 h schütteln. Die Lösung wird vom Harz abgesaugt und fünfmal mit je 2 ml DMF, Toluol und Dichlormethan gewaschen.

Allgemeine Vorschrift zur Abspaltung der Acetatschutzgruppe am polymeren Träger

Zu einer Supsension von 0.0050 mmol beladenem Polymer in 2 ml Dioxan/Methanol gibt man 0.3 ml einer 30 %igen Natriummethanolatlösung. Die Mischung wird 3 h bei Raumtemperatur geschüttelt. Die Lösung wird vom Harz abgesaugt und mit erst Methanol/Dioxan fünfmal und dann füfmal mit je 2 ml DMF und Dichlormethan gewaschen

Allgemeine Vorschrift zur Abspaltung der tert-Butyldiphenylsilylschutzgruppe am polymeren Träger

Zu einer Suspension von 0.056 mmol beladenem Polymer in 1.5 ml THF gibt man 0.56 ml (0.56 mmol, 1 M) Tetrabutylammoniumfluorid in THF. Die Mischung wird 4 h geschüttelt. Die Lösung wird vom Harz abgesaugt und fünfmal mit je 2 ml DMF und Dichlormethan gewaschen.

Allgemeine Vorschrift zur Abspaltung der Ethoxethyletherschutzgruppe am polymeren Träger

Zu einer Suspension von 0.050 mmol beladenem Polymer in 1.5 ml Dioxan/Methanol gibt man para-Toluolsulfonsäure und rührt das Gemisch bei 40°C für 4 h. Die Lösung wird vom Harz abgesaugt und mit 0.5N HCl - Lösung gwaschen und anschließend fünfmal mit je 2 ml DMF und Dichlormethan gewaschen

Die Isopropylschutzgruppe kann unter analogen Bedingungen abgespalten werden. Die Reaktionszeiten oder Temperaturemn können sich dabei verändern.

Allgemeine Vorschrift zur Abspaltung der Allyletherschutzgruppe am polymeren Träger

Zu einer Suspension von 0.060 mmol beladenem Polymer in 1.5 ml THF gibt man bei - 70°C ein Lösung aus 198 mg Zirconocendichlorid und 0.63 ml BuLi (1.7 M) in THF. Das Gemisch wird nach beendeter Zugabe 4 H bei Raumtemperatur gerührt. Die Lösung wird vom Harz abgesaugt und mit 0.5N HCl - Lösung gwaschen und anschließend fünfmal mit je 2 ml DMF und Dichlormethan gewaschen.

Beispiel 20

Methyl 2-O-methyl-6-O-tert.-butyldiphenylsilyl-3-O-propyl- α/β-D-glucopyranosid

166 mg (0.1 mmol) des Polymers werden gemäß der allgemeinen Arbeitsvorschrift für eine Alkylierung mit Methyliodid umgesetzt. Nach Filtration an Kieselgel erhält man 16 mg eines farblosen Öls. Das Produkt wird durch präparative HPLC gereinigt und einzelne Fraktionen durch massenspektrometrisch Analyse sowie NMR-Spektren identifiziert.
$R_f$= 0.45, 0.34 (PE/EtOAc = 4:1 v/v).
FBA-MS (NBA-pos, LiCl) : (m/z) = 495.2 (100%, $[M+Li]^+$, ber.: 295.2); 496.2 (26%, $[M+Li]^+$, $^{13}C$, ber.: 496.2).

Beispiel 21.

Methyl 2-O-benzyl-6-O-tert.-butyldiphenylsilyl-3-O-propyl -α/β-D-glucopyranosid

166 mg (0.1 mmol) des Polymers werden gemäß der allgemeinen Arbeitsvorschrift für eine Alkylierung mit Benzylbromid umgesetzt und das Kohlenhydrat vom Harz abgespalten. Nach Filtration an Kieselgel erhält man 15 mg eines farblosen Öls. Das Produkt wird durch präparative HPLC gereinigt und einzelne Fraktionen durch massenspektrometrisch Analyse sowie NMR-Spektren identifiziert.
$R_f$= 0.53, 0.47 (PE/EtOAc = 4:1 v/v).
$C_{33}H_{44}O_6Si$ (564.8)
FBA-MS (NBA-pos, LiCl) : (m/z) = 571.1 (100%, $[M+Li]^+$, ber.: 571.3); 572.1 (35%, $[M+Li]^+$, $^{13}C$ , ber.: 572.3).

Beispiel 22

Methyl 2-O-propyl-6-O-tert.-butyldiphenylsilyl-3-O-propyl-α/β-D-glucopyranosid

166 mg (0.1 mmol) des Polymers werden gemäß der allgemeinen Arbeitsvorschrift für eine Alkylierung mit n-Propyliodid umgesetzt und das Kohlenhydrat vom Harz abgespalten. Nach Filtration an Kieselgel erhält man 18 mg eines farblosen Öls.
$R_f$= 0.49, 0.46 (PE/EtOAc = 4:1 v/v).
$C_{29}H_{44}O_6Si$ (516.8)
FBA-MS (NBA-pos, LiCl) : (m/z) = 523.3 (100%, $[M+Li]^+$, ber.: 523.2); 524.3 (33%, $[M+Li]^+$, ber.: 524.2).

Beispiel 23

Methyl 2-O-(2'-naphthyl)-methyl-6-O-tert.-butyldiphenylsilyl-3-O-propyl -α/β-Dglucopyranosid

166 mg (0.1 mmol) des Polymers werden gemäß der allgemeinen Arbeitsvorschrift für eine Alkylierung mit (2-Naphthyl)-methylenbromid umgesetzt und das Kohlenhydrat vorn Harz abgespalten. Nach Filtration an Kieselgel erhält man 22 mg eines schwach gelblichen Öls. Das gewünschte Produkt konnte massenspektrometrisch nachgewiesen werden.

Beispiel 24

Methyl 2-O-isopropyl-6-O-tert.-butyldiphenylsilyl-3-O-propyl-α/β-D-glucopyranosid

166 mg (0.1 mmol) des Polymers werden gemäß der allgemeinen Arbeitsvorschrift für eine Alkylierung mit 2-Propylbromid umgesetzt und das Kohlenhydrat vom Harz abgespalten. Nach Filtration an Kieselgel erhält man 17 mg. Massenspektrometrisch kann nachgewiesen werden.
HPLC (Gradient 54/80) : $R_t$ (min) = 3.61 (15.3%, DTBpy); 14.2 (11.0%, 2-OH); 15.7 (19.2%).

Beispiel 25

Methyl 2-O-(4'-cyanobenzyl)-6-O-tert.-butyldiphenylsilyl-3-O-propyl-α/β-D-Glucopyranosid

166 mg (0.1 mmol) des Polymers werden gemäß der allgemeinen Arbeitsvorschrift für eine Alkylierung mit o-Cyanobenzylbromid umgesetzt und das Kohlenhydrat vom Harz abgespalten Nach Filtration an Kieselgel erhält man 22 mg eines schwach gelbliches Öl. $R_f$= 0.52, 0.47 (PE/EtOAc = 4:1 v/v).
$C_{33}H_{43}NO_6Si$ (577.8)
FBA-MS (NBA-pos, LiCl) : (m/z) = 241.1 (69%); 596.3 (38%, $[M+Li]^+$, ber.: 596.4); 597.3 (22%, $[M+Li]^+$, $^{13}C$, ber.: 597.4); 746.4 (66%, $[M+C_4H_8{}^{79}BrO+H]^+$, ber.: 746.4); 747.4 (64%, $[M+C_4H_8{}^{79}BrO+H]^+$, $^{13}C$, ber.: 747.4); 748.4 (100%, $[M+C_4H_8{}^{81}BrO+H]^+$, ber.: 748.4); 749.4 (57%, $[M+C_4H_8{}^{81}BrO+H]^+$, $^{13}C$, ber.: 749.4).

Beispiel 26

Methyl 2-O-heptyl-6-O-tert.-butyldiphenylsilyl-3-O-propyl-α/β-D-glucopyranosid

166 mg (0.1 mmol) des Polymers werden gemäß der allgemeinen Arbeitsvorschrift für eine Alkylierung mit 1-lodheptan umgesetzt und das Kohlenhydrat vom Harz abgespalten. Nach Filtration an Kieselgel erhält man 25 mg eines farblosen Öls.
$R_f$= 0.65, 0.49 (PE/EtOAc = 4:1 v/v).
$C_{33}H_{43}NO_6Si$ (577.79)
FBA-MS (NBA-pos, LiCl) : (m/z) = 241.1 (100%); 579.4 (74%, $[M+Li]^+$, ber.: 579.4); 580.4 (31 %, $[M+Li]^+$, $^{13}C$, ber.: 580.4); 729.4 (72%, $[M+C_4H_8{}^{79}BrO+H]^+$, ber.: 729.4); 730.4 (38%, $[M+C_4H_8{}^{79}BrO+H]^+$, $^{13}C$, ber.: 730.4); 731.4 (79%, $[M+C_4H_8{}^{81}BrO+H]^+$, ber.: 731.4); 732.4 (34%, $[M+C_4H_8{}^{81}BrO+H]^+$, $^{13}C$, ber.: 723.4).

Beispiel 27

Methyl 2-O-(2'-methoxy-5'-nitrobenzyl)-6-O-tert.-butyldiphenylsilyl-3-O-propyl-α/β-Dglucopyranosid
166 mg (0.1 mmol) des Polymers werden gemäß der allgemeinen Arbeitsvorschrift für eine Alkylierung mit 2-methoxy-5-nitrobenzylbromid umgesetzt und das Kohlenhydrat vom Harz abgespalten Nach Filtration an Kieselgel erhält man 15 mg eines schwach gelblichen Öls.
$R_f$= 0.48, 0.43 (PE/EtOAc = 4:1 v/v).
$C_{33}H_{45}NO_9Si$ (627.8)

Beispiel 28

Methyl 2-O-methyl-6-O-benzyl-3-O-propyl-α/β-D-glucopyranosid

166 mg (0.1 mmol) des Polymers werden gemäß der allgemeinen Arbeitsvorschrift für eine Alkylierung mit Methyliodid umgesetzt. Anschließend wird die TBDPS-Gruppe entfernt und danach mit Benzylbromid alkyliert. Nach

Abspaltung des Kohlenhydrates vom Harz und Filtration an Kieselgel erhält man 24 mg eines farblosen Öls. Für eine gesicherte Charakterisierung des Produktes sowie eine Zuordnung der beiden Anomeren wurde das Produkt durch präparative HPLC (Gradient 90/10) gereinigt.
$C_{18}H_{28}O$ (340.4)
$R_f$= 0.63, 0.54 (PE/EtOAc = 3:1 v/v).
400 MHz-$^1$H-NMR ($CDCl_3$) : δ [ppm] = 7.32-7.31 (m, 5H, Ph); 4.83 (d, 1H, $J_{2,1}$= 3.81 Hz, H-1); 4.60 (d, 1 H, $J_{gem}$= 12.32 Hz, $CH_2$Ph); 4.55 (d, 1 H, $J_{gem}$= 12.03 Hz, $CH_2$Ph); 4.20 (dd, 0.3H, $J_{1,2}$= 3.23 Hz, $J_{3,2}$= 12.03 Hz, H-2), 4.12 (dd, 0.7H, $J_{1,2}$= 2.94 Hz, $J_{3,2}$= 11.45 Hz, H-2); 4.00 (dd, 0.3H, $J_{vic}$= 11.89 Hz, $J_{gem}$= 6.61 Hz, $OCH_2$ Pr); 3.92 (dd, 0.7H, $J_{vic}$= 11.44 Hz, $J_{gem}$= 7.34 Hz, $OCH_2$Pr); 3.83-3.82 (m, 1.6H, H-6a/b); 3.81-3.39 (m, 8.4Hz, H-3, H-4, H-6b); 3.47, 3.41 (2x s, 6H, $OCH_3$); 3.23-3.21 (m, 1H, H-5); 1.91 ($s_b$, 1H, OH); 1.61-1.54 (m, 2H, $OCH_2CH_2$ Pr); 0.91 (t, 3H, $J_{gem}$= 7.34 Hz, $CH_3$ Pr);

Beispiel 29

Benzyl 2-O-methyl-6-O-methyl-3-O-propyl-α/β-D-glucopyranosid

166 mg (0.1 mmol) des Polymers werden gemäß der allgemeinen Arbeitsvorschrift für eine Alkylierung mit Methyliodid umgesetzt. Anschließend wird die TBDPS-Gruppe entfernt und danach mit Methyliodid alkyliert. Nach Abspaltung des Kohlenhydrates vom Harz und Filtration an Kieselgel erhält man 20 mg eines farblosen Öls.
$R_f$= 0.46 0.34 0.1 (OH) (PE/EtOAc = 3:1 v/v).
$C_{18}H_{28}O_6$ (340.4)
FBA-MS (NBA-pos, LiCl) : (m/z) = 233.1 ($Gly^+$, ber.: 233.1); 347.0 ($[M+Li]^+$, ber.: 347.2); 497.2 ($[M+C_4H_8{}^{79}BrO+Li]^+$, ber.: 497.2); 499.2 ($[M+C_4H_8{}^{81}BrO+Li]^+$, ber.: 497.2).

Beispiel 30

Methyl 2-O-methyl-6-O-heptyl-3-O-propyl-α/β-D-glucopyranosid

166 mg (0.1 mmol) des Polymers werden gemäß der allgemeinen Arbeitsvorschrift für eine Alkylierung mit Methyliodid umgesetzt. Anschließend wird die TBDPS-Gruppe entfernt und danach mit Heptyliodid alkyliert. Nach Abspaltung des Kohlenhydrates vom Harz und Filtration an Kieselgel erhält man 27 mg eines farblosen Öls.
$R_f$= 0.53, 0.42 (PE/EtOAc = 3:1 v/v).
$C_{18}H_{36}O_6$ (348.5)
FBA-MS (NBA-pos, LiCl) : (m/z) = 355.2 (22%, [M+Li]+, ber.: 355.2); 369.2 (88%); 505.2 (100%, $[M+C_4H_8{}^{79}BrO+Li]^+$, ber.: 505.2); 507.2 (99%, $[M+C_4H_8{}^{81}BrO+Li]^+$, ber.: 507.2).

Beispiel 31

Isopropyl 2-O-methyl-6-O-heptyl-3-O-propyl-α/β-D-glucopyranosid

166 mg (0.1 mmol) des Polymers werden gemäß der allgemeinen Arbeitsvorschrift für eine Alkylierung mit Methyliodid umgesetzt. Anschließend wird die TBDPS-Gruppe entfernt und danach mit Heptyliodid alkyliert. Nach Abspaltung des Kohlenhydrates vom Harz und Filtration an Kieselgel erhält man 21 mg eines farblosen Öls.
$R_f$= 0.69, 0.63 (PE/EtOAc = 3:1 v/v).
$C_{20}H_{40}O_6$ (376.5)
FBA-MS (NBA-pos, LiCl) : (m/z) = 533.2 (100%, $[M+C_4H_8{}^{79}BrO+Li]^+$, ber.: 533.2); 534.2 (28%, $[M+C_4H_8{}^{79}BrO+Li]^+$, $^{13}$C, ber.: 534.2); 535.2 (99%, $[M+C_4H_8{}^{81}BrO+Li]^+$, ber.: 535.2); 536.2 (26%, $[M+C_4H_8{}^{81}BrO+Li]^+$,$^{13}$C, ber.: 536.2).

Beispiel 32

Ethyl 2-O-methyl-6-O-(2'-methoxy-5'-nitrobenzyl)-3-O-propyl-α/β-D-glucopyranosid

166 mg (0.1 mmol) des Polymers werden gemäß der allgemeinen Arbeitsvorschrift für eine Alkylierung mit Methyliodid umgesetzt. Anschließend wird die TBDPS-Gruppe entfernt und danach mit 2-Methoxy-5-nitrobenzylbromid alkyliert. Nach Abspaltung des Kohlenhydrates vom Harz und Filtration an Kieselgel erhält man 10 mg eines farblosen Öls.
$R_f$= 0.35, 0.27 (PE/EtOAc = 3:1 v/v).

$C_{18}H_{36}O_6$ (429.5)
FBA-MS (NBA-pos, LiCl) : (m/z) = 384.1 (3%, $Gly^+$, ber.: 384.2); 435.1 (22%); 436.1 (22%, $[M+Li]^+$, ber.: 436.2); 586.1 (97%, $[M+C_4H_8{}^{79}BrO+Li]^+$, ber.: 586.2); 587.1 (36%, $[M+C_4H_8{}^{79}BrO+Li]^+$, $^{13}C$ , ber.: 387.2); 588.1 (100%, $[M+C_4H_8{}^{81}BrO+Li]^+$, ber.: 588.2); 589.1 (34%, ($[M+C_4H_8{}^{81}BrO+Li]^+$, $^{13}C$ , ber.: 589.2).

Beispiel 33

Methyl 2-O-benzyl-6-O-isopropyl-3-O-propyl-α/β-D-glucopyranosid

166 mg (0.1 mmol) des Polymers werden gemäß der allgemeinen Arbeitsvorschrift für eine Alkylierung mit Benzylbromid umgesetzt. Anschließend wird die TBDPS-Gruppe entfernt und danach mit 2-Brompropan alkyliert. Nach Abspaltung des Kohlenhydrates vom Harz und Filtration an Kieselgel erhält man 9 mg eines schwach gelblichen Öls.
$R_f$= 0.62 (PE/EtOAc = 3:1 v/v).
$C_{20}H_{32}O_6$ (368.5)
FBA-MS (NBA-pos, LiCl) : (m/z) = 301.1 (83%); 373.2 (19%); 525.2 (17%, $[M+C_4H_8{}^{79}BrO+Li]^+$, ber.: 525.2); 527.2 (15%, $[M+C_4H_8{}^{81}BrO+Li]^+$, ber.: 527.2); 573.2 (13%); 575.2 (17%); 667.3 (21%); 669.3 (19%).

Beispiel 34

Ethyl 2-O-benzyl-6-O-(4'-cyanobenzyl)-3-O-propyl-α/β-D-glucopyranosid

166 mg (0.1 mmol) des Polymers werden gemäß der allgemeinen Arbeitsvorschrift für eine Alkylierung mit Benzylbromid umgesetzt. Anschließend wird die TBDPS-Gruppe entfernt und danach mit 4-Cyanobenzylbromid alkyliert. Nach Abspaltung des Kohlenhydrates vom Harz und Filtration an Kieselgel erhält man 25 mg eines farblosen Öls. $R_f$= 0.66, 0.55 (PE/EtOAc = 3:1 v/v).
$C_{26}H_{33}NO_6$ (455.6)
FBA-MS (NBA-pos, LiCl) : (m/z) = 410.2 (5%, $Gly^+$, ber.: 410.2); 462.2 (34%, $[M+Li]^+$, ber.: 462.2); 587.2 (13%, $^{79}Br$); 589.2 (13%, $^{81}Br$); 612.2 (97%, $[M+C_4H_8{}^{79}BrO+Li]^+$, ber.: 612.2); 613.2 (43%, $[M+C_4H_8{}^{79}BrO+Li]^+$, $^{13}C$ , ber.: 613.2); 614.2 (100%, $[M+C_4H_8{}^{81}BrO+Li]^+$, ber.: 614.2); 615.2 (32%, $[M+C_4H_8{}^{81}BrO+Li]^+$, $^{13}C$, ber.: 615.2); 654.2 (11%, $^{79}Br$); 656.2 (13%, $^{81}Br$).

Beispiel 35

Methyl 2-O-benzyl-6-O-heptyl-3-O-propyl-α/β-D-glucopyranosid

166 mg (0.1 mmol) des Polymers werden gemäß der allgemeinen Arbeitsvorschrift für eine Alkylierung mit Benzylbromid umgesetzt. Anschließend wird die TBDPS-Gruppe entfernt und danach mit Heptyliodid alkyliert. Nach Abspaltung des Kohlenhydrates vom Harz und Filtration an erhält man 32 mg eines farblosen Öls.
$R_f$= 0.82, 0.76 (PE/EtOAc = 3:1 v/v).
$C_{24}H_{40}O_6$ (424.6)
FBA-MS (NBA-pos, LiCl) : (m/z) = 431.3 (14%, $[M+Li]^+$, ber.: 431.2); 581.2 (100%, $[M+C_4H_8{}^{79}BrO+Li]^+$, ber.: 581.3); 582.2 (40%, $[M+C_4H_8{}^{79}BrO+Li]^+$, $^{13}C$, ber.: 582.3); 583.2 (100%, $[M+C_4H_5{}^{81}BrO+Li]^+$, ber.: 583.3); 584.2 (30%, $[M+C_4H_8{}^{81}BrO+Li]^+$, $^{13}C$, ber.: 584.3).

Beispiel 36

Isopropyl 2-O-benzyl-6-O-cyclohexylmethyl-3-O-propyl-α/β-D-glucopyranosid

166 mg (0.1 mmol) des Polymers werden gemäß der allgemeinen Arbeitsvorschrift für eine Alkylierung mit Benzylbromid umgesetzt. Anschließend wird die TBDPS-Gruppe entfernt und danach mit Cyclohexylmethylenbromid alkyliert. Nach Abspaltung des Kohlenhydrates vom Harz und Filtration an Kieselgel erhält man 15 mg eines farblosen Öls.
$R_f$= 0.85, 0.61 (PE/EtOAc = 3:1 v/v).
$C_{26}H_{42}O_6$ (450.6)
FBA-MS (NBA-pos, LiCl) : (m/z) = 301.1 (59%); 443.3 (47%); 457.3 (34%, $[M+Li]^+$, ber.: 457.3); 517.2 (22%, $^{79}Br$); 519.2 (22%, $^{81}Br$); 607.2 (98%, $[M+C_4H_8{}^{79}BrO+Li]^+$, ber.: 607.3); 608.2 (42%, $[M+C_4H_8{}^{79}BrO+Li]^+$, $^{13}C$, ber.: 608.3); 609.2 (100%, $[M+C_4H_8{}^{81}BrO+Li]^+$, ber.: 609.3); 610.2 (34%, $[M+C_4H_8{}^{81}BrO+Li]^+$, $^{13}C$ , ber.: 610.3); 669.4 (22%).

Beispiel 37

Methyl 2-O-propyl-6-O-(4'-cyanobenzyl)-3-O-propyl-α/β-D-glucopyranosid

166 mg (0.1 mmol) des Polymers werden gemäß der allgemeinen Arbeitsvorschrift für eine Alkylierung mit n-Propyliodid umgesetzt. Anschließend wird die TBDPS-Gruppe entfernt und danach mit 4-Cyanobenzylbromid alkyliert. Nach Abspaltung des Kohlenhydrates vom Harz und Filtration an Kieselgel erhält man 17 mg eines farblosen Öls.
$R_f$= 0.63, 0.53 (PE/EtOAc = 3:1 v/v).
$C_{25}H_{31}NO_6$ (393.5)
FBA-MS (NBA-pos, LiCl) : (m/z) = 382.0 (9%, $^{79}$Br); 384.0 (9%, $^{81}$Br); 400.2 (28%, $[M+Li]^+$, ber.: 400.2); 414.2 (11%); 442.2 (11%); 477.2 (9%, $^{79}$Br); 479.2 (9%, $^{81}$Br); 515.2 (9%); 550.2 (100%, $[M+C_4H_8{}^{79}BrO+Li]^+$, ber.: 550.2); 551.2 (36%, $[M+C_4H_8{}^{79}BrO+Li]^+$, $^{13}$C, ber.: 551.2); 552.2 (98%, $[M+C_4H_8{}^{81}BrO+Li]^+$, ber.: 552.2); 553.2 (28%, $[M+C_4H_8{}^{81}BrO+Li]^+$, $^{13}$C, ber.: 553.2).

Beispiel 38

Isopropyl 2-O-propyl-6-O-benzyl-3-O-propyl-α/β-D-glucopyranosid

166 mg (0.1 mmol) des Polymers werden gemäß der allgemeinen Arbeitsvorschrift für eine Alkylierung mit n-Propyliodid umgesetzt. Anschließend wird die TBDPS-Gruppe entfernt und danach mit Benzylbromid alkyliert. Nach Abspaltung des Kohlenhydrates vom Harz und Filtration an Kieselgel erhält man 18 mg eines farblosen Öls.
$R_f$= 0.73, 0.64 (PE/EtOAc = 3:1 v/v).
$C_{22}H_{36}O_6$ (396.5)
FBA-MS (NBA-pos, LiCl) : (m/z) = 229.1 (94%); 389.2 (66%); 403.2 (43%, $[M+Li]^+$, ber.: 403.3); 445.2 (19%); 505.2 (27%, $^{79}$Br); 507.2 (27%, $^{81}$Br); 553.2 (82%, $[M+C_4H_8{}^{79}BrO+Li]^+$, ber.: 553.3); 554.2 (33%, $[M+C_4H_8{}^{79}BrO+Li]^+$, $^{13}$C, ber.: 554.3); 555.2 (85%, $[M+C_4H_8{}^{81}BrO+Li]^+$, ber.: 555.3); 556.2 (26%, $[M+C_4H_8{}^{81}BrO+Li]^+$, $^{13}$C, ber.: 556.3).

Beispiel 39

Benzyl 2-O-propyl-6-O-methyl-3-O-propyl-α/β-D-glucopyranosid

166 mg (0.1 mmol) des Polymers werden gemäß der allgemeinen Arbeitsvorschrift für eine Alkylierung mit n-Propyliodid umgesetzt. Anschließend wird die TBDPS-Gruppe entfernt und danach mit Methyliodid alkyliert. Nach Abspaltung des Kohlenhydrates vom Harz und Filtration an Kieselgel erhält man 149 mg eines farblosen Öls. Das Produkt enthält noch größere Mengen an Benzylalkohol.
$R_f$= 0.46, 0.40 (PE/EtOAc = 3:1 v/v).
$C_{20}H_{32}O_6$ (368.5)
FBA-MS (NBA-pos, LiCl) : (m/z) = 261.2 (8%, $Gly^+$, ber.: 261.2); 375.2 (25%, $[M+Li]^+$, ber.: 375.2); 525.2 (15%, $[M+C_4H_8{}^{79}BrO+Li]^+$, ber.: 525.2); 526.2 (5%, $[M+C_4H_8{}^{79}BrO+Li]^+$, $^{13}$C, ber.: 526.2); 527.2 (15%, $[M+C_4H_8{}^{81}BrO+Li]^+$, ber.: 527.2); 528.2 (4%, $[M+C_4H_8{}^{81}BrO+Li]^+$, $^{13}$C, ber.: 528.2).

Beispiel 40

Methyl 2-O-propyl-6-O-heptyl-3-O-propyl-α/β-D-glucopyranosid

166 mg (0.1 mmol) des Polymers werden gemäß der allgemeinen Arbeitsvorschrift für eine Alkylierung mit n-Propyliodid umgesetzt. Anschließend wird die TBDPS-Gruppe entfernt und danach mit Heptyliodid alkyliert. Nach Abspaltung des Kohlenhydrates vom Harz und Filtration an Kieselgel erhält man 24 mg eines farblosen Öls.
$R_f$= 0.58, 0.51 (PE/EtOAc = 3:1 v/v).
$C_{20}H_{40}O_6$ (376.5)
FBA-MS (NBA-pos, LiCl) : (m/z) = 383.3 (35%, $[M+Li]^+$, ber.: 383.3); 397.3 (100%); 439.3 (28%); 453.4 (48%).

Beispiel 41

Ethyl 2-O-pentyl-6-O-heptyl-3-O-propyl-α/β-D-glucopyranosid

166 mg (0.1 mmol) des Polymers werden gemäß der allgemeinen Arbeitsvorschrift für eine Alkylierung mit Pentyliodid umgesetzt. Anschließend wird die TBDPS-Gruppe entfernt und danach mit Heptyliodid alkyliert. Nach Abspaltung des Kohlenhydrates vom Harz und Filtration an Kieselgel erhält man 20 mg eines farblosen Öls.
$R_f$= 0.87, 0.80 (PE/EtOAc = 3:1 v/v).
$C_{23}H_{46}O_6$ (418.3)
FBA-MS (NBA-pos, LiCl) : (m/z) = 425.3 (76%,$[M+Li]^+$, ber.: 452.3); 426.3 (19%, $[M+Li]^+$, $^{13}C$ , ber.: 426.3); 575.3 (100%, $[M+C_4H_8{}^{79}BrO+Li]^+$, ber.: 575.3); 576.3 (38%, $[M+C_4H_8{}^{79}BrO+Li]^+$, $^{13}C$, ber.: 576.3); 577.3 (98%, $[M+C_4H_8{}^{81}BrO+Li]^+$, ber.: 577.3); 578.3 (28%, $[M+C_4H_8{}^{81}BrO+Li]^+$, $^{13}C$, ber.: 578.3).

Beispiel 42

Isopropyl 2-O-pentyl-6-O-methyl-3-O-propyl-α/β-D-glucopyranosid

166 mg (0.1 mmol) des Polymers werden gemäß der allgemeinen Arbeitsvorschrift für eine Alkylierung mit Pentyliodid umgesetzt. Anschließend wird die TBDPS-Gruppe entfernt und danach mit Methyliodid alkyliert. Nach Abspaltung des Kohlenhydrates vom Harz und Filtration an Kieselgel erhält man 18 mg eines farblosen Öls.
$R_f$= 0.67, 0.64 (PE/EtOAc = 3:1 v/v).
$C_{18}H_{36}O_6$ (348.3)
FBA-MS (NBA-pos, LiCl) : (m/z) = 341.0 (100%); 355.1 (62%, $[M+Li]^+$, ber.: 355.2); 505.2 (83%, $[M+C_4H_8{}^{79}BrO+Li]^+$, ber.: 505.2); 506.2 (28%, $[M+C_4H_8{}^{79}BrO+Li]^+$, $^{13}C$. ber.: 506.2); 507.2 (80%, $[M+C_4H_8{}^{81}BrO+Li]^+$, ber.: 507.2); 508.2 (19%, $[M+C_4H_8{}^{81}BrO+Li]^+$, $^{13}C$ , ber.: 508.2).

Beispiel 43

Benzyl 2-O-heptyl-6-O-cyclohexylmethyl-3-O-propyl-α/β-D-glucopyranosid

166 mg (0.1 mmol) des Polymers werden gemäß der allgemeinen Arbeitsvorschrift für eine Alkylierung mit Heptyliodid umgesetzt. Anschließend wird die TBDPS-Gruppe entfernt und danach mit Cyclohexylmethylenbromid alkyliert. Nach Abspaltung des Kohlenhydrates vom Harz und Filtration an Kieselgel erhält man 136 mg eines farblosen Öls. das Produkt enthält noch größere Mengen Benzylalkohol.
$R_f$= 0.77, 0.67 (PE/EtOAc = 3:1 v/v).
$C_{30}H_{50}O_6$ (506.7)
FBA-MS (NBA-pos, LiCl) : (m/z) = 285.2 (65%); 309.2 (100%); 339.2 (45%); 399.3 (39%, $Gly^+$, ber.: 399.3); 451.3 (48%); 513.3 (22%, $[M+Li]^+$, ber.: 513.3); 663.3 (24%, $[M+C_4H_8{}^{79}BrO+Li]^+$, ber.: 663.3); 665.3 (34%, $[M+C_4H_8{}^{81}BrO+Li]^+$, ber.: 665.3).

Beispiel 44

Methyl 2-O-heptyl-6-O-benzyl-3-O-propyl-α/β-D-glucopyranosid

166 mg (0.1 mmol) des Polymers werden gemäß der allgemeinen Arbeitsvorschrift für eine Alkylierung mit Heptyliodid umgesetzt. Anschließend wird die TBDPS-Gruppe entfernt und danach mit Benzylbromid alkyliert. Nach Abspaltung des Kohlenhydrates vom Harz und Filtration an Kieselgel erhält man 32 mg eines farblosen Öls.
$R_f$= 0.86, 0.77 (PE/EtOAc = 3:1 v/v).
$C_{24}H_{40}O_6$ (424.6)
FBA-MS (NBA-pos, LiCl) : (m/z) = 341.2 (7%, $[6-OH+Li]^+$, ber.: 341.3); 431.3 (13%, $[M+Li]^+$, ber.: 413.3); 459.2 (15%, $^{79}Br$); 461.3 (14%, $^{81}Br$); 491.2 (16%, $[6-OH+C_4H_8{}^{79}BrO+Li]^+$, ber.: 491.3); 493.2 (15%, $[6-OH+C_4H_8{}^{81}BrO+Li]^+$, ber.: 493.3); 581.2 (95%, $[M+C_4H_8{}^{79}BrO+Li]^+$, ber.: 581.3); 582.3 (38%, $[M+C_4H_8{}^{79}BrO+Li]^+$, $^{13}C$, ber.: 582.3); 583.2 (100%, $[M+C_4H_8{}^{79}BrO+Li]^+$, ber.: 583.3); 584.2 (31%, $[M+C_4H_8{}^{81}BrO+Li]^+$, $^{13}C$, ber.: 584.3); 589.2 (68%, $^{79}Br$); 590.2 (25%, $^{79}Br$, $^{13}C$); 591.2 (64%, $^{81}Br$), 592.2 (19%, $^{81}Br$, $^{13}C$).

Beispiel 45

Isopropyl 2-O-heptyl-6-O-(4'-cyanobenzyl)-3-O-propyl-D-α/β-glucopyranosid

166 mg (0.1 mmol) des Polymers werden gemäß der allgemeinen Arbeitsvorschrift für eine Alkylierung mit Heptyliodid umgesetzt. Anschließend wird die TBDPS-Gruppe entfernt und danach mit 4-Cyanobenzylbromid alkyliert. Nach Abspaltung des Kohlenhydrates vom Harz und Filtration an Kieselgel erhält man 21 mg eines farblosen Öls.
$R_f$= 0.76, 0.74 (PE/EtOAc = 3:1 v/v).
$C_{27}H_{43}NO_6$ (477.6)

FBA-MS (NBA-pos, LiCl) : (m/z) = 408.2 (10%, $Gly^+$, ber.: 408.2); 470.3 (20%); 484.3 (21%, $[M+Li]^+$, ber.: 484.3); 582.4 (22%); 617.3 (24%, $^{79}Br$); 619.3 (24%, $^{81}Br$); 634.2 (85%, $[M+C_4H_8{}^{79}BrO+Li]^+$, ber.: 634.3); 635.2 (38%, $[M+C_4H_8{}^{79}BrO+Li]^+$, $^{13}C$, ber.: 635.3); 636.2 (92%, $[M+C_4H_8{}^{81}BrO+Li]^+$, ber.: 636.3); 637.2 (31%, $[M+C_4H_8{}^{81}BrO+Li]^+$, $^{13}C$, ber.: 637.3).

Beispiel 46

Methyl 2-O-heptyl-6-O-isopropyl-3-O-propyl-α/β-D-glucopyranosid

166 mg (0.1 mmol) des Polymers werden gemäß der allgemeinen Arbeitsvorschrift für eine Alkylierung mit Heptyliodid umgesetzt. Anschließend wird die TBDPS-Gruppe entfernt und danach mit n-Propyliodid alkyliert. Nach Abspaltung des Kohlenhydrates vom Harz und Filtration an Kieselgel erhält man 13 mg eines farblosen Öls.
$R_f$= 0.80, 0.72 (PE/EtOAc = 3:1 v/v).
$C_{20}H_{40}O_6$ (376.5)
FBA-MS (NBA-pos, LiCl) : (m/z) = 383.3 (36%, $[M+Li]^+$, ber.: 383.3); 397.3 (76%); 533.3 (19%, $[M+C_4H_8{}^{79}BrO+Li]^+$, ber.: 533.3); 535.3 (16%, $[M+C_4H_8{}^{81}BrO+Li]^+$, ber.: 535.3); 611.2 (40%).

Beispiel 47

Methyl 2-O-heptyl-6-O-(4'-brombenzyl)-3-O-propyl-D-glucopyranosid

166 mg (0.1 mmol) des Polymers werden gemäß der allgemeinen Arbeitsvorschrift für eine Alkylierung mit Heptyliodid umgesetzt. Anschließend wird die TBDPS-Gruppe entfernt und danach mit 4-Brombenzylbromid alkyliert. Nach Abspaltung des Kohlenhydrates vom Harz und Filtration an Kieselgel erhält man 16 mg eines farblosen Öls.
$R_f$= 0.84, 0.73 (PE/EtOAc = 3:1 v/v).
$C_{24}H_{39}BrO_6$ (503.5)
FBA-MS (NBA-pos, LiCl) : (m/z) = 453.4 (100%); 509.2 (41%, $[M+Li]^+$, $^{79}Br$, ber.: 509.3); 511.2 (37%, $[M+Li]^+$, $^{81}Br$, ber.: 511.3); 523.2 (84%, $^{79}Br$); 525.2 (83%, $^{81}Br$); 659.0 (34%, $[M+C_4H_8{}^{79}BrO+Li]^+$, $^{79}Br$, ber.: 659.3); 661.0 (59%, $[M+C_4H_8{}^{79}BrO+Li]^+$, $^{81}Br$, ber.: 661.3); 663.0 (31%, $[M+C_4H_8{}^{81}BrO+Li]^+$, $^{81}Br$, ber.: 663.3).

Beispiel 48

Methyl 2-O-ethyl-6-O-benzyl-3-O-propyl-α/β-D-glucopyranosid

166 mg (0.1 mmol) des Polymers werden gemäß der allgemeinen Arbeitsvorschrift für eine Alkylierung mit Ethyliodid umgesetzt. Anschließend wird die TBDPS-Gruppe entfernt und danach mit Benzylbromid alkyliert. Nach Abspaltung des Kohlenhydrates vom Harz und Filtration an Kieselgel erhält man 22 mg eines farblosen Öls.
$R_f$= 0.82, 0.77 (PE/EtOAc = 3:1 v/v).
$C_{19}H_{30}O_6$ (354.4)
FBA-MS (NBA-pos, LiCl) : (m/z) = 271.2 (7%, $[6\text{-}OH+Li]^+$, ber.: 271.2); 361.3 (15%, $[M+Li]^+$, ber.: 361.2); 421.2 (11%, $[6\text{-}OH+C_4H_8{}^{79}BrO+Li]^+$, ber.: 421.2); 423.2 (9%, $[6\text{-}OH+C_4H_8{}^{81}BrO+Li]^+$, ber.: 423.2); 449.3 (38%); 451.3 (41%); 511.3 (96%, $[M+C_4H_8{}^{79}BrO+Li]^+$, ber.: 511.2); 512.3 (36%, $[M+C_4H_8{}^{79}BrO+Li]^+$, $^{13}C$, ber.: 512.2); 513.3 (100%, $[M+C_4H_8{}^{81}BrO+Li]^+$, ber.: 513.2); 514.3 (26%, $[M+C_4H_8{}^{81}BrO+Li]^+$, $^{13}C$, ber.: 514.2).

Beispiel 49

Methyl 2-O-ethyl-6-O-(2'-methoxy-5'-nitrobenzyl)-3-O-propyl-α/β-D-glucopyranosid

166 mg (0.1 mmol) des Polymers werden gemäß der allgemeinen Arbeitsvorschrift für eine Alkylierung mit Ethyliodid umgesetzt. Anschließend wird die TBDPS-Gruppe entfernt und danach mit 2-Methoxy-5-nitrobenzylbromid alkyliert. Nach Abspaltung des Kohlenhydrates vom Harz und Filtration an Kieselgel erhält man 15 mg eines farblosen Öls.
$R_f$= 0.43 (PE/EtOAc = 3:1 v/v).
$C_{20}H_{31}NO_9$ (429.5)
FBA-MS (NBA-pos, LiCl) : (m/z) = 436.2 (9%, $[M+Li]^+$, ber.: 436.2); 586.1 (95%, $[M+C_4H_8{}^{79}BrO+Li]^+$, ber.: 586.2); 587.1 (35%, $[M+C_4H_8{}^{79}BrO+Li]^+$, $^{13}C$, ber.: 587.2); 588.1 (100%, $[M+C_4H_8{}^{81}BrO+Li]^+$, ber.: 588.2); 588.1 (28%, $[M+C_4H_8{}^{81}BrO+Li]^+$, $^{13}C$, ber.: 589.2).

Beispiel 50

Benzyl 2-O-ethyl-6-O-heptyl-3-O-propyl-α/β-D-glucopyranosid

166 mg (0.1 mmol) des Polymers werden gemäß der allgemeinen Arbeitsvorschrift für eine Alkylierung mit Ethyliodid umgesetzt. Anschließend wird die TBDPS-Gruppe entfernt und danach mit Methyliodid alkyliert. Nach Abspaltung des Kohlenhydrates vom Harz und Filtration an Kieselgel erhält man 116 mg eines farblosen Öls. Das Produkt enthält noch größere mengen Benzylalkohol.
$R_f$= 0.61, 0.54 (PE/EtOAc = 3:1 v/v).
$C_{25}H_{42}O_6$ (438.6)
FBA-MS (NBA-pos, LiCl) : (m/z) = 445.3 (29%, $[M+Li]^+$, ber.: 445.3); 595.3 (100%, $[M+C_4H_8{}^{79}BrO+Li]^+$, ber.: 595.3); 596.3 (40%, $[M+C_4H_8{}^{79}BrO+Li]^+$, $^{13}C$, ber.: 596.3); 597.3 (98%, $[M+C_4H_8{}^{81}BrO+Li]^+$, ber.: 597.3); 598.3 (31%, $[M+C_4H_8{}^{81}BrO+Li]^+$, $^{13}$ ber.: 595.3).

Beispiel 51

Ethyl 2-O-(2'-cyanobenzyl)-6-O-heptyl-3-O-propyl-α/β-D-glucopyranosid

166 mg (0.1 mmol) des Polymers werden gemäß der allgemeinen Arbeitsvorschrift für eine Alkylierung mit 2-Cyanobenzylbromid umgesetzt. Anschließend wird die TBDPS-Gruppe entfernt und danach mit Heptyliodid alkyliert. Nach Abspaltung des Kohlenhydrates vom Harz und Filtration an Kieselgel erhält man 26 mg eines farblosen Öls.
$R_f$= 0.78, 0.70 (PE/EtOAc = 3:1 v/v).
$C_{26}H_{41}NO_6$ (463.6)
FBA-MS (NBA-pos, LiCl) : (m/z) = 418.2 (12%, $Gly^+$, ber.: 418.3); 470.3 (100%, $[M+Li]^+$, ber.: 470.3); 471.3 (32%, $[M+Li]^+$, $^{13}C$, ber.: 471.3); 568.4 (78%); 603.3 (15%, $^{79}Br$); 605.3 (15%, $^{79}Br$); 620.2 (57%, $[M+C_4H_8{}^{79}BrO+Li]^+$, ber.: 620.3); 621.2 (25%, $[M+C_4H_8{}^{79}BrO+Li]^+$, $^{13}C$, ber.: 621.3); 622.2 (55%, $[M+C_4H_8{}^{81}BrO+Li]^+$, ber.: 622.3); 623.2 (19%, $[M+C_4H_8{}^{81}BrO+Li]^+$, $^{13}C$, ber.: 623.3).

Beispiel 52

Isopropyl 2-O-(2'-cyanobenzyl)-6-O-methyl-3-O-propyl-α/β-D-glucopyranosid

166 mg (0.1 mmol) des Polymers werden gemäß der allgemeinen Arbeitsvorschrift für eine Alkylierung mit 2-Cyanobenzylbromid umgesetzt. Anschließend wird die TBDPS-Gruppe entfernt und danach mit Methyliodid alkyliert. Nach Abspaltung des Kohlenhydrates vom Harz und Filtration an Kieselgel erhält man 21 mg eines farblosen Öls.
$R_f$= 0.52, 0.47 (PE/EtOAc = 3:1 v/v).
$C_{21}H_{31}NO_6$ (393.5)
FBA-MS (NBA-pos, LiCl) : (m/z) = 334.1 (8%, $Gly^+$, ber.: 334.2); 386.2 (12%); 400.2 (27%, $[M+Li]^+$, ber.: 400.2); 414.2 (12%); 449.2 (10%, $^{79}Br$); 451.2 (10%, $^{81}Br$); 550.2 (98%, $[M+C_4H_8{}^{79}BrO+Li]^+$, ber.: 550.2); 551.2 (36%, $[M+C_4H_8{}^{79}BrO+Li]^+$, $^{13}C$, ber.: 551.2); 552.2 (100%, $[M+C_4H_8{}^{81}BrO+Li]^+$, ber.: 552.2); 553.2 (28%, $[M+C_4H_8{}^{81}BrO+Li]^+$, $^{13}C$, ber.: 553.2).

Beispiel 53

Methyl 2,4-Di-O-benzyl-6-O-methyl-3-O-propyl-α/β-D-glucopyranosid

166 mg (0.1 mmol) des Polymers werden analog den oben beschrieben Versuchen in das Derivat umgewandelt. Für die Abspaltung der 1-Ethoxyethylschutzgruppe wird die Kohlenhydratmatrix wird in einer Mischung aus 4 ml Dioxan, 0.4 ml Alkohol (Methanol, Propanol, Oktanol oder Benzylalkohol) und einer Spatelspitze Pyridinium-toluol-4-sulfonat suspendiert. Anschließend wird die Spritze mit einer Plastikkappe verschlossen und für 16 h bei Raumtemp. geschüttelt. Im Anschluß daran wird das Harz fünfmal je 3 ml Dioxan p.a. sowie zweimal mit 3 ml DMF gewaschen.
Nach der Abspaltung der 1-Ethoxyethylschutzgruppe wird die 4-Position mit Benzylbromid alkyliert und das Kohlenhydrat von Harz abgespalten Man erhält 12-16 mg eines farblosen Öls. Da alle Rohprodukte laut DC, HPLC und MS identische Komponenten enthalten, werde sie vereinigt und durch Chromatographie an Kieselgel mit Petrolether/Ethylacetat (12:1) gereinigt.
$C_{25}H_{34}O_6$ (430.6)
FAB-MS (NBA-pos, LiCl) : (m/z) = 437.1 (100%, $[M+Li]^+$, ber.: 437.2); 438.1 (28%, $[M+Li]^+$, $^{13}C$, ber.: 438.2); 513.3 (28%, $[C_{31}H_{38}O_6+Li]^+$ □ 6-OBn, ber.: 506.3).

400 MHz-$^1$H-NMR ($CDCl_3$) : δ [ppm] = 7.50-7.25 (m, 10H, Ph); 4.86 (d, 1H, $J_{gem}$ = 10.86 Hz, $CH_2Ph$); 4.77 (d, 1H, $J_{gem}$ = 12.32, $CH_2Ph$); 4.60 (d, 1 H, $J_{gem}$ =12.03 Hz, $CH_2Ph$); 4.56 (d, 1H, $J_{gem}$ = 10.86 Hz, $CH_2Ph$); 4.52 (d, 1H, $J_{2,1}$= 3.81 Hz, H-1); 3.87-3.24 (m, 8H, H-2, H-3, H-4, H-5, H-6a/b, $OCH_2$ Pr); 3.32, 3.30 (2x s, 6H, $OCH_3$); 1.66-1.61 (m, 2H, $OCH_2CH_2$ Pr); 0.92 (t, $J_{gem}$ = 7.48 Hz, $CH_3$ Pr).
6-Benzylderivat :
400 MHz-$^1$H-NMR ($CDCl_3$) : δ [ppm] = 7.51-7.26 (m, 15H, Ph); 4.86 (d, 1H, $J_{gem}$ = 11.15 Hz, $CH_2Ph$); 4.80 (d, 1H, $J_{gem}$ = 10.57 Hz, $CH_2Ph$); 4.67 (d, 1H, $J_{gem}$ = 11.15 Hz, $CH_2Ph$); 4.61-4.54 (m, 2H, $CH_2Ph$); 4.50 (d, 1 H, $J_{gem}$ = 10.57 Hz, $CH_2Ph$); 4.25-4.18 (m, 3H, H-1 & $OCH_2$ Pr); 3.80-3.29 (m, 6H, H-2, H-3, H-4, H-5, H-6a/b); 3.54 (s, 3H, $OCH_3$); 1.68-1.60 (m, 2H, $OCH_2CH_2$ Pr); 0.91-0.86 (m, 3H, $CH_3$ Pr).

Beispiel 54

Methyl 2-O-benzyl-4-O-(2'-bromo-1'-ethoxy)-ethyl-6-O-methyl-3-O-propyl-α/β-Dglucopyranosid

166 mg (0.1 mmol) des Polymers wird analog den oben beschrieben Versuchen in das Derivat umgewandelt. Durch eine Behandlung des Harzes mit verschiedenen Kombinationen von Lösungsmitteln und 1 M Zitronensäure (4 ml DMF + 0.4 ml 1M Zitronensäure; 4 ml DMF + 0.4 ml 1 M Zitronensäure + 4 h Ultraschall; 4 ml DMF + 0.04 ml 1M Zitronensäure; 4 ml Dioxan + 0.04 ml 1M Zitronensäure; 4 ml $CH_2Cl_2$ + 1 ml Aceton + 0.1 ml 1 M Zitronensäure) wird versucht, die 1-Ethoxyethylschutzgruppe abzuspalten. Anschließend wird das Harz einer Benzylierung unterworfen und das-Kohlenhydrat vom polymeren Träger abgespalten Man erhält 13-17 mg eines farblosen Öls. Da alle Rohprodukte laut DC, HPLC und MS identische Komponenten enthalten, werde sie vereinigt und durch Chromatographie an Kieseigel mit Petrolether/Ethylacetat (8:1) gereinigt. $C_{22}H_{35}BrO_7$ (491.4)
FAB-MS (NBA-pos, LiCl) des Rohproduktes : (m/z) = 497.4 (100%, $[M+Li]^+$, $^{79}$Br, ber.: 497.2); 498.4 (31%, $[M+Li]^+$, $^{79}$Br, $^{13}$C, ber.. 498.2); 499.4 (98%, $[M+Li]^+$, $^{81}$Br, ber.: 499.2); 500.4 (22%, $[M+Li]^+$, $^{81}$Br, $^{13}$C, ber.: 500.2); 573.2 (28%, $[C_{28}H_{39}{}^{79}BrO_7+Li]^+$ □ 6-OBn, ber.: 573.3); 575.2 (31%, $[C_{28}H_{39}{}^{81}BrO_7+Li]^+$ □ 6-OBn, ber.: 575.3).
1. Diastereomer:
400 MHz-$^1$H-NMR ($CDCl_3$) : δ [ppm] = 7.33-7.25 (m, 5H, Ph); 4.87-4.84 (m, 1H, $CHCH_2$ EEBr); 4.72 (d, 1H, $J_{gem}$ = 12.03 Hz, $OCH_2Ph$); 4.64 (d, 1 H, $J_{2,1}$= 3.82 Hz, H-1); 4.56 (d, 1H, $J_{gem}$= 12.03 Hz, $OCH_2Ph$); 3.90-3.85 (m, 1 H, $OCH_2$); 3.75-3.54 (m, 6H, H-2, H-6a/b, $OCH_2$); 3.52 (s, 3H, $OCH_3$); 3.48-3.31 (m, 5H, H-3, H-4, H-5 & $CH_2Br$ EEBr); 3.36 (s, 3H, $OCH_3$); 1.63-1.57 (m, 2H, $OCH_2CH_2$ Pr); 1.25-1.18 (m, 3H, $OCH_2CH_3$ EEBr & ); 0.89 (t, $J_{gem}$ = 7.49 Hz, $CH_3$ Pr).
400 MHz-$^1$H-NMR ($CDCl_3$) : δ [ppm] = 7.34-7.26 (m, 5H, Ph); 4.97-4.95 (m, 1 H, $CHCH_2$ EEBr); 4.72 (d, 1 H, $J_{gem}$= 12.03 Hz, $OCH_2Ph$); 4.66 (d, 1H, $J_{2,1}$= 3.52 Hz, H-1); 4.56 (d, 1H, $J_{gem}$= 12.03 Hz, $OCH_2Ph$); 3.93-3.87 (m, 1H, $OCH_2$); 3.70-3.57 (m, 6H, H-2, H-6a/b, $OCH_2$); 3.54-3.30 (m, 5H, H-3, H-4, H-5 & $CH_2Br$ EEBr); 3.49 (s, 3H, $OCH_3$); 3.36 (s, 3H, $OCH_3$); 1.64-1.59 (m, 2H, $OCH_2CH_2$ Pr); 1.23-1.19 (m, 3H, $OCH_2CH_3$ EEBr & ); 0.89 (t, $J_{gem}$ = 6.75 Hz, $CH_3$ Pr).
HPLC (Gradient 90/10) :
Während der Chromatographie kann das in 6-Position benzylierte Derivat Methyl 2,6-Di-O-benzyl-4-O-(2□-bromo-1□-ethoxy)-ethyl-3-O-propyl-D-glucopyranosid, welches schon in dem FAB-Massenspektrum beobachtet wurde, abgetrennt werden.
$C_{28}H_{39}BrO_7$ (567.51)
400 MHz-$^1$H-NMR ($CDCl_3$) : δ [ppm] = 7.33-7.25 (m, 10H, Ph); 4.96-4,49 (m, 6H, H-1, $OCH_2Ph$ & $CHCH_2Br$ EEBr); 3.92-3.10 (m, 12H, H-2, H-3, H-4, H-5, H-6a/b, $OCH_2$ & $CH_2Br$ EEBr), 3.36, 3.33 (s, 3H, $OCH_3$); 1.64-1.54 (m, 2H, $OCH_2CH_2$ Pr); 1.25-1.19 (m, 3H, $OCH_2CH_3$ EEBr & ); 0.89 (t, $J_{gem}$ = 7.49 Hz, $CH_3$ Pr).

Beispiel 55

Methyl 2-O-benzyl-4-O-tert.-butyloxycarbonylmethyl-6-O-methyl-3-O-propyl-α/β-Dglucopyranosid

166 mg (0.1 mmol) des Polymers werden gemäß der allgemeinen Arbeitsvorschrift für eine Alkylierung mit Benzylbromid umgesetzt. Anschließend wird die TBDPS-Gruppe entfernt und danach mit Methyliodid alkyliert. Nachdem die 1-Ethoxyethylschutzgruppe abgespalten wurde wird mit Bromessigsäure-tert.-butylester umgesetzt Nach Abspaltung des Kohlenhydrates vom Harz und Filtration an Kieselgel erhält man 23 mg eines farblosen Öls. $R_f$ = 0.64, 0.59 (PE/EtOAc = 3:1 v/v). $C_{24}H_{38}O_8$ (454.6).
FAB-MS (NBA-pos, LiCl) : (m/z) = 461.3 (75%, $[M+Li]^+$, ber.: 461.3); 462.3 (23%, $[M+Li]^+$, $^{13}$C, ber.: 462.3); 561.3 (100%, $[C_{29}H_{46}O_{10}+Li]^+$ □ 6-$OCH_2CO_2$tBu, ber.: 561.3); 562.3 (33%, $[C_{29}H_{46}O_{10}+Li]^+$, $^{13}$C , ber.: 562.3).

Beispiel 56

Bibliothek mit 1,2,6-funktionalisierten Glycosederivaten (Verbindung der Formel I mit X gleich O, $R^3$ gleich Propyl und $R^4$ gleich Wasserstoff)

80 mg des beladenen Harzes wurden gemäß den allgemeinen Arbeitsvorschriften funktionalisiert (Tabelle1)

Beispiel 57

Bibliothek mit 1,2,4,6-funktionaliserten Glycosederivaten (Verbindung der Formel I mit X gleich O, $R^1$ gleich Methyl, $R^3$ gleich Propyl)

80 mg des beladenen Harzes werden gemäß den allgemeinen Arbeitsvorschriften funktionalisiert. Die Alkylierung an der 4-Position gelingt durch Verwendung von Bromessigsäure-tert.-butylester.

| Verbindung | $R^2$ | $R^4$ | $R^5$ | MS-Analyse (m/z) (FAB, NBA+LiCl) |
|---|---|---|---|---|
| 1 | Bn | Bn | Me | 437.1 |
| 2 | Bn | $CH_2CO_2tB$ | Me | 461.3 |

Tabelle 1

| Verbindung | $R^2$ | $R^5$ | $R^1$ | MS-Analyse (m/z) (FAB, NBA+LiCl) |
|---|---|---|---|---|
| 1 | Me | Bn | Me | 347.2 |
| 2 | Me | Me | Bn | 347.2 |
| 3 | Me | Hep | Me | 355.2 |
| 4 | Me | Hep | iPr | 383.2 |
| 5 | Me | MNBn Et | Et | 436.1 |
| 6 | Bn | iPr | Me | 375.1 |
| 7 | Bn | Cbn | Et | 462.2 |
| 8 | Bn | Hep | Me | 431.3 |
| 9 | Bn | cHex | iPr | 457.3 |
| 10 | Bn | iBu | Me | 389.2 |
| 11 | Pr | Cbn | Me | 400.2 |
| 12 | Pr | Bn | iPr | 403.2 |
| 13 | Pr | Me | Bn | 375.2 |
| 14 | Pr | Hep | Me | 383.3 |
| 15 | Pent | iPr | Me | 355.2 |
| 16 | Pent | Hep | Et | 425.3 |
| 17 | Pent | Me | iPr | 355.3 |
| 18 | Hep | cHex | Bn | 513.3 |
| 19 | Hep | Bn | Me | 431.3 |
| 20 | Hep | Cbn | iPr | 484.3 |
| 21 | Hep | iPr | Me | 383.3 |
| 22 | Hep | BrBn | Me | 509.2 |
| 23 | Et | Bn | Me | 361.3 |
| 24 | Et | MNBn | Me | 436.2 |
| 25 | Et | Hep | Bn | 445.3 |
| 26 | Cbr | iPr | Me | 400.3 |
| 27 | Cbr | Hep | Et | 470.3 |
| 28 | Cbr | Me | iPr | 400.2 |
| Abkürzungen : MNBn = 2-Methoxy-5-nitrobenzyl, CBn = o-Cyanobenyzl, Pent = Pentyl, cHex = Cyclohexylmethylen, | | | | |

Beispiel 58

S-(6'-O-tert-Butyldiphenylsilyl-3',4'-O-isopropyliden-β-D-galactopyranosyl-2'-O -methyl)-4-mercaptobuttersäuremethylester (32)

Eine Lösung von 3.0 g (5.2 mmol)19 in 30 ml THF wird mit 0.6 g (5.3 mmol) Kalium-*tert*-butylat unter einer Argonatmosphäre 45 min bei 0 °C gerührt und anschließend mit 0.38 ml (6.0 mmol) Methyliodid versetzt. Da durch dünnschichtchromatographische Kontrolle nach wenigen Stunden nur wenig Umsatz erkennbar ist, wird nochmals die gleiche Menge an Reagenzien zugesetzt. Der ausfallende Feststoff wird durch Zusatz von 15 ml DMF in Lösung gebracht. Nach Rühren für 12 h wird i. Vak. eingeengt, mit Toluol kodestilliert und durch Flash-Chromatographie an Kieselgel gereinigt (Säule 18 × 6 cm, Laufmittel Petrolether/Ethylacetat 10:1). Ausb. 1.8 g (59 %), farbloses Öl, $[\alpha]_D^{25}$ = -15.9 (c = 1, $CHCl_3$); $R_F$ = 0.34 (Petrolether/Ethylacetat 8:1).
400 MHz-$^1$H-NMR ($CDCl_3$): δ[ppm] = 7.69-7.66; 7.41-7.33 (m, 10H, $SiPh_2$), 4.28 (d, 1 H, $J_{1,2}$ = 9.7 Hz, 1'-H), 4.27 (dd, 1 H, $J_{4,3}$ = 5.5 Hz, $J_{4,5}$ = 2.3 Hz, 4'-H), 4.09 (dd, 1 H, $J_{3,2}$ = 7.0 Hz, $J_{3,4}$ = 5.6 Hz, 3'-H), 3.89 (m, 2H, 6'-$H_{a,b}$), 3.79 (ddd, 1 H, $J_{5,4}$ = 2.3 Hz, $J_{5,6a} \approx J_{5,6b}$ = 6.2 Hz, 5'-H), 3.59; 3.55 (2s, 6H, $OCH_3$), 3.17 (dd, 1 H, $J_{2,1}$ = 10.0 Hz, $J_{2,3}$ = 6.8 Hz, 2'-H), 2.77 (dt, 1H, $J_{vic}$ = 6.5 Hz, $J_{gem}$ = 12.9 Hz, $SCH_a$), 2.64 (dt, 1 H, $J_{vic}$ = 6.5 Hz, $J_{gem}$ = 12.9 Hz, $SCH_b$), 2.39 ($m_c$, 2H, $CH_2COOMe$), 1.91 ($m_c$, 2H, $SCH_2CH_2$), 1.50; 1.33 (2s, 6H, $C(CH_3)_2$), 1.03 (s, 9H, $SiC(CH_3)_3$.
100.6 MHz-$^{13}$C-NMR ($CDCl_3$): δ[ppm] = 173.3 (CO), 135.6; 135.5 ($C_p$-$SiPh_2$), 133.5; 133.4 ($C_r$-$SiPh_2$), 129.7; 127.7; 127.6 ($C_{o,m}$-$SiPh_2$), 109.7 ($C(CH_3)_2$), 83.6; 81.8; 79.4; 76.8; 73.4; 62.8 (C-1'-C-6'), 59.7 ($OCH_3$), 51.4 ($COOCH_3$), 32.7 ($SCH_2$), 29.6 ($CH_2COOMe$), 28.0 ($C(CH_3)_2$), 26.7 ($SiC(CH_3)_3$), 26.2 ($C(CH_3)_2$), 25.0 ($SCH_2CH_2$), 19.2 ($SiC(CH_3)_3$).

| | | | | |
|---|---|---|---|---|
| $C_{31}H_{44}O_7SSi$ (588.8) | Ber. | C 63.23 | H 7.53 | S 5.44 |
| | Gef. | C 63.09 | H 7.61 | S 5.41 |

Beispiel 59

*S*-(6'-*O*-*tert*-Butyldiphenylsilyl-3',4'-*O*-isopropyliden-β-D-galactopyranosyl)-4-mercaptobuttersäure-polymergebunden (33, 34)

Das Thiogalactosid 19 wird nach der allgemeinen Vorschrift auf 0.390 g (0.6 mmol) Aminomethylpolystyrol zu 33 gekuppelt.
Beladung nach Schwefel-Anteil der Elementaranalyse: 0.77-0.81 mmol/g. Das Thiogalactosid 19 wird nach der allgemeinen Vorschrift auf 2.0 g (0.56 mmol) Tentagel zu 34 gekuppelt.
Beladung (gravimetrisch): 0.20 mmol/g.

Beispiel 60

*S*-(6'-*O*-*tert*-Butyldiphenylsilyl-3',4'-*O*-isopropyliden-2'-*O*-β-D-galactopyranosyl)-4-mercaptobuttersäure-polymergebunden (35, 36)

Das 2-*O*-Methylthiogalactosid 32 wird nach der allgemeinen Arbeitsvorschrift auf 1.0 g (0.6 mmol) Aminomethylpolystyrol zu 35 gekuppelt.
Beladung nach Schwefelanteil der Elementaranalyse:. 0.78 mmol/g.
Das 2-*O*-Methylthiogalactosid 32 wird nach der allgemeinen Arbeitsvorschrift auf 2.0 g (0.56 mmol) Tentagel zu 36 gekuppelt.
Beladung (gravimetrisch): 0.27 mmol/g.

Beispiel 61

Methyl-6-O-tert-butyldiphenylsilyl-3,4-O-isopropyliden-2-O-methyl-α/β-Dgalactopyranosid

a) durch Abspaltung von 35: Nach der allgemeinen Vorschrift werden 400 mg (0.312 mmol) 79 mit Methanol als Alkohol behandelt. Die Reinigung erfolgt durch Flash-Chromatographie an Kieselgel (Säule 18 □4 cm, Laufmittel Petrolether/Ethylacetat 8:1).
Ausb. 70 mg (24 %) α-Anomer, farbloses Öl; 134 mg (46 %) β-Anomer, farbloses Öl. Durch nochmaliges Nachwaschen des Harzes und Vereinigen mit den Mischfraktionen werden weitere 43 mg (15 %) als Anomerengemisch erhalten.

b) durch Abspaltung von 36: Nach der allgemeinen Vorschrift wird 80 mit Methanol als Alkohol glycosyliert. Die Reinigung erfolgt durch Flash-Chromatographie an Kieselgel (Säule 18 × 4 cm, Laufmittel Petrolether/Ethylacetat 8: 1 ).

Ausb. 12 mg (4 %) α-Anomer, farbloses Öl; 44 mg (15 %) β-Anomer, farbloses Öl. Daneben werden 47 mg (17 %) Hydrolysezucker als Anomerengemisch isoliert.

c) durch Alkylierung mit Natriumhydrid/Methyliodid: 370 mg (0.3 mmol) 33 werden in 15 ml DMF/THF (1:1) suspendiert und mit 0.090 g (3.0 mmol) Natriumhydrid (80 % in Mineralöl) 20 min unter einer Argonatmosphäre bei Raumtemp. vorgeschüttelt. Nach Zugabe von 0.19 ml (3.0 mmol) Methyliodid schüttelt man 12 h, filtriert nach Zugabe von 5 ml Methanol ab und wäscht mehrfach mit DMF nach. Die Abspaltung erfolgt nach der allgemeinen Vorschrift mit Methanol als Alkohol. Die Reinigung erfolgt durch Flash-Chromatographie an Kieselgel (Säule 15 x 3 cm, Laufmittel Petrolether/Ethylacetat 8:1).

Ausb. 92 mg (32 %) α-Anomer, klares Öl; 43 mg (15 %) β-Anomer, klares Öl.

Daneben werden 14 mg (5 %) α-Anomer und 32 mg (11 %) β-Anomer des nicht alkylierten Methylglycosids isoliert.

d) durch Alkylierung mit KOtBu/Methyliodid: Wie unter c) bei 0 °C, 4 h.

Ausb. 55 mg (19 %), klares Öl, Anomerengemisch.

Beispiel 62

Methyl-6-O-tert-butyldiphenylsilyl-3,4-O-isopropyliden-2-O-benzyl-β-Dgalactopyranosid

a) durch Alkylierung von 33 mit Natriumhydrid/Benzylbromid: 768 mg (0.6 mmol) 33 werden wie oben bei Raumtemp. unter einer Argonatmosphäre 12 h mit 180 mg (6.0 mmol) Natriumhydrid (80 % in Mineralöl) und 0.72 ml (6.0 mmol) Benzylbromid zur Reaktion gebracht und abgespalten. Die Reinigung erfolgt durch *Flash*-Chromatographie an Kieselgel (Säule 15 × 4 cm, Laufmittel Petrolether/Ethylacetat 15:1).

Ausb. 67 mg (20 %) α-Anomer, klares Öl; 26 mg (8 %) β-Anomer, trübes Öl. Daneben werden 4 mg (2 %) als Anomerengemisch erhalten.

*b)* durch Alkylierung von 34 mit Natriumhydrid/Benzylbromid: 2.5 g (0.5 mmol) 34 werden wie oben bei Raumtemp. unter einer Argonatmosphäre 12 h mit 900 mg (30.0 mmol) Natriumhydrid (80 % in Mineralöl) und 3.6 ml (30.0 mmol) Benzylbromid zur Reaktion gebracht und abgespalten. Die Reinigung erfolgt durch *Flash* Chromatographie an Kieselgel (Säule 18 x 3 cm, Laufmittel Petrolether/Ethylacetat 10:1).

Ausb. 100 mg (37 %) Anomerengemisch, gelbes Öl.

c) durch Alkylierung mit Kalium-tert-butylat/Benzylbromid: 640 mg (0.5 mmol) 33 werden in 20 ml DMF gequollen. Nach Zusatz von 0.56 g (5.0 mmol) Kalium-*tert*-butylat wird unter einer Argonatmosphäre 20 min geschüttelt. Anschließend werden 0.59 ml (5.0 mmol) Benzylbromid und 0.22 g (0.6 mmol) Tetrabutylammoniumiodid zugegeben und die Mischung 16 h geschüttelt. Man setzt 20 ml Methanol zu, filtriert ab und wäscht mehrmals mit DMF und absol. THF. Das Harz wird i. Vak. getrocknet. Die Abspaltung erfolgt nach der allgemeinen Vorschrift mit Methanol als Alkohol. Die Reinigung erfolgt durch *Flash*-Chromatographie an Kieselgel (Säule 18 × 3 cm, Laufmittel Petrolether/Ethylacetat 8:1).

Ausb. 124 mg (44 %) α-Anomer, klares Öl; 22 mg (8 %) β-Anomer, farbloses Öl.

*d*) durch Alkylierung mit Phosphazen-Base $P_4$-t-Bu/Benzylbromid: 384 mg (0.33 mmol) 33 werden in 10 ml DMF gequollen und unter einer Argonatmosphäre auf 0 °C gekühlt. Man setzt 1.32 ml (1.32 mmol, 1 M in *n*-Hexan) $P_4$-*tert*-Bu zu und läßt schütteln. Nach 15 min gibt man 0.59 ml (5.0 mmol) Benzylbromid zu und läßt bei 0 °C 16 h schütteln. Das Harz wird abfiltriert und mehrfach mit DMF und absol. THF gewaschen. Nach Trocknen i. Vak. wird nach der allgemeinen Vorschrift mit Methanol als Alkohol vom Träger abgespalten. Die Reinigung erfolgt durch *Flash*-Chromatographie an Kieselgel (Säule 18 × 3 cm, Laufmittel Petrolether/Ethylacetat 10:1).

Ausb. 163 mg (85 %) Anomerengemisch (α.β ca. 5:1), farbloses Öl.

α-*Anomer:*

$[\alpha]_D^{25}$ = +44.9 ($c$ = 1, $CHCl_3$); $R_F$ = 0.20 (Petrolether/Ethylacetat 8:1).

β-*Anomer:*

$[\alpha]_D^{25}$ = +17.2 ($c$ = 1, $CHCl_3$); $R_F$ = 0.30 (Petrolether/Ethylacetat 8:1).

Beispiel 63

Methyl-2-O-(o-cyanobenzyl)-6-O-tert-butyldiphenylsilyl-3,4-O-isopropyliden-α/β-Dgalactopyranosid

400 mg (0.312 mmol) 33 werden nach der allgemeinen Arbeitsvorschrift (Variante A) mit o-Cyanobenzylbromid umgesetzt und mit Methanol glycosylierend abgespalten. Das Rohprodukt wird durch Flash-Chromatographie an Kieselgel gereinigt (Säule 20 □ 2 cm, Laufmittel Petrolether/Ethylacetat 12:1).
α-Anomer:
Ausb. 26 mg (14 %), farbloses Öl, $[\alpha]_D^{25}$ = +50.5 (c = 1.0, $CHCl_3$); $R_F$ = 0.39 (Petrolether/Ethylacetat 4:1), HPLC (Säule C, Gradient 1): 20.91 min.
β-Anomer:
Ausb. 12 mg (7 %), farbloses Öl, $[\alpha]_D^{25}$ = +19.6 (c = 0.33, $CHCl_3$); $R_F$ = 0.46 (Petrolether/Ethylacetat 4:1), HPLC (Säule C, Gradient 1): 20.91 min.

Beispiel 64

Methyl-2-O-(p-bromobenzyl)-6-O-tert-butyldiphenylsilyl-3,4-O-isopropyliden-α/β-Dgalactopyranosid

400 mg (0.312 mmol) 33 werden nach der allgemeinen Arbeitsvorschrift mit *p*-Brombenzylbromid umgesetzt und mit Methanol glycosylierend abgespalten. Das Rohprodukt wird durch Flash-Chromatographie an Kieselgel gereinigt (Säule 20 × 2 cm, Laufmittel Petrolether/Ethylacetat 12:1).
α-*Anomer:*
Ausb. 12 mg (6 %), farbloses Öl, $[\alpha]_D^{25}$ = +38.4 (*c* = 1.0, $CHCl_3$); $R_F$ = 0.17 (Petrolether/Ethylacetat 12:1), HPLC (Säule C, Gradient 1): 22.13 min.
β-*Anomer:*
Ausb. 3 mg (2 %), farbloses Öl, $[\alpha]_D^{25}$ = +39.3 (*c* = 0.1, $CHCl_3$); $R_F$ = 0.23 (Petrolether/Ethylacetat 12:1), HPLC (Säule C, Gradient 1): 22.13 min.

Beispiel 65

Methyl-2-O-ethyl-6-O-tert-butyldiphenylsilyl-3,4-O-isopropyliden-α/β-Dgalactopyranosid

400 mg (0.312 mmol) 33 werden nach der allgemeinen Arbeitsvorschrift mit Ethyliodid umgesetzt und mit Methanol glycosylierend abgespalten. Das Rohprodukt wird durch *Flash*-Chromatographie an Kieselgel gereinigt (Säule 15 × 2.5 cm, Laufmittel Petrolether/Ethylacetat 12:1).
α-*Anomer:*
Ausb. 22 mg (14 %), farbloses Öl, $[\alpha]_D^{25}$ = +73.2 (c = 1, $CHCl_3$); $R_F$ = 0.49 (Petrolether/ Ethyl- acetat 4:1), HPLC (Säule C, Gradient 1): 19.62 min.
β-*Anomer:*
Ausb. 10 mg (7 %), farbloses Öl, $[\alpha]_D^{25}$ = +1.1 (c = 0.33, $CHCl_3$); $R_F$ = 0.49 (Petrolether/Ethylacetat 4:1 ), HPLC (Säule C, Gradient 1): 19.62 min.

Beispiel 66

S-(2'-O-Benzyl-3',4'-O-isopropyliden-α/β-D-galactopyranosyl)-4-mercaptobuttersäure-polymergebunden

Man alkyliert 1 g (0.78 mmol) 33 nach der allgemeinen Vorschrift (Variante A) mit Benzylbromid und entfernt die Silylschutzgruppe. Das Polymer wird i. Vak. getrocknet.

Beispiel 67

Methyl-2-O-benzyl-6-O-(2'-naphtylmethyl)-3,4-O-isopropyliden-α/β-Dgalactopyranosid

400 mg (0.312 mmol) S-(2'-O-Benzyl-3',4'-O-isopropyliden-α/β-D-galactopyranosyl)-4-mercaptobuttersäure-polymergebunden werden nach den allgemeinen Arbeitsvorschriften mit 2-Brommethyl-naphtalin umgesetzt und mit Methanol glycosylierend abgespalten. Das Reinprodukt wird durch Flash-Chromatographie an Kieselgel (Säule 20 × 2 cm, Laufmittel Petrolether/Ethylacetat 4:1) als Anomerengemisch im Verhältnis α.β= 10:1 erhalten.
Ausb. 40 mg (38 %), gelbes Öl, $[\alpha]_D^{25}$ = +79.2 (*c* = 1, $CHCl_3$); $R_F$ = 0.25 (Petrolether/Ethylacetat 4:1)); HPLC (Säule

C, Gradient 1): 15.28 min.

Beispiel 68

Methyl-2-*O*-propyl-6-*O*-*tert*-butyldiphenylsilyl-3,4-*O*-isopropyliden-α/β-Dgalactopyranosid

400 mg (0.312 mmol) 33 werden nach der allgemeinen Arbeitsvorschrift mit Propyliodid umgesetzt und mit Methanol glycosylierend abgespalten. Das Rohprodukt wird durch *Flash*-Chromatographie an Kieselgel gereinigt (Säule 17 × 2.5 cm, Laufmittel Petrolether/Ethylacetat 12:1).
α-Anomer:
Ausb. 7 mg (5 %), farbloses Öl, $[\alpha]_D^{25}$ = +56.9 (c = 0.2, $CHCl_3$); $R_F$ = 0.51 (Petrolether/Ethylacetat 4:1), HPLC (Säule C, Gradient 1): 21.03 min.
β-Anomer:
Ausb. 2 mg (2 %), farbloses Öl, $[\alpha]_D^{25}$ = +2.7 (c = 0.1, $CHCl_3$); $R_F$ = 0.57 (Petrolether/Ethylacetat 4:1), HPLC (Säule C, Gradient 1): 19.62 min.

Beispiel 69

Methyl-2-O-heptyl-6-O-tert-butyldiphenylsilyl-3,4-O-isopropyliden-α/β-Dgalactopyranosid

400 mg (0.312 mmol) 33 werden nach der allgemeinen Arbeitsvorschrift (Variante A) mit Heptyliodid unter Zusatz von 18-Krone-6 umgesetzt und mit Methanol glycosylierend abgespalten. Das Rohprodukt wird durch *Flash*-Chromatographie an Kieselgel gereinigt (Säule 20 × 2 cm, Laufmittel Petrolether/Ethylacetat 15:1).
α-Anomer:
Ausb. 12 mg (7 %), farbloses Öl, $[\alpha]_D^{25}$ = +52.6 (c = 0.6, $CHCl_3$); $R_F$ = 0.34 (Petrolether/Ethylacetat 10:1), HPLC (Säule C, Gradient 1): 24.27 min.
β-Anomer:
Ausb. 8 mg (5 %) farbloses Öl, $[\alpha]_D^{25}$ = +14.6 (*c* = 0.4, $CHCl_3$); $R_F$ = 0.40 (Petrolether/Ethylacetat 10:1), HPLC (Säule C (C8), Gradient 1): 24.27 min.

Beispiel 70

Ethyl-3,4-O-isopropyliden-1-thio-β-D-galactopyranosid

Eine Mischung aus 19.1 g (85.4 mmol) Ethyl-1-thio-β-D-galactopyranosid , 360 ml (2.93 mol) Acetondimethylacetal und 0.3 g (16 mmol) *p*-Toluolsulfonsäure-Monohydrat wird bei Raumtemp. gerührt. Nach 18 h setzt man 1.2 ml Triethylamin zu und engt i. Vak. zur Trockene ein. Der Rückstand wird in 180 ml Dichlormethan suspendiert und mit 2.4 ml 50%iger Trifluoressigsäure versetzt. Nach 15 min werden 3.6 ml Triethylamin zugegeben und die Mischung i. Vak. eingeengt. Das erhaltene Öl wird durch *Flash*-Chromatographie an Kieselgel gereinigt (Säule 30 × 10 cm, Laufmittel Petrolether/Ethylacetat 2:3).
Ausb. 15.7 g (70 %), farblose Kristalle, Schmp. 89 °C, [α]= +16.5 (c = 1, $CHCl_3$); $R_F$ = 0.33 (Petrolether/Ethylacetat 1:9).

Beispiel 71

Ethyl-6-O-tert-butyldiphenylsilyl-3,4-O-isopropyliden-1-thio-β-D-galactopyranosid

Eine Lösung von 9.00 g (34 mmol) Beispielverbindung 70 und 4.62 g (68 mmol) Imidazol in 100 ml absol. DMF wird mit 10.9 ml (43 mmol) *tert*-Butyldiphenylsilylchlorid versetzt und bei Raumtemp. 3 h gerührt. Die Reaktion wird durch Zugabe von 50 ml Wasser abgebrochen. Man fügt 150 ml Dichlormethan zu und wäscht die organische Phase dreimal mit je 50 ml Wasser. Die vereinigten wäßrigen Phasen werden mit 100 ml Dichlormethan extrahiert, die vereinigten organischen Phasen über Magnesiumsulfat getrocknet und das Lösungsmittel i. Vak. entfernt. Das erhaltene Öl wird an Kieselgel chromatographiert (Säule 20 × 5 cm, Laufmittel Petrolether/Ethylacetat 4:1). Ausb. 12.66 g (74 %), farblose Kristalle, $[\alpha]_D^{25}$ = +0.7 (c = 1, $CHCl_3$); $R_F$ = 0.28 (Petrolether/Ethylacetat 4:1).

Beispiel 72

Ethyl-2-*O*-acetyl-3,4-*O*-isopropyliden-6-*O*-*tert*-butyldiphenylsilyl-1-thio-β-Dgalactopyranosid

Zu einer Lösung von 12.6 g (25 mmol) Beispielverbindung 71 in 100 ml absol. Pyridin tropft man unter Eiskühlung 50 ml (520 mmol) Acetylchlorid. Nach 18 h destilliert man das Lösungsmittel i. Vak. ab, nimmt in 150 ml Dichlormethan auf, wäscht sukzessive mit je 50 ml 0.5 N Salzsäure, ges. Natriumhydrogencarbonat-Lösung und ges. Natriumchlorid-Lösung und trocknet die organische Phase über Magnesiumsulfat. Nach Einengen i. Vak. wird durch Chromatographie an Kieselgel von Verunreinigungen abgetrennt (Säule 25 × 5 cm, Laufmittel Petrolether/Ethylacetat 4:1). Das ölige Produkt erstarrt nach mehreren Tagen. Ausb. 13.6 g (8:3 %), farblose Kristalle, Schmp. 76 °C, $[\alpha]_D^{25}$ = +15.6 ($c$ = 1, $CHCl_3$); $R_F$ = 0.62 (Petrolether/Ethylacetat 4:1).

Beispiel 73

Ethyl-2-O-acetyl-6-O-tert-butyldiphenylsilyl-1-thio-β-D-galactopyranose

a) durch Deacetalisierung mit Essigsäure: Eine Lösung von 140 mg (0.26 mmol) Beispielverbindung 72 in 20 ml 60%iger Essigsäure wird unter Rühren für 2 h auf 60 °C erwärmt. Man engt i. Vak. ein und kodestilliert mit 10 ml Toluol. Der Rückstand wird in 20 ml Dichlormethan aufgenommen und mit 10 ml ges. Natriumhydrogencarbonat-Lösung gewaschen. Das Rohprodukt wird durch Chromatographie an Kieselgel gereinigt (Säule 15 × 2 cm, Laufmittel Petrolether/Ethylacetat 2:3).
Ausb. 59 mg (45 %), farbloses Öl; die analytischen Daten stimmen mit den unter b) angegebenen überein.

b) durch Deaceaalisierung mit p-TsOH: Man löst 11.36 g (21 mmol) Beispielverbindung 72 in 200 ml Chloroform, versetzt mit 0.19 g (1 mmol) p-Toluolsulfonsäure-Monohydrat sowie 12.27 ml (146 mmol) Ethandithiol und erhitzt unter Rückfluß. Nach 45 min läßt man auf Raumtemp. abkühlen und wäscht mit je 50 ml ges. Natriumhydrogencarbonat-Lösung, 0.5 N Salzsäure und Wasser. Die organische Phase wird über Magnesiumsulfat getrocknet und anschließend vom Lösungsmittel befreit. Eine chromatographische Reinigung (Säule 30 × 5 cm, Laufmittel Petrolether/Ethylacetat 2:3) des Rohprodukts liefert nach Trocknen i. Vak. die Titelverbindung.
Ausb. 8.44 g (80 %), farbloses, nach Tagen erstarrendes Öl, $[\alpha]_D^{25}$ = +9.4 (c = 1, - $CHCl_3$); $R_F$ = 0.39 (Petrolether/Ethylacetat 1:1).

Beispiel 74

Herstellung von Bibliotheken des Typs Alkyl-3,4-O-isopropyliden-2-O-alkyl-6-Ocarbamoyl-galactosid

a) Allgemeine Vorschrift zur Kupplung der Galactosylmercaptobuttersäuremethylester auf aminofunktionalisierte polymere Träger

Zu einer Lösung von 14.6 mmol des Thiogalactosids in 400 ml THF/Methanol/Wasser (2:2:1) gibt man 1.9 g (30 mmol) Lithiumhydroxid und läßt 8 h bei Raumtemp. rühren. Anschließend neutralisiert man (0.5 N Salzsäure oder Phosphatpuffer-Lösung), fügt 400 ml ges. Natriumchlorid-Lösung zu und extrahiert dreimal mit 200 ml Ethylacetat. Man trocknet über Magnesiumsulfat und befreit i. Vak. vom Lösungsmittel. Der Rückstand wird in 150 ml Dichlormethan oder DMF aufgenommen und die erhaltene Lösung in einem Festphasenreaktor mit 19.8 mmol des betreffenden aminofunktionalisierten Polymers, 3.7 ml (14.6 mmol) *N*,*N*'-Diisopropylcarbodiimid und 3.86 g (14.6 mmol) *N*-Hydroxybenzotriazol ü. N. geschüttelt. Anschließend saugt man ab und wäscht zehnmal mit je 50 ml DMF und Dichlormethan. Das beladene Polymer wird i. Vak. getrocknet und die Beladung mit Kohlenhydratmatrix über Elementaranalyse bestimmt.

b) Allgemeine Arbeitsvorschrift zum "Capping" der Polymere

Eine Mischung von 1.56 mmol des betreffenden Polymers mit einer Lösung von 0.45 ml (7.80 mmol) Essigsäure, 2.7 ml (15.60 mmol) *N*,*N*-Diisopropylethylamin und 3.74 g (7.80 mmol) PfPyU in 40 ml DMF wird 18 h geschüttelt. Nach dem Absaugen der flüssigen Phase wird gründlich mit DMF, Dichlormethan und Diethylether gewaschen und i. Vak. getrocknet.

c) Allgemeine Arbeitsvorschrift zur Alkylierung gecappter Polymere

Man schüttelt 0.078 mmol des betreffenden Polymers mit einer Lösung von 88 mg (0.78 mmol) Kalium-*tert*-butylat in 2 ml DMF. Nach 15 min wird abfiltriert und das Harz sofort mit einer Lösung von 41 mg (0.16 mmol) 18-Krone-6 und 0.78 mmol des betreffenden Alkylhalogenids in 2 ml DMF. Man läßt 3 h schütteln und saugt dann ab. Das Harz wird gründlich mit DMF, Methanol, Dichlormethan und Diethylether gewaschen und i. Vak. getrocknet.

d) Allgemeine Vorschrift zur Abspaltung der *tert*-Butyldiphenylsilylschutzgruppe am polymeren Träger

Zu einer Suspension von 0.056 mmol beladenem Polymer in 1.5 ml THF gibt man 0.56 ml (0.56 mmol, 1 M) Tetrabutylammoniumfluorid in THF. Die Mischung wird 4 h geschüttelt. Die Lösung wird vom Harz abgesaugt und fünfmal mit je 2 ml DMF und Dichlormethan gewaschen.

e) Allgemeine Arbeitsvorschrift für Carbamoylierungen

Variante A (mit 1,1'-Carbonyldiimidazol und Aminen)

Eine Lösung von 0.25 g (1.56 mmol) 1,1'-Carbonyldiimidazol in 2 ml DMF oder Dioxan wird mit 0.1 ml (0.58 mmol) *N,N*-Diisopropylethylamin, einer Spatelspitze DMAP und einer Spatelspitze KO*t*Bu zu 0.078 mmol Polymer gegeben. Man schüttelt 2 h und filtriert ab. Das Polymer wird anschließend ü. N. mit einer Lösung von 1.56 mmol des entsprechenden Amins in 2 ml DMF geschüttelt. Man filtriert und wäscht je fünfmal mit DMF und Dichlormethan.

Variante B (mit Isocyanaten)

Eine Lösung von 1.56 mmol des entsprechenden Isocyanats in 2 ml Dioxan wird zu 0.078 mmol Polymer gegeben. Dann setzt man eine Spatelspitze DMAP zu und schüttelt je nach Reaktivität der umzusetzenden OH-Gruppe 3-7 h. Man filtriert und wäscht gründlich mit Dioxan, Methanol und Dichlormethan.

f) Allgemeine Vorschrift zur Abspaltung der Galactosederivate vom polymerenTräger

Eine Suspension von 0.056 mmol polymergebundenem Galactosederivat in 1.5 ml abs. Dichlormethan wird in einer 5 ml- PE-Spritze (PE-Fritte, Plastikkappe) mit 0.3 ml einer 3.5%igen (0.36 ml Brom auf 10 ml Dichlormethan bzw. 5 - 10 eq. Brom) Lösung von Brom in abs. Dichlormethan und 0.08 ml (0.36 mmol) 2,6-Di-*tert*-butylpyridin oder entsprechender Menge polymergebundenem 2,6-Di-*tert*-Butylpyridin bei Raumtemp. geschüttelt. Nach 15 min setzt man 0.2 ml Cyclohexen, 0.2 ml des zu glycosylierenden abs. Alkohol und 25 mg (0.056 mmol) Tetraethylammoniumbromid zu. Nach 4 h wird abfiltriert und fünfmal mit 1 ml Dichlormethan gewaschen. Die vereinigten Filtrate werden i. Vak. vom Lösungsmittel befreit. Das erhaltene Rohprodukt wird in wenig Dichlormethan auf eine Kieselgelkartusche aufgebracht. Man eluiert zunächst mit 30 ml Petrolether. Diese Fraktion wird verworfen. Das Produkt erhält man durch Eluieren mit geeigneten Petrolether/Ethylacetat-Gemischen. Teilt man den Ansatz auf verschiedene Alkohole, so wird mit Brom-Lösung und 2,6-Di-*tert*-Butylpyridin vorteilhaft mit geschüttelt. Die Lösung wird aus der Spritze in ein Gefäß gespritzt und 5x mit abs. Dichlormethan nachgewaschen. Diese erhaltene Mischung wird auf verschiedene Gefäße verteilt, welche das Ammoniumbromid, Cyclohexen und den jeweiligen Alkohol enthalten. Die Gefäße werden verschlossen und geschüttelt. Zu langes Stehen an der Luft ist bei den Brom-Lösungen zu vermeiden, da diese feuchtigkeitsempfindlich sind.

Parallelsynthese

Man behandelt 6 × 3.1 g (2.42 mmol) 33 nach der allgemeinen Vorschrift mit PfPyU und Essigsäure. Die so erhaltenen Harze werden nach der allgemeinen Vorschrift mit Methyliodid, Ethyliodid, Heptyliodid, Benzylbromid, *p*-Bromobenzylbromid und *o*-Cyanobenzylbromid alkyliert. Die Silylschutzgruppe wird anschließend nach der allgemeinen Vorschrift abgespalten. Dann werden je 150 mg (ca. 0.119 mmol) der erhaltenen Harze entweder nach der allgemeinen Vorschrift mit Diethylamin, Benzylamin, Methoxyethylamin, Cyclopropylmethylamin oder Glycin-*tert*-Butylester oder nach der allgemeinen Vorschrift mit *p*-Chlorphenylisocyanat, *o*-Trifluormethylphenylisocyanat, *o*-Nitrophenylisocyanat, *p*-Cyanophenylisocyanat, *m,p*-Dichlorphenylisocyanat, *m*-Fluorphenylisocyanat, Phenylisocyanat, *n*-Propylisocyanat, *tert*-Butylisocyanat oder Ethyloxycarbonylmethylisocyanat zur Reaktion gebracht. Die Polymere werden in 2 ml Dichlormethan gequollen und mit jeweils 0.9 ml einer 3.5%igen Brom-Lösung in Dichlormethan sowie 0.24 ml (1.08 mmol) 2,6-Di-*tert*-butylpyridin 20 min geschüttelt. Die Polymere werden abfiltriert und mehrmals mit Dichlormethan gewaschen. Die erhaltenen Lösungen werden gedrittelt und mit je 55 mg (0.26 mmol) Tetraethylammoniumbromid,

0.2 ml Cyclohexen und 0.2 ml Methanol, Ethanol oder *i*-Propanol 5 h geschüttelt. Die Lösungen werden i. Vak. eingeengt. Die Rückstände werden in je 250 µl Dichlormethan aufgenommen und auf Kieselgelkartuschen aufgebracht. Man wäscht mit je 30 ml Petrolether und verwirft das Eluat. Anschließend wird mit je 5 ml Petrolether/Ethylacetat (1:1) eluiert und das erhaltene Produkt i. Vak. eingeengt. Durch Aktivierung mit CDI (Variante A) werden folgenden Verbindungen der Formel (A) erhalten:

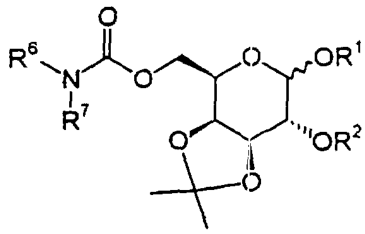

(A)

| | $R^2$ | $R^6$ | $R^7$ | $R^1$ | Ausb. | $R_T$ a) | $R_F$ b) |
|---|---|---|---|---|---|---|---|
| 1 | 4-BrBn | $CH_3O(CH_2)_2$ | H | Me | 2 mg (10 %) | 18.41 | 0.60 |
| 2 | 4-BrBn | *c*-$PrCH_2$ | H | Me | 3 mg (15 %) | 17.69 | 0.58 |
| 3 | 4-BrBn | *t*-$BuOOCCH_2$ | H | Me | 9 mg (41 %) | 18.28, 18.59 | 0.59 |
| 4 | 2-CNBn | *c*-$PrCH_2$ | H | Me | 4 mg (23 %) | 15.81 | 0.28 |
| 5 | 2-CNBn | *t*-$EuOOCCH_2$ | H | Me | 4 mg (20 %) | 17.35 | 0.58 |
| 6 | Bn | Et | Et | Et | 11 mg (64 %) | 18.21, 18.48 | 0.62, 0.68 |
| 7 | Bn | *c*-$PrCH_2$ | H | Et | 6mg (35%) | 16.94 | 0.55 |
| 8 | Bn | Bn | H | Et | 10 mg (54%) | 17.69,18.03 | 0.60 |
| 9 | Bn | *t*-$BuOOCCH_2$ | H | Et | 6 mg (31 %) | 17.60, 17.91 | 0.57 |
| 10 | 4-BrBn | Et | Et | Et | 5 mg (25 %) | 19.29, 19.72 | 0.75 |
| 11 | 4-BrBn | $CH_3O(CH_2)_2$ | H | Et | 4 mg (19%) | 19.05 | 0.36 |
| 12 | 4-BrBn | *c*-$PrCH_2$ | H | Et | 4 mg (20 %) | 18.04 | 0.70 |
| 13 | 4-BrBn | *t*-$BuOOCCH_2$ | H | Et | 6 mg (30 %) | 18.97, 19.33 | 0.72 |
| 14 | 2-CNBn | Bn | H | Et | 5 mg (26 %) | 17.13, 17.34 | 0.60 |
| 15 | 2-CNBn | $CH_3O(CH_2)_2$ | H | Et | 2 mg (11 %) | 13.86 | 0.21 |
| 16 | 2-CNBn | *c*-$PrCH_2$ | H | Et | 5 mg (28 %) | 16.30, 16.53 | 0.59 |
| 17 | 2-CNBn | *t*-$BuOOCCH_2$ | H | Et | 5 mg (25 %) | 17.04, 17.23 | 0.66 |
| 18 | Bn | Et | Et | *i*-Pr | 9 mg (51%) | 17.79, 17.96 | 0.74 |
| 19 | Bn | Bn | H | *i*-Pr | 10 mg (53%) | 18.36, 18.65 | 0.72 |
| 20 | Bn | $CH_3O(CH_2)_2$ | H | *i*-Pr | 3 mg (17 %) | 15.84, 16.21 | 0.43 |
| 21 | Bn | *c*-$PrCH_2$ | H | *i*-Pr | 10 mg (57%) | 17.63, 17.94 | 0.70 |
| 22 | Bn | *t*-$BuOOCCH_2$ | H | *i*-Pr | 8 mg (40 %) | 18.22, 18.51 | 0.74 |
| 23 | 4-BrBn | Et | Et | *i*-Pr | 3 mg (15 %) | 20.48, 20.68 | 0.48 |
| 24 | 4-BrBn | $CH_3O(CH_2)_2$ | H | *i*-Pr | 5 mg (24 %) | 19.26, 19.70 | 0.11 |
| 25 | 4-BrBn | *c*-$PrCH_2$ | H | *i*-Pr | 3 mg (15 %) | 19.10 | 0.49 |
| 26 | 4-BrBn | *t*-$BuOOCCH_2$ | H | *i*-Pr | 12 mg (52 %) | 19.26, 19.50 | 0.50 |
| 27 | 2-CNBn | Bn | H | *i*-Pr | 6 mg (30 %) | 17.79, 17.94 | 0.66 - |
| 28 | 2-CNBn | *c*-$PrCH_2$ | H | *i*-Pr | 5 mg (27 %) | 17.03, 17.18 | 0.64 |
| 29 | 2-CNBn | *t*-$BuOOCCH_2$ | H | *i*-Pr | 5 mg (24 %) | 16.23 | 0.66 |

Durch Aktivierung mit Isocyanaten (Variante B) werden folgenden Verbindungen der Formel B erhalten

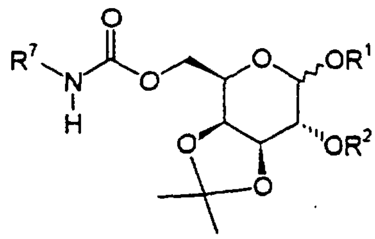

B

| | $R^7$ | $R^2$ | Ausb. | $R_T$ a) | $R_F$ b) |
|---|---|---|---|---|---|
| 1 | 4-ClPh | Me | 5 mg | 18.75 | 0.20; 0.16 |
| 2 | 2-$CF_3$Ph | Me | 5 mg (30 %) | 18.94 | 0.31; 0.26 |
| 3 | 2-$NO_2$Ph | Me | 5 mg (31 %) | 18.80 | 0.30; 0.26 |
| 4 | 4-CN-Ph | Me | 3 mg (20 %) | 13.93 | 0.06 |
| 5 | 3,4-$Cl_2$Ph | Me | 4 mg (26 %) | 19.35 | 0.20; 0.14 |
| 6 | 4-ClPh | Me | 5 mg (31 %) | 16.53 | 0.34 |
| 7 | 2-$CF_3$Ph | Me | 5 mg (29 %) | 17.63 | 0.46 |
| 8 | 2-$NO_2$Ph | Me | 9 mg (54 %) | 16.36 | 0.46 |
| 9 | 4-CN-Ph | Me | 4 mg (25 %) | 14.75 | 0.13 |
| 10 | 3,4-$Cl_2$Ph | Me | 5 mg (29 %) | 17.84 | 0.29 |
| 11 | 4-ClPh | Me | 9 mg (48 %) | 21.35 | 0.63 |
| 12 | 2-$CF_3$Ph | Me | 14 mg (69 %) | 21.27 | 0.61 |
| 13 | 2-$NO_2$Ph | Me | 11 mg (57 %) | 21.08 | 0.70 |
| 14 | 4-CN-Ph | Me | 6 mg (32 %) | 20.04 | 0.36 |
| 15 | 3,4$Cl_2$Ph | Me | 7 mg (35 %) | 23.38 | 0.63 |
| 16 | 4-ClPh | Me | 5 mg (23 %) | 19.90;20.05 | 0.30 |
| 17 | 2-$CF_3$Ph | Me | 10 mg (43 %) | 19.49 | 0.40 |
| 18 | 2-$NO_2$Ph | Me | 9 mg (41 %) | 19.78;19.88 | 0.39 |
| 19 | 4-CN-Ph | Me | 3 mg (14 %) | 18.53;18.64 | 0.13 |
| 20 | 3,4$Cl_2$Ph | Me | 9 mg (39 %) | 20.65;20.95 | 0.34; 0.26 |
| 21 | 4-ClPh | Me | 4 mg | 16.12;16.92 | 0.29; 0.21 |
| 22 | 2-$CF_3$Ph | Me | 7 mg (34 %) | 20.72 | 0.33 |
| 23 | 2-$NO_2$Ph | Me | 6 mg (30 %) | 17.83 | 0.31 |
| 24 | 4-CN-Ph | Me | 3 mg | 14.46 | 0.10 |
| 25 | 3,4-$Cl_2$Ph | Me | 4 mg (19 %) | 19.26;19.69 | 0.24 |
| 26 | 4-ClPh | Et | 4 mg (25 %) | 18.98 | 0.29 |
| 27 | 2-$CF_3$Ph | Et | 5 mg (29 %) | 15.93;16.09 | 0.42 |
| 28 | 2-$NO_2$Ph | Et | 4 mg (24 %) | 14.49 | 0.40 |
| 29 | 4-CN-Ph | Et | 3 mg | 12.21 | 0.15 |
| 30 | 3,4-$Cl_2$Ph | Et | 4 mg (23 %) | 19.69;19.90 | 0.33 |
| 31 | 4-ClPh | Et | 4 mg (24 %) | 17.26 | 0.54 |
| 32 | 2-$CF_3$Ph | Et | 8 mg (44 %) | 16.79 | 0.38 |
| 33 | 2-$NO_2$Ph | Et | 7 mg (41 %) | 17.24 | 0.55 |
| 34 | 4-CN-Ph | Et | 4 mg (24 %) | 15.55 | 0.27 |
| 35 | 3,4-$Cl_2$Ph | Et | 4 mg (22 %) | 18.63 | 0.46 |
| 36 | 4-ClPh | Et | 9 mg (46 %) | 21.91;22.07 | 0.75 |
| 37 | 2-$CF_3$Ph | Et | 11 mg (53 %) | 20.89;21.16 | 0.71; 0.64 |
| 38 | 2-$NO_2$Ph | Et | 9 mg (45 %) | 22.14 | 0.80 |

(fortgesetzt)

| | $R^7$ | $R^2$ | Ausb. | $R_T$[a)] | $R_F$[b)] |
|---|---|---|---|---|---|
| 39 | 4-CN-Ph | Et | 6 mg (31 %) | 20.64;20.79 | 0.57 |
| 40 | 3,4-$Cl_2$Ph | Et | 8 mg (38 %) | 22.28 | 0.77 |
| 41 | 4-ClPh | Et | 4 mg (18 %) | 18.98 | 0.46 |
| 42 | 2-$CF_3$Ph | Et | 10 mg (42 %) | 19.89;20.18 | 0.55 |
| 43 | 2-$NO_2$Ph | Et | 6 mg (27 %) | 20.59;20.74 | 0.55 |
| 44 | 4-CN-Ph | Et | 4 mg (18 %) | 18.73;19.31 | 0.25 |
| 45 | 3,4-$Cl_2$Ph | Et | 8 mg (34 %) | 19.39;19.53 | 0.45 |
| 46 | 4-ClPh | Et | 6 mg (30 %) | 16.11 | 0.35 |
| 47 | 2-$CF_3$Ph | Et | 6 mg (29 %) | 20.68;21.0[e)] | 0.45; 0.36 |
| 48 | 2-$NO_2$Ph | Et | 6 mg (29 %) | 18.61 | 0.41 |
| 50 | 3,4-$Cl_2$Ph | Et | 3 mg (14 %) | 19.68;19.82 | 0.33 |
| 51 | 4-ClPh | *i*-Pr | 4 mg (24 %) | 17.12;18.89 | 0.35 |
| 52 | 2-$CF_3$Ph | i-Pr | 11 mg (61 %) | 21.66 | 0.48 |
| 53 | 2-$NO_2$Ph | *i*-Pr | 8 mg (47 %) | 17.16 | 0.45 |
| 55 | 3,4-$Cl_2$Ph | *i*-Pr | 4 mg (22 %) | 19.66 | 0.35 |
| 56 | 4-ClPh | *i*-Pr | 6 mg (35 %) | 17.90;18.07 | 0.66 |
| 57 | 2-$CF_3$Ph | *i*-Pr | 9 mg (48 %) | 17.60;17.84 | 0.55 |
| 58 | 2-$NO_2$Ph | *i*-Pr | 8 mg (45 %) | 17.22;18.06 | 0.66 |
| 59 | 4-CN-Ph | *i*-Pr | 4 mg (24 %) | 16.31 | 0.38 |
| 60 | 3,4-$Cl_2$Ph | *i*-Pr | 4 mg (22 %) | 16.35 | 0.54 |
| 61 | 4-ClPh | *i*-Pr | 6 mg (30 %) | 22.47 | 0.77 |
| 62 | 2-$CF_3$Ph | *i*-Pr | 10 mg (47 %) | 22.15;22.39 | 0.71; 0.64 |
| 63 | 2-$NO_2$Ph | *i*-Pr | 9 mg (44 %) | 22.61;22.92 | 0.79 |
| 64 | 4-CN-Ph | *i*-Pr | 5 mg (25 %) | 21.25 | 0.59 |
| 65 | 3,4-$Cl_2$Ph | *i*-Pr | 7 mg (36 %) | 23.47 | 0.80 |
| 66 | 4-ClPh | *i*-Pr | 7 mg (31 %) | 21.04 | 0.52 |
| 67 | 2-$CF_3$Ph | *i*-Pr | 10 mg (42 %) | 23.71[e)] | 0.62 |
| 68 | 2-$NO_2$Ph | *i*-Pr | 8 mg (34 %) | 21.30 | 0.57 |
| 69 | 4-CN-Ph | *i*-Pr | 7 mg (31 %) | 22.43;22.5[e)] | 0.43; 0.33 |
| 70 | 3,4-$Cl_2$Ph | *i*-Pr | 10 mg (41 %) | 21.72 | 0.60; 0.52 |
| 72 | 2-$CF_3$Ph | *i*-Pr | 6 mg (27 %) | 23.78[e)] | 0.47; 0.41 |
| 73 | 2-$NO_2$Ph | *i*-Pr | 6 mg (28 %) | 22.16[e)] | 0.54; 0.50 |
| 74 | 4-CN-Ph | *i*-Pr | 3 mg (15 %) | 16.78[e)] | 0.29 |
| 75 | 3,4-$Cl_2$Ph | *i*-Pr | 7 mg (32 %) | 23.13[e)] | 0.48; 0.42 |
| 76 | 4-Br-Ph | Me | 4 mg (19 %) | 15.29;15.73 | 0.27 |
| 77 | Pr | Me | 5 mg (30%) | 17.27 | 0.16 |
| 79 | 3-F-Ph | Me | 4 mg (21 %) | 16.92; 17.06 | 0.25 |
| 80 | $EtOOCCH_2$ | Me | 4 mg (22 %) | 17.28 | 0.09 |
| 81 | 4-Br-Ph | Et | 4 mg (18 %) | 18.98 | 0.45 |
| 82 | Pr | Et | 5 mg (29 %) | 16.02;19.41 | 0.25 |
| 83 | *t*-Bu | Et | 4 mg (22 %) | 17.72 | 0.46 |
| 84 | 3-F-Ph | Et | 4 mg (21 %) | 19.35 | 0.48 |
| 85 | $EtOOCCH_2$ | Et | 4 mg (21 %) | 17.99 | 0.21 |
| 86 | 4-Br-Ph | *i*-Pr | 3 mg (13 %) | 19.44 | 0.48 |
| 87 | Pr | *i*-Pr | 5 mg (28 %) | 16.84 | 0.32 |
| 88 | *t*-Bu | *i*-Pr | 3 mg (16 %) | 13.65 | 0.48 |

(fortgesetzt)

| | $R^7$ | $R^2$ | Ausb. | $R_T$[a)] | $R_F$[b)] |
|---|---|---|---|---|---|
| 89 | 3-F-Ph | *i*-Pr | 3 mg (15 %) | 19.90 | 0.55; 0.48 |
| 90 | $EtOOCCH_2$ | *i*-Pr | 2 mg (10 %) | 19.16[e)] | 0.50 |

a) Säule B, Grad.2; b) Petroether/Ethylacetat (2:1); e) Säule B, Grad 3
Säule B: Beckmann-Anlage (System Gold), Säule Nucleosil 100-5 C18 gemessen bei 220 und 254 nm
Grad. 2 = $CH_3CN:H_2O$ 10:90 - 90-10, Fluß 1 ml/min, 0.1% TFA
Grad. 3 = $CH_3CN:H_2O$ 10:90 - 90-10, Fluß 0.65 ml/min, 0.1 % TFA

Beispiel 75

Funktionalisierung der 1-, 2-, 4- und 6-Position

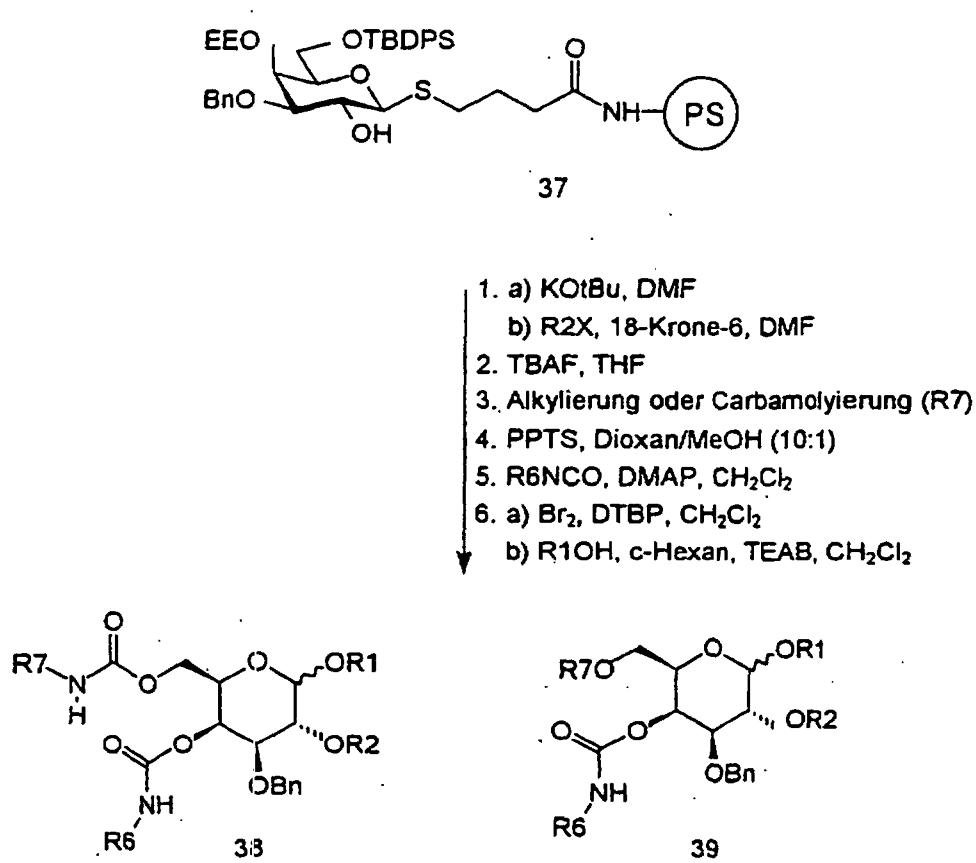


37

38 39

Folgende Verbindungen werden erhalten:

| | $R^2$ | $R^7$ | $R^6$ | $R^1$ | Ausb. (%) |
|---|---|---|---|---|---|
| 38-1 | Pr | *o*-$NO_2$Ph | *o*-$CF_3$Ph | Me | 20[a)] |
| 38-2 | *p*-BrBn | *c*-$PrCH_2$ | *o*-$NO_2$Ph | Et | 15 |
| 38-3 | Hep | *p*-CN-Bn | *o*-$CF_3$Ph | *i*-Pr | 6 |
| 39-1 | *p*-*t*BuBn | $CH_2COO$*t*Bu | *p*-ClPh | Me | 14 |

Beispiel 76

4-*S*-(2'-*O*-Acetyl-3'-*O*-benzyl-6'-*O*-*tert*-butyldiphenylsilyl-β-D-galactopyranosyl)-4-mercaptobuttersäuremethylester

Man erhitzt eine Mischung von 4.0 g (6.9 mmol) Beispielverbindung 14a und 2.1 g (8.32 mmol) Dibutylzinnoxid in 50 ml Benzol 2 h am Wasserabscheider unter Rückfluß. Anschließend destilliert man 25 ml Benzol ab und versetzt mit 3.1 g (8.3 mmol) Tetrabutylammoniumbromid und 1.4 ml (11.8 mmol) Benzylbromid. Die Lösung wird 16 h bei 50 C gerührt und dann i. Vak. weitgehend eingeengt. Man nimmt in 50 ml Dichlormethan auf und wäscht dreimal mit je 10 ml Wasser. Nach Trocknen über Magnesiumsulfat.wird das Lösungsmittel i. Vak. entfernt. Das Rohprodukt wird durch Chromatographie an Kieselgel gereinigt (Säule 20 □ 5 cm, Laufmittel Petrolether/Ethylacetat 4: 1 ).
Ausb. 2.66 g (58 %), gelbliches Öl, $[\alpha]_D^{25}$ = +3.0 (*c* = 1, $CHCl_3$); $R_F$ = 0.25 (Petrolether/Ethylacetat 4:1)

Beispiel 77

*S*-(2'-*O*-Acetyl-3'-*O*-benzyl-4'-*O*-[1"-(*R*/*S*)-ethoxyethyl]-6'-*O*-*tert*-butyldiphenylsilyl-β-D-galactopyranosyl)-4-mercaptobuttersäuremethylester

Eine Lösung von 2.58 g (3.86 mmol) Beispielverbindung 76 in 100 ml Dichlormethan wird nach Zugabe von 50 ml Ethylvinylether und 0.49 g (1.93 mmol) Pyridinium-ptoluolsulfonat 4 h bei Raumtemp. gerührt. Der Ansatz wird in ges.
Natriumhydrogencarbonat-Lösung gegossen und die wäßrige Phase mit Ethylacetat extrahiert. Anschließend werden die vereinigten organischen Phasen über Magnesiumsulfat getrocknet und i. Vak. vom Lösungsmittel befreit. Die Reinigung erfolgt durch Chromatographie an Kieselgel (Säule 15 x 3 cm, Laufmittel Petrolether/Ethylacetat 4:1).
.Ausb. 1.25 g (45 %), gelbliches Öl, $[\alpha]_D^{25}$ =-13.3 (*c* = 1, $CHCl_3$); $R_F$ = 0.22 (Petrolether/Ethylacetat 4:1).

Beispiel 78

*S*-(3'-*O*-benzyl-4'-*O*-[1"-(*R*/*S*)-ethoxyethyl]-6'-*O*-*tert*-butyldiphenylsilyl-β-Dgalactopyranosyl)-4-mercaptobuttersäuremethylester-polymergebunden

Man belädt nach der allgemeinen Arbeitsvorschrift 1.45 g (1.60 mmol) Aminomethylpolystyrol mit 1.25 g (1.69 mmol) Beispielverbindung 81. Beladung nach Schwefel-Anteil der Elementaranalyse: 0.61 mmol/g.

Beispiel 79

Funktionalisierung der 1, 2, 3, 4 und 6-Position

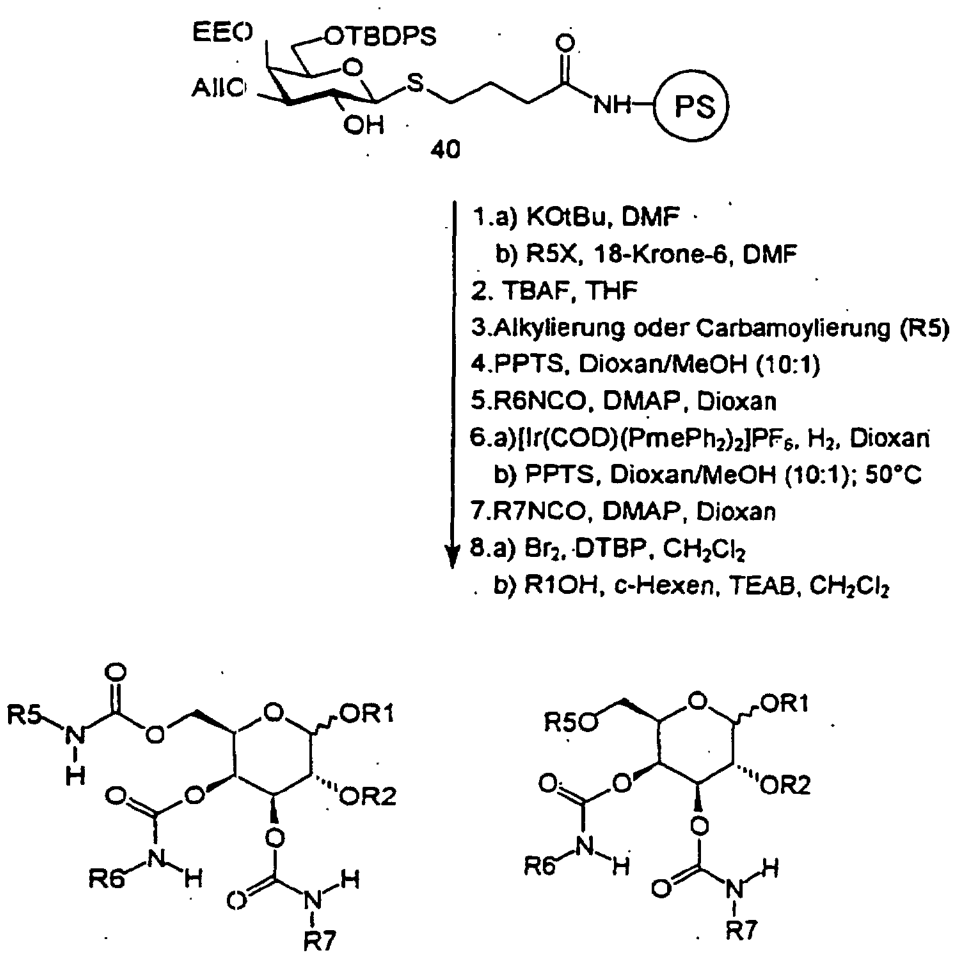


40

41

42

Folgende Verbindungen werden erhalten:

| | $R^2$ | $R^5$ | $R^6$ | $R^7$ | $R^1$ | Ausb. (%) |
|---|---|---|---|---|---|---|
| 41-1 | *p-t*BuBn | 2 × Et (aus $Et_2NH$) | *p*-Cl-Ph | *o*-$NO_2$Ph | Me | 23 |
| 41-2 | Hep | *o*-$NO_2$Ph | *o*-$CF_3$Ph | *p*-CN-Ph | Me | 16* |
| 41-3 | *p*-BrBn | *m,p*-$Cl_2$Ph | *o*-$CF_3$Ph | *p*-Cl-Ph | *i*-Pr | 5 |
| 42-1 | *p*-BrBn | Hep | *o*-$NO_2$Ph | *p*-Cl-Ph | Me | 38 |
| 42-2 | *p-t*BuBn | Bu | *o*-$NO_2$Ph | *p*-CN-Ph | *i*-Pr | 4 |
| 42-3 | Bn | $CH_2COO$*t*Bu | *o*-$NO_2$Ph | *p*-Cl-Ph | Me | 6 |

*) Minderkomponente (nach Chromatographie)

Beispiel 80

S-(3'-O-allyl-4'-O-[1"-(R/S)-ethoxyethyl]-6'-O-tert-butyldiphenylsilyl-β-Dgalactopyranosyl)-4-mercaptobuttersäure-polymergebunden (40)

Man belädt nach der allgemeinen Arbeitsvorschrift 8.60 g (9.5 mmol) Aminomethylpolystyrol mit 6.70 g (10.0 mmol) 21 (Bsp. 14c).Beladung nach Schwefel-Anteil der Elementaranalyse: 0.93 mmol/g.

**Patentansprüche**

1. Verbindungen der Formel IIa, IIb und IIc

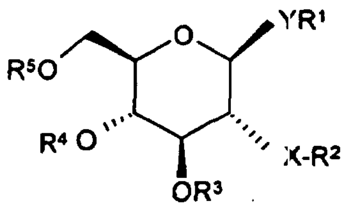

IIa

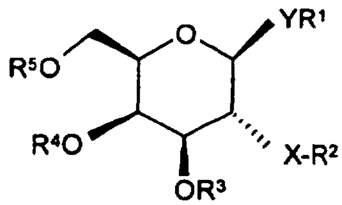

IIb

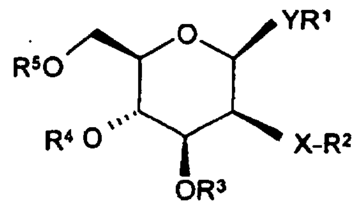

IIc

in denen bedeutet:

Y S oder O;
X O oder N;
$R^1$ eine Linkergruppe, die über eine kovalente Bindung mit einem durch ein Heteroatom funktionalisierten Träger verknüpft werden kann;
$R^2$ für den Fall, daß X gleich O ist,
Acetyl oder Benzoyl;
für den Fall, daß X gleich N ist,
eine Phthaloylschutzgruppe, DDE (1-(4,4-Dimethyl-2,6-dioxocyclohexyliden-ethyl) oder NDE (2-Acetyl-4-nitro-indan-1,3-dion);
$R^3$ eine Allylschutzgruppe;
$R^4$ Ethoxyethyl oder SEM (2-(Trimethylsilyl)ethoxymethyl);
$R^5$ tert.Butyldimetlhylsilyl oder tert. Butyldiphenylsilyl.

2. Verbindungen der Formel IIa, IIb oder IIc gemäß Anspruch 1, in der Y S bedeutet.

3. Verbindungen der Formel IIb gemäß Anspruch 1 oder 2, in denen $R^3$ und $R^4$ zusammen Isopropyliden oder Benzyliden bedeuten und die übrigen Reste X, Y, $R^1$, $R^2$ und $R^5$ wie in Anspruch 1 oder 2 definiert sind.

4. Verbindungen der Formel IIa, IIb oder IIc gemäß einem oder mehreren der Ansprüche 1 bis 3, in denen die Linkergruppe $R^1$ eine Gruppe der Formel III

$$-(C_1\text{-}C_6)\text{-alkylen-}[N\text{-}C(O)]_n\text{-}[(C_6\text{-}C_{12})\text{-arylen}]_p\text{-}(C_0\text{-}C_6)\text{-alkylen-}C(O)R^9 \qquad (III)$$

bedeutet, worin n und p 0 oder 1 bedeuten, wobei p und n nicht beide gleichzeitig 1 sein können;

$R^9$ $OR^{10}$ oder $NR^{11}R^{11}$ bedeutet, wobei
$R^{10}$ H, $(C_1\text{-}C_6)$-alkyl, $(C_1\text{-}C_6)$-Alkyl-$(C_6\text{-}C_{12})$-Aryl bedeutet, und
$R^{11}$ unabhängig voneinander H, $(C_1\text{-}C_6)$-alkyl, $(C_1\text{-}C_6)$-Alkyl-$(C_6\text{-}C_{12})$-Aryl oder einen polymeren festen Träger bedeutet.

**Claims**

1. Compounds of the formulae IIa, IIb and IIc:

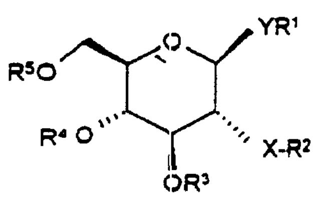

IIa

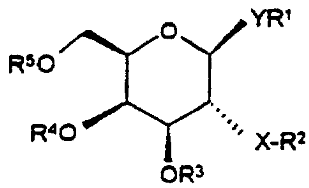

IIb

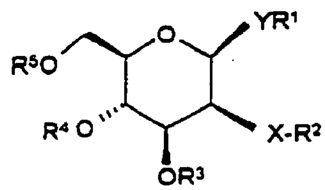

IIc

in each of which:

- Y is S or O;
- X is O or N;
- $R^1$ is a linker group which can be linked via a covalent bond to a support functionalized by a heteroatom;
- $R^2$, in the case in which X is equal to O, is acetyl or benzoyl; in the case in which X is equal to N, is a phthaloyl protective group, DDE (1-(4,4-dimethyl-2,6-dioxocyclohexylideneethyl) or NDE (2-acetyl-4-nitroindan-1,3-dione);
- $R^3$ is an allyl protective group;
- $R^4$ is ethoxyethyl or SEM (2-(trimethylsilyl)ethoxymethyl);
- $R^5$ is tert-butyldimethylsilyl or tert-butyldiphenylsilyl.

**2.** Compounds of the formulae IIa, IIb or IIc as claimed in claim 1 where Y is S.

**3.** Compounds of the formula IIb as claimed in claim 1 or 2, in each of which $R^3$ and $R^4$ together are isopropylidene or benzylidene and the other radicals X, Y, $R^1$, $R^2$ and $R^5$ are as defined in claim 1 or 2.

**4.** Compounds of the formula IIa, IIb or IIc as claimed in one or more of claims 1 to 3, in each of which the linker group $R^1$ is a group of the formula III

$$(C_1\text{-}C_6)\text{-alkylene-}[N\text{-}C(O)]_n\text{-}[(C_6\text{-}C_{12})\text{-arylene}]_p\text{-}(C_0\text{-}C_6)\text{-alkylene-}C(O)R^9 \qquad \text{(III)}$$

in which n and p are 0 or 1, where p and n cannot simultaneously be 1;

- $R^9$ is $OR^{10}$ or $NR^{11}R^{11}$, where
- $R^{10}$ is H, $(C_1\text{-}C_6)$-alkyl, $(C_1\text{-}C_6)$-alkyl-$(C_6\text{-}C_{12})$-aryl, and
- $R^{11}$ independently of one another is H, $(C_1\text{-}C_6)$-alkyl, $(C_1\text{-}C_6)$-alkyl-$(C_6\text{-}C_{12})$-aryl or a polymeric solid support.

## Revendications

**1.** Composés de formule IIa, IIb et IIc,

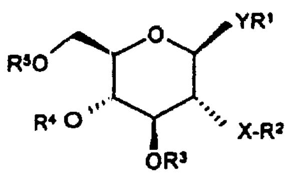

IIa

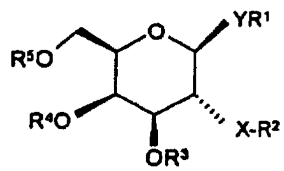

IIb

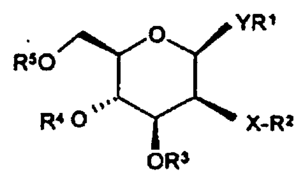

IIc

dans lesquelles :

Y représente S ou O ;

X représente O ou N ;

$R^1$ représente un groupe de liaison, qui peut être lié à un support fonctionnalisé par un hétéroatome par l'intermédiaire d'une liaison covalente ;

$R^2$ représente un acétyle ou un benzoyle au cas où X représente O,
représente un groupe protecteur phtaloyle, un DDE (1-4,4-diméthyl-2,6-dioxocyclohexylidène-éthyle) ou NDE(2-acétyl-4-nitro-indan-1,3-dione), au cas où X représente N ;

$R^3$ représente un groupe protecteur allyle ;

$R^4$ représente un éthoxyéthyle ou un SEM(2-(triméthylsilyl)éthoxyméthyle) ;

$R^6$ représente un tert-butyldiméthylsilyle ou un tert-butyldiphénylsilyle.

**2.** Composés de formules IIa, IIb ou IIc selon la revendication 1 dans laquelle Y représente S.

**3.** Composés de formule IIb selon la revendication 1 ou 2 dans laquelle $R^3$ et $R^4$ représentent conjointement un isopropylidène ou un benzylidène et les autres radicaux X, Y, $R^1$ , $R^2$ et $R^5$ sont tels que définis dans la revendication 1 ou 2.

**4.** Composés de formules IIa, IIb ou IIc selon l'une quelconque des revendications 1 à 3 dans lesquelles le groupe de liaison $R^1$ représente un groupe de formule III,
a
dans laquelle n et p valent 0 ou 1, où p et n ne peuvent pas tous les deux valoir simultanément 1 ;
$R^9$ représente $OR^{10}$ ou $NR^{11}R^{11}$, où
$R^{10}$ représente H, un alkyle en ($C_{1-6}$), un ($C_{1-6}$)-alkyl-($C_{6-12}$)-aryle, et
$R^{11}$ représente H, indépendamment l'un de l'autre, un alkyle en ($C_{1-6}$), un ($C_{1-6}$)-alkyl- ($C_{6-12}$)-aryle, ou un support solide polymère.